# EUROPEAN PATENT SPECIFICATION

(11) **EP 2 768 962 B1**
(45) Date of publication and mention of the grant of the patent: **19.12.2018**
(21) Application number: 12781038.0
(22) Date of filing: 17.10.2012
(51) Int. Cl.: C12N 15/82, C12P 7/08

(54) **PRODUCTION, STORAGE AND USE OF CELL WALL-DEGRADING ENZYMES**
HERSTELLUNG, LAGERUNG UND VERWENDUNG VON ZELLWANDABBAUENDEN ENZYMEN
PRODUCTION, STOCKAGE ET UTILISATION D'ENZYMES DÉGRADANT LA PAROI CELLULAIRE

(30) Priority: 17.10.2011 EP 11008353
(43) Date of publication of application: 27.08.2014
(73) Proprietor: Nomad Bioscience GmbH, 80333 München (DE)
(72) Inventor: HAHN, Simone, 06114 Halle (DE); GIRITCH, Anatoli, 06108 Halle (DE); GLEBA, Yuri, 10117 Berlin (DE)
(74) Representative: Blodig, Wolfgang
(86) International application number: PCT/EP2012/004349
(87) International publication number: WO 2013/056829

(56) References cited:
- WO-A1-98/16651
- WO-A2-98/11235
- WO-A2-2007/100897
- US-A- 6 013 860
- STICKLEN MARIAM B: "Plant genetic engineering for biofuel production: towards affordable cellulosic ethanol", NATURE REVIEWS GENETICS, NATURE PUBLISHING GROUP, GB, vol. 9, no. 6, 1 June 2008 (2008-06-01), pages 433-443, XP002527904, ISSN: 1471-0056, DOI: 10.1038/NRG2336
- Weinberg, Z. G.: "Preservation of forage crops by solid state lactic acid fermenation ensiling"; "4" In: "Current developments in solid state fermentation", 2008, XP008151742, pages 443-467, the whole document
- DEWAR, W.A., ET AL.: "The hydrolysis of grass hemicelluloses during ensilage", J. SCI. FD AGRIC., vol. 14, 1 June 1963 (1963-06-01), pages 411-417, XP002675901,

## Description

### Field of the Invention

The present invention relates to a process of degrading plant cell wall components of plant material. The invention also relates to a process of storing cell wall-degrading enzymes and to a use of silage containing one or more than one active cell wall-degrading enzymes for degrading plant cell wall components of plant material or for producing bioalcohol. Further, a process of producing a bioalcohol such as bioethanol from plant material is provided.

### Background of the Invention

Production of bioalcohol, mostly of bioethanol, is widely used throughout the world as an additive to fuel and gasoline as an at least partial replacement of fossil sources for fuels. Concerns, whether justified or not, with climate change aggravated by fossil fuel-derived carbon dioxide have led to a steady increase of bioethanol production in recent years. However, bioethanol production based on plants grown on farm fields consumes enormous areas of limited agricultural land and thus competes with food production and leads to increased food prices.

Conventional processes for bioethanol production rely on fermentation of glucose derived from starch. Although cellulose that is a major component of plant cell walls also consists of glucose units, cellulose-based bioethanol (cellulosic bioethanol) has so far not played a significant role in bioethanol production, since breakdown of cellulose to glucose is difficult on an industrial scale. For example, chemical hydrolysis steps to break down cellulose to cellulose fragments or glucose are frequently used before microbial processes are employed. For these reasons, cellulose, albeit abundantly produced by plants, is difficult to convert to bioethanol. Improvements in the use of cellulose for bioalcohol production are therefore highly desired. A review on biofuel production is provided by Sticklen, Nature Reviews 9 (2008), 433-443.

In order to avoid chemical hydrolysis or pre-hydrolysis steps in the breakdown of cellulose, enzymatic processes are widely pursued (see e.g. Bansal et al., Biotechnology Advances 27 (2009) 833-848). However, multiple enzymes are required to break down cellulose or plant cell wall material to glucose. Further, the enzymes required for the breakdown need to be produced in production processes independent from the glucose fermentation process, and represent a high cost factor for the production cellulosic bioalcohol. Further, these production processes are sophisticated processes requiring expert knowledge and equipment as found in the biotech industry, but cannot be carried out decentralized such as by farmers on farms where the plant material to be converted to bioalcohol is produced, which makes the entire bioalcohol production process from cellulose complex and non-economical.

WO 98/16651 A2 relates to the production of cellulose-degrading enzymes in genetically recombinant plants. It describes that whole plants transformed to express cellulases can be used directly or added to ensiled plant matter to increase the extent of fermentation of the ensiled matter.

Departing from the prior art, it is a problem of the present invention to provide plant cell wall-degrading enzymes in an economical way. It is a further problem of the invention to provide a process for producing and/or storing plant cell wall-degrading enzymes that can be carried out easily such as by farmers. It is a further problem to provide a process of degrading plant cell wall components of plant material and to provide a process for bioalcohol production.

### Summary of the Invention

These problems are solved according to the claims. Also described is:
(1) A process of producing a preparation of cell wall-degrading enzymes, comprising: (i) expressing in plants or plant parts one or more than one heterologous cell wall-degrading enzyme; (ii) harvesting said plants or plant part containing said one or more than one cell wall-degrading enzyme; and (iii) storing the harvest as a silage.
(2) A process of producing a preparation of cell wall-degrading enzymes, comprising:
   (i) transfecting plants or parts thereof with a nucleic acid molecule comprising a nucleic acid construct containing a nucleotide sequence encoding one or more than one cell wail-degrading enzyme and expressing in said plants or parts thereof said one or more than one cell wall-degrading enzyme; (ii) harvesting said plants or parts containing said one or more than one cell wall-degrading enzyme; and (iii) storing the harvest as a silage.
(3) The process according to (1) or (2), wherein said one or more than one cell wall-degrading enzyme is a cellulolytic enzyme selected from or such as endoglucanases, exoglucanases, exocellulases, β-glucosidases, or combinations thereof; hemicellulases; pectinases; and/or lignin degrading enzymes.
(4) The process according to any one of (1) to (3), wherein said cell wall-degrading enzymes are secretory enzymes in the organism from which they are derived.
(5) The process according to any one of (1) to (4), wherein step (iii) comprises storing said harvested plants or parts thereof for at least 1 month in an atmosphere of reduced oxygen at ambient temperature.
(6) The process according to any one of (1) to (5), wherein step (iii) comprises lactic acid bacteria fermentation or acidification to produce acidic conditions in said silage.
(7) The process according to any one of (1) to (6), wherein said cell wall-degrading enzymes expressed in the cytosol of cells of said plants or parts thereof, or said cell wall-degrading enzymes are targeted to the apoplast or to plastids of said plant or plant parts.
(8) The process according to (1), wherein step (i) comprises transfecting said plants or parts thereof with a nucleic acid molecule comprising a nucleic acid construct containing a DNA sequence encoding said one or more cell wall-degrading enzyme(s).
(9) The process according to any one of (1) to (8), wherein step (i) comprises spraying aerial parts of said plant with an aqueous suspension containing cells of an *Agrobacterium* strain comprising a DNA molecule comprising a nucleic acid construct containing a DNA sequence encoding said one or more than one cell wall-degrading enzyme.
(10) The process according to any one of (1) to (8), wherein step (i) comprises spraying aerial parts of said plant with an aqueous suspension containing cells of an *Agrobacterium* strain comprising a DNA molecule comprising a nucleic acid construct encoding a DNA or RNA replicon encoding said one or more than one cell wall-degrading enzyme.
(11) The process according to any one of (1) to (10), wherein step (iii) comprises storing the harvested plants or plant parts in a closed container or bag under conditions of reduced oxygen content for enabling lactic acid fermentation.
(12) Silage containing one or more than one active cell wall-degrading enzyme, said silage obtainable by storing plant or plant parts containing expressed cell wall-degrading enzymes under reduced oxygen content for allowing lactic acid fermentation, wherein said one or more than one cell wall-degrading enzyme may be heterologous to said plant or said plant part.

The inventors have surprisingly found that cell wall-degrading enzymes when expressed in plants, notably when expressed to a high level, can be stored as a silage of these plants over extended periods of time with little loss of enzymatic activity. Thus, expressed cell wall-degrading enzymes do not need to be purified from other plant components, such as proteases, for maintaining activity, which saves substantial costs. The inventors have further found that enzymes that degrade plant cell wall material are of exceptional stability upon storage of the plant or plant parts having expressed the enzymes. The stability under these conditions is higher than that of many native proteins. Accordingly, storage in silage leads to an enrichment of these enzymes in total soluble protein. In the form of the silage of the plants or plant parts containing expressed cell wall-degrading enzymes, the cell wall-degrading enzymes can be stored until the enzymes are required, e.g. for a process of degrading plant cell wall components of plant material in a process of producing a bioalcohol. Upon use, the silage can be added, in a reaction medium, to the plants or plant material cell wall material of which should be degraded. The invention has the advantage that plants containing cell wall-degrading enzymes can be easily produced and stored on a farm using techniques generally known to a farmer, as ensiling of forage is a widely used method for preserving animal feed over periods where fresh forage is limited. Purification of pant cell wall-degrading enzymes and costs associated therewith can be avoided. In the production of bioalcohol, the silage also enters the saccharification and alcohol fermentation process. Thus, the invention is of unprecedented efficiency and cost-effectiveness.

### Brief Description of the Figures

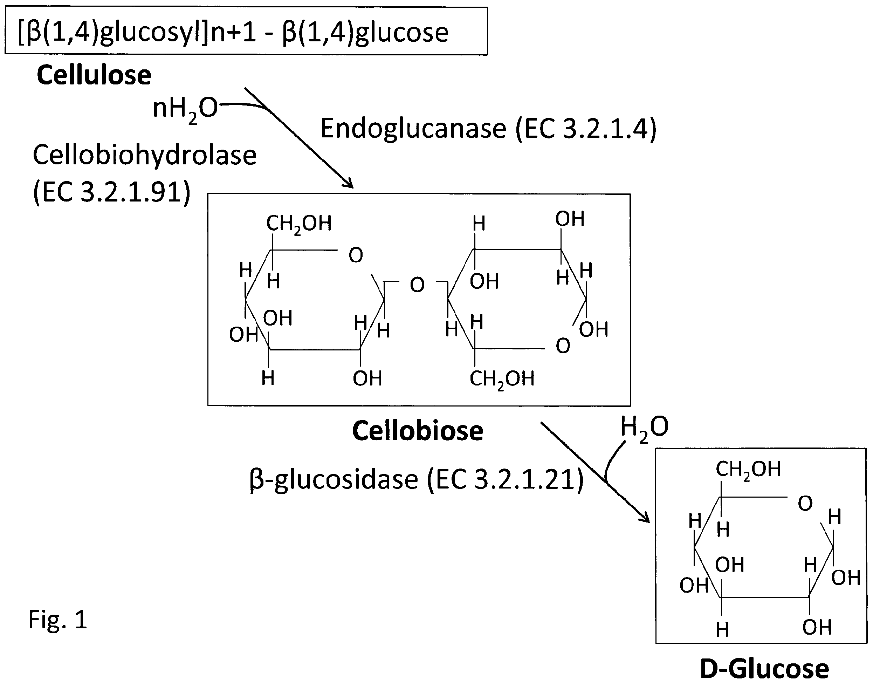

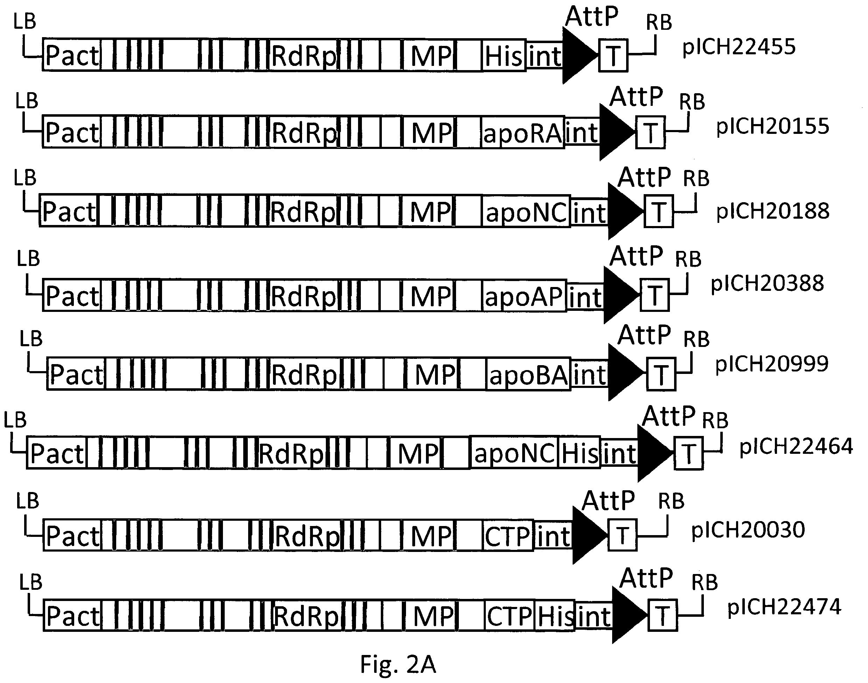

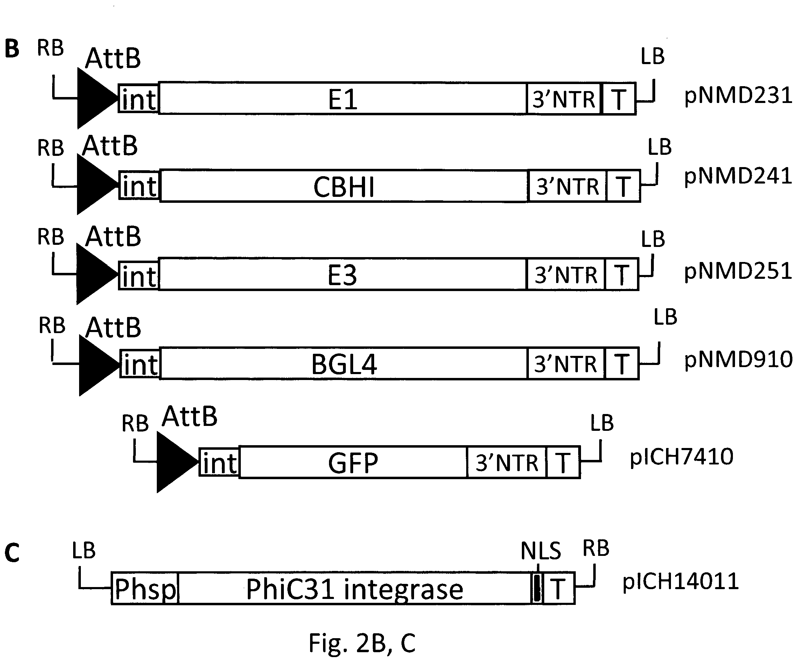

Fig. 1 outlines the relationship between the enzyme activities involved in the hydrolysis of cellulose. Endoglucanases cleave cellulose chains internally, providing free reducing and nonreducing chain termini that are subject to cellobiohydrolase (exoglucanase) cleavage. Cellobiohydrolases cleave cellulose chains termini releasing cellooligosaccharides, preferentially cellobiose. β-glucosidase hydrolyses cellobiose to glucose.
Fig. 2 shows schematically vectors used for the optimization of transient expression of cell wall degrading enzymes.
   (*A*) TMV-based 5'-provectors modules: pICH22455 construct for cytosolic expression of proteins of interest with N-terminal His (6)-tag and enterokinase cleavage site (His-EK); constructs for apoplast targeting pICH20155, pICH20188, pICH20388 and pICH20999 containing apoplast targeting presequences from rice amylase (apoRA), *Nicotiana plumbaginifolia* calreticulin (apoNC), apple pectinase (apoAP) and barley amylase (apoBA), respectively. Plasmid plCH22464 contains apoplast targeting presequence from *Nicotiana plumbaginifolia* calreticulin followed by His(6)-tag and enterokinase cleavage site (His-EK). pICH20030 construct for chloroplast targeting of expressed protein of interest contains chloroplast targeting presequence (CTP).
   (*B*) TMV-based 3'-provector modules: 3' modules for the expression of endoglucanase E1 from *Acidothermus cellulolyticus* (pNMD231), Exoglucanase 1 (CBHI) from *Trichoderma reesei* (pNMD241), exocellulase E3 from *Thermobifida fusca* (pNMD251), β-glucosidase BGL4 from *Humicola grisea* (pNMD910) and GFP (pICH7410).
   (*C*) Integrase module pICH14011. LB and RB, binary left and right borders, respectively; Pact, *Arabidopsis* actin 2 promoter; Phsp, *Arabidopsis* heat shock protein hsp 81.1 promoter; T, *nos* terminator; RdRp, RNA-dependent RNA polymerase; MP, movement protein; int, intron; AttP and AttB, recombination sites; apoRA, apoNC, apoAP and apoBA, apoplast targeting presequences from rice α-amylase, *Nicotiana plumbaginifolia* calreticulin, apple pectinase and barley amylase, respectively; CTP, chloroplast targeting presequence; NLS, nuclear localization signal.

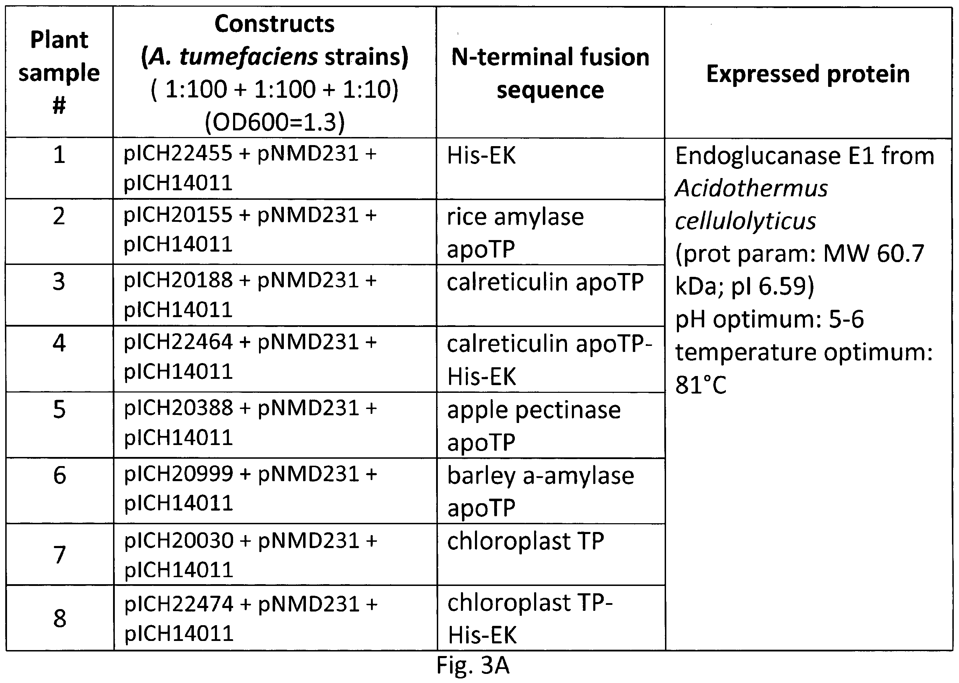

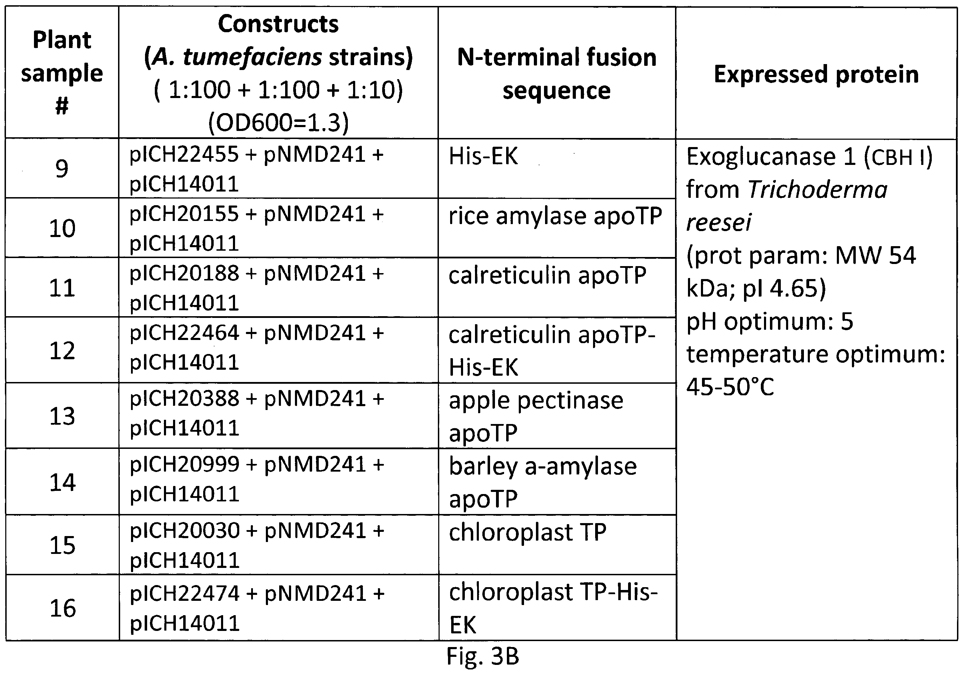

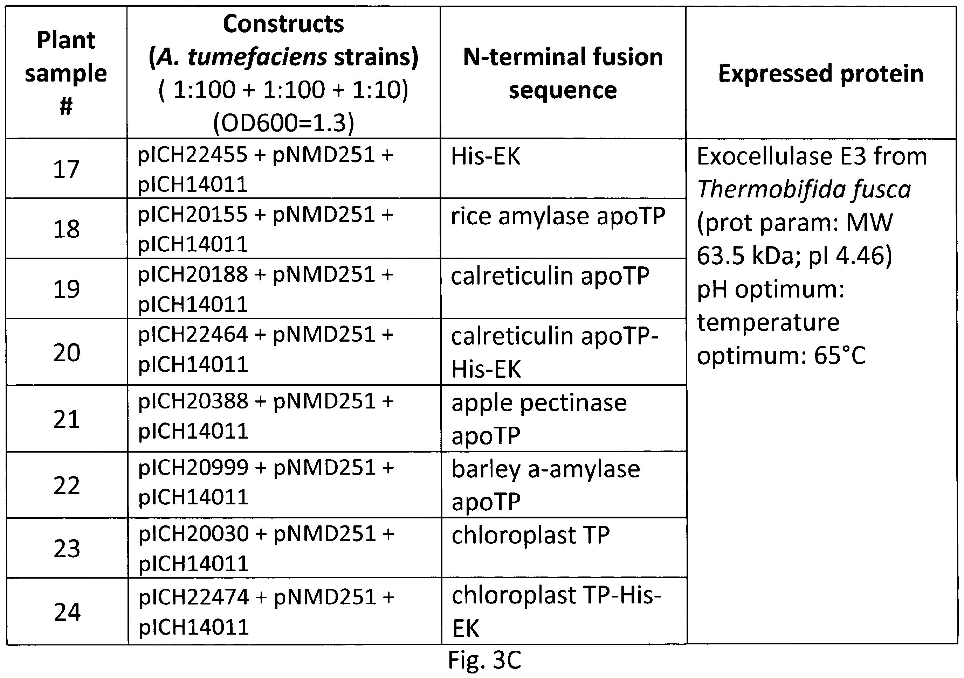

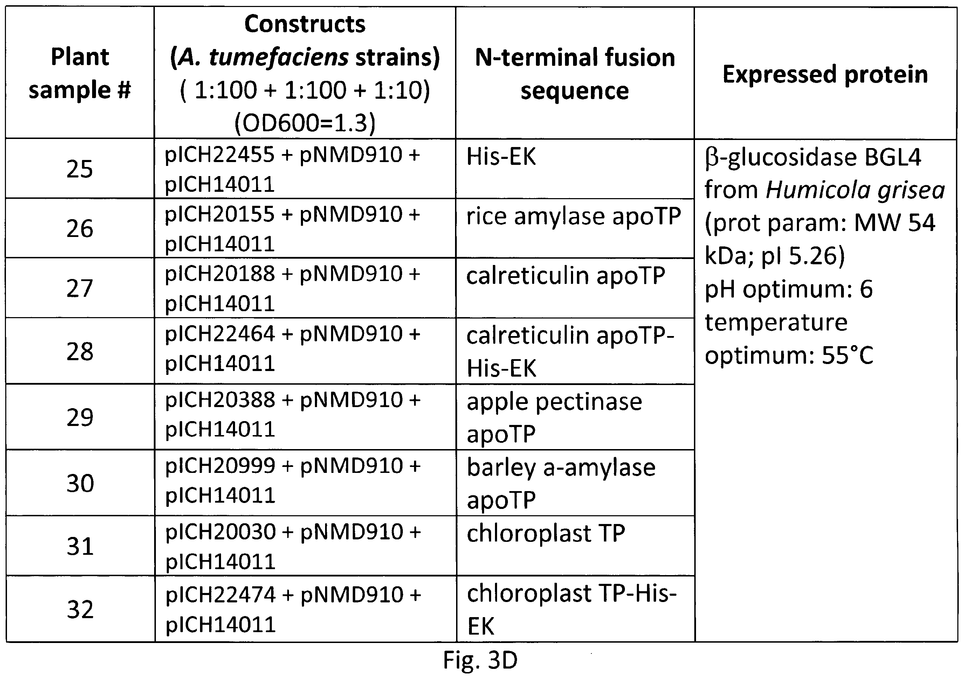

Fig. 3 provides an overview over agrobacterial inoculation for the optimisation of transient expression of cellulases in *Nicotiana benthamiana* as translational fusions to different N-terminal sequences. For each fusion protein, samples were inoculated into 3 different plants; plant material was harvested at 7 or 8 dpi (days post inoculation).

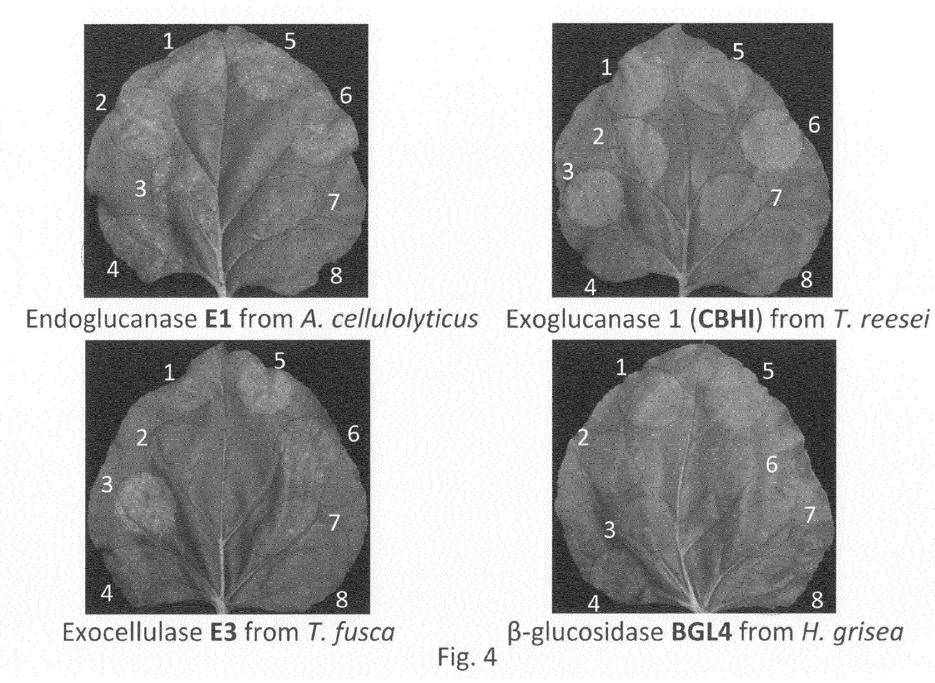

Fig. 4 photographs showing phenotypes of *Nicotiana benthamiana* leaves transiently expressing endoglucanase E1 from *Acidothermus cellulolyticus,* exoglucanase 1 (CBHI) from *Trichoderma reesei,* exocellulase E3 from *Thermobifida fusca* and β-glucosidase Bgl4 from *Humicola grisea* as fusion to different sequences at 8 dpi. N-terminal fusion sequences:
   1 - His(6)-tag and enterokinase cleavage site (His-EK);
   2 - rice amylase apoplast targeting presequence;
   3 - *Nicotiana plumbaginifolia* calreticulin apoplast targeting presequence;
   4 - *Nicotiana plumbaginifolia* calreticulin apoplast targeting presequence followed by His(6)-tag and enterokinase cleavage site (His-EK);
   5 - apple pectinase apoplast targeting presequence;
   6 - barley α-amylase apoplast targeting presequence;
   7 - chloroplast targeting presequence;
   8 - chloroplast targeting presequence followed by His(6)-tag and enterokinase cleavage site (His-EK).

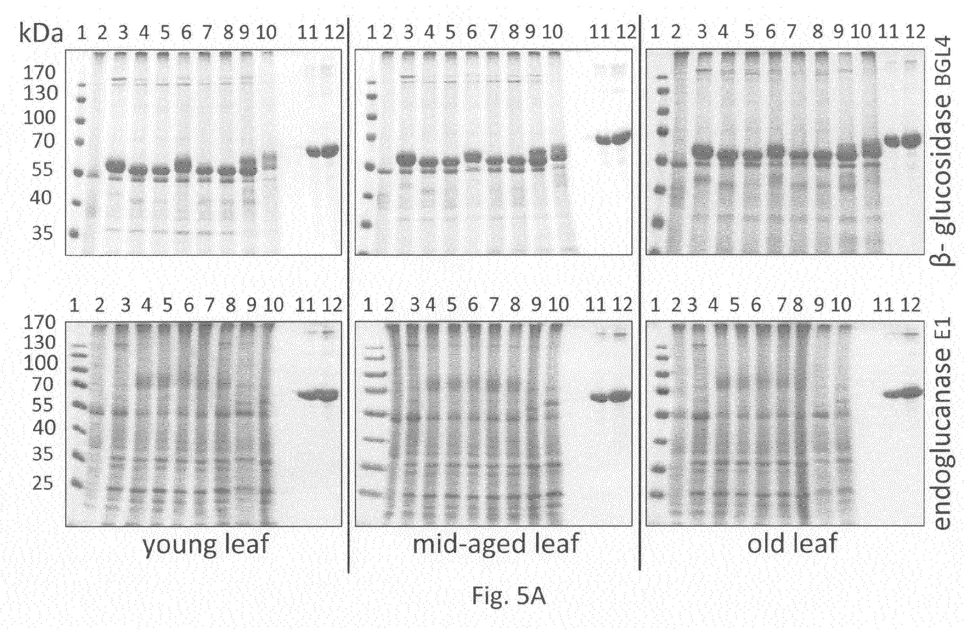

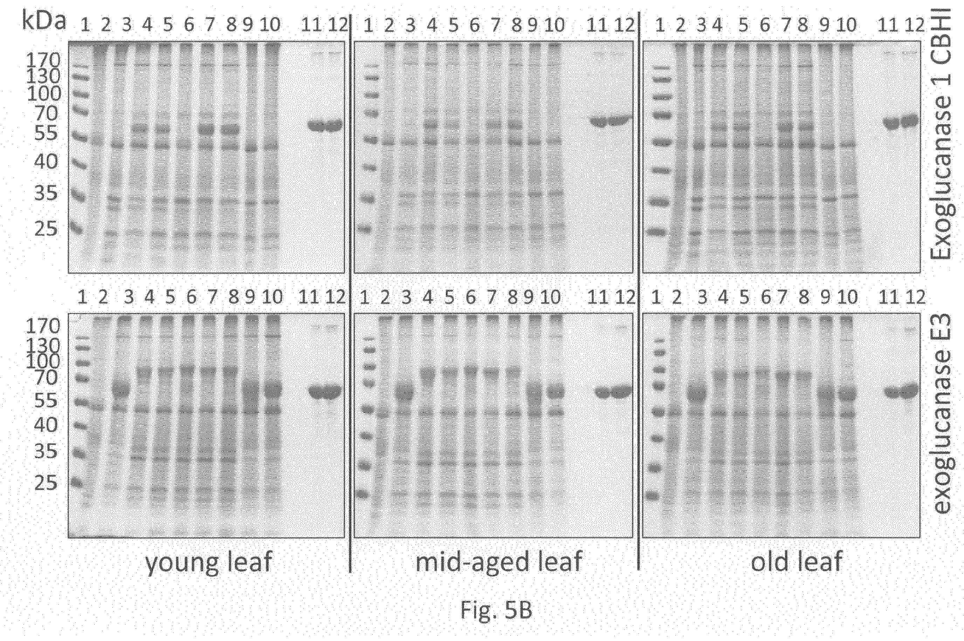

Fig. 5 shows results of optimisation of transient expression of cellulases via screening cellulase fusions to different N-terminal sequences for highest expression using TMV provectors. 1:100 dilutions of *Agrobacterium* cultures were inoculated by syringe; plant material was harvested at 11 dpi. Protein extracts containing 10 µg TSP were separated in 10% SDS-PAGE and stained using Coomassie. Leaves of different ages (young, mid-aged and old) were analyzed for each plant. The lanes are as follows:
   1 - PageRuler™ Prestained Protein Ladder (#SM0671, Fermentas);
   2 - uninfected leaf tissue;
   3 - fusion with His(6)-tag followed by enterokinase cleavage site (His-EK);
   4 - fusion with rice amylase apoplast targeting presequence;
   5 - fusion with *Nicotiana plumbaginifolia* calreticulin apoplast targeting presequence;
   6 - fusion with *Nicotiana plumbaginifolia* calreticulin apoplast targeting presequence followed by His(6)-tag and enterokinase cleavage site (His-EK);
   7 - fusion with apple pectinase apoplast targeting presequence;
   8 - fusion with barley α-amylase apoplast targeting presequence;
   9 - fusion with chloroplast targeting presequence;
   10 - fusion with chloroplast targeting presequence followed by His(6)-tag and enterokinase cleavage site (His-EK);
   11 - Bovine Serum Albumin (Sigma, A4503);
   12 - Bovine Serum Albumin (A4503, Sigma).

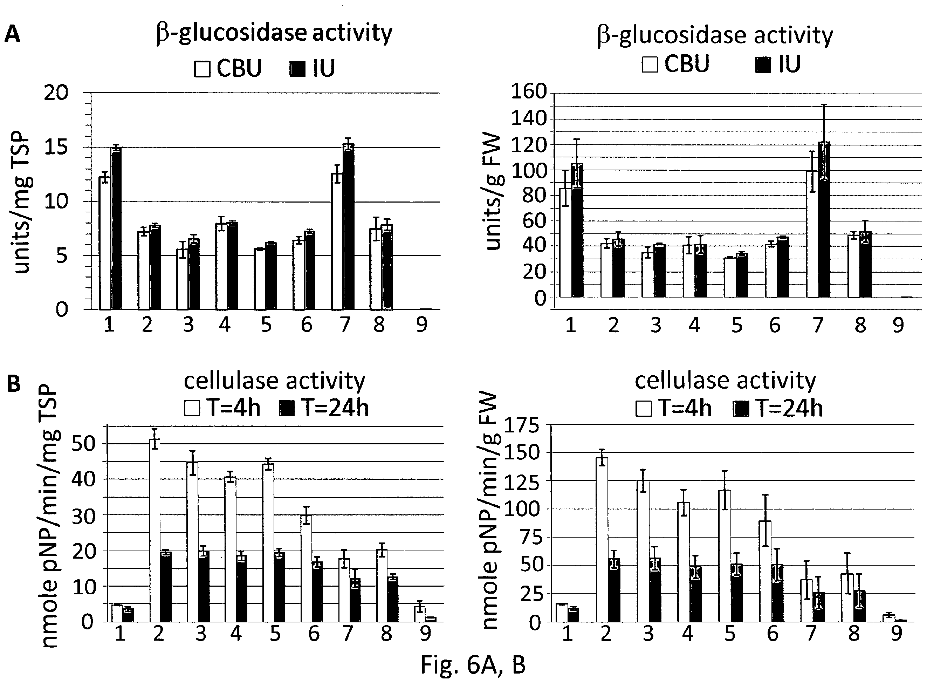

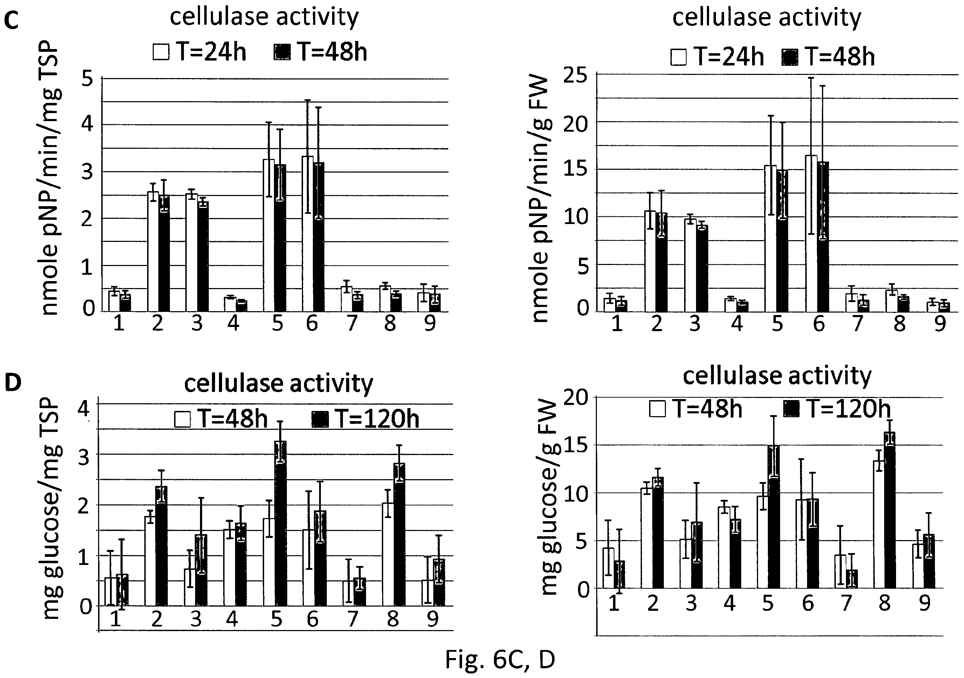

Fig. 6 shows the optimisation of transient expression of cellulases via screening cellulase fusions to different N-terminal sequences for highest enzymatic activity per mg TSP and grams fresh weight of plant tissue using TMV provectors.
   (A) β-glucosidase Bgl4, (*B*) endoglucanase E1, (*C*) exoglucanase CBHI, (*D*) exoglucanase E3. TSP (total soluble protein) extracts are prepared from plant material expressing the following cellulase fusions:
      1 - His(6)-tag and enterokinase cleavage site (His-EK);
      2 - rice amylase apoplast targeting presequence;
      3 - calreticulin apoplast targeting presequence;
      4 - calreticulin apoplast targeting presequence followed by His(6)-tag and enterokinase cleavage site (His-EK);
      5 - apple pectinase apoplast targeting presequence;
      6 - barley α-amylase apoplast targeting presequence;
      7 - chloroplast targeting presequence;
      8 - chloroplast targeting presequence followed by His(6)-tag and enterokinase cleavage site (His-EK);
      9 - TSP samples prepared from non-infected plant material.

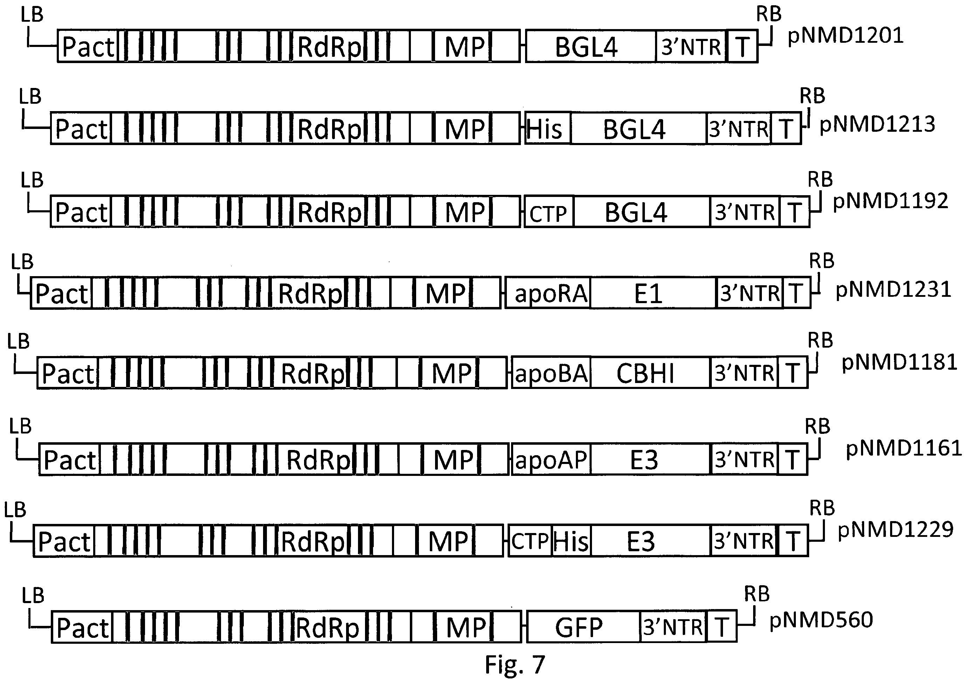

Fig. 7 shows schematically TMV-based assembled vectors used for the preparative large-scale production of cell wall degrading enzymes:
   pNMD1201 construct for cytosolic expression of β-glucosidase BGL4 from *Humicola grisea,* pNMD1213 construct for the expression of BGL4 with N-terminal His (6)-tag and enterokinase cleavage site (His-EK),
   pNMD1192 construct for the expression of BGL4 with chloroplast targeting presequence, pNMD1231 construct for the expression of endoglucanase E1 from *Acidothermus cellulolyticus* as a fusion with apoplast targeting presequence from rice amylase,
   pNMD1181 construct for the expression of exoglucanase 1 (CBHI) from *Trichoderma reesei* as a fusion with barley amylase apoplast targeting presequence,
   pNMD1161 construct for the expression of exocellulase E3 from *Thermobifida fusca* as a fusion with apple pectinase apoplast targeting presequence,
   pNMD1129 construct for the expression of E3 as a fusion with chloroplast targeting presequence followed by His (6)-tag and enterokinase cleavage site (His-EK). For the annotation of construct elements, see the Fig. 2 description.

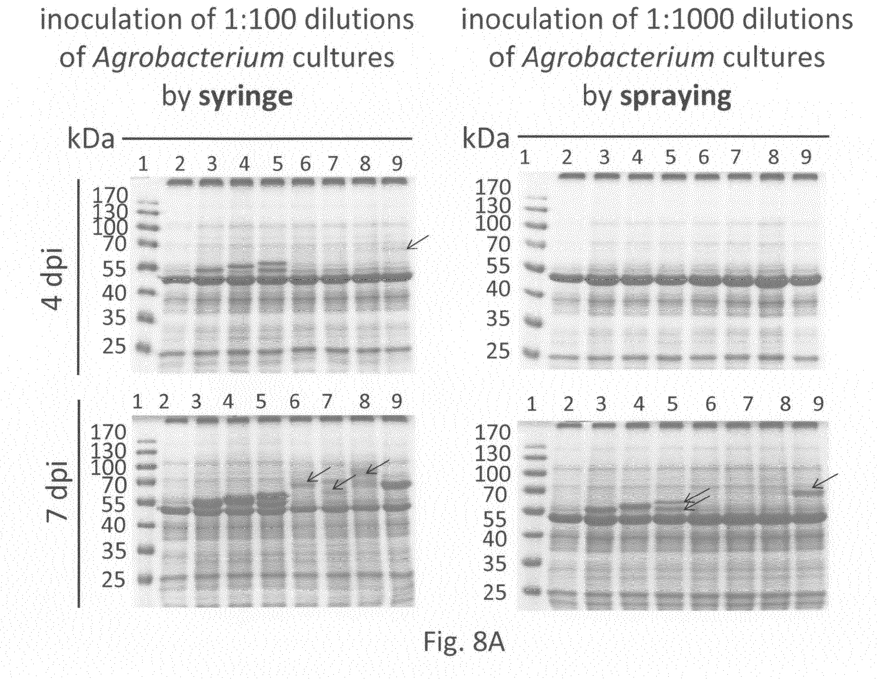

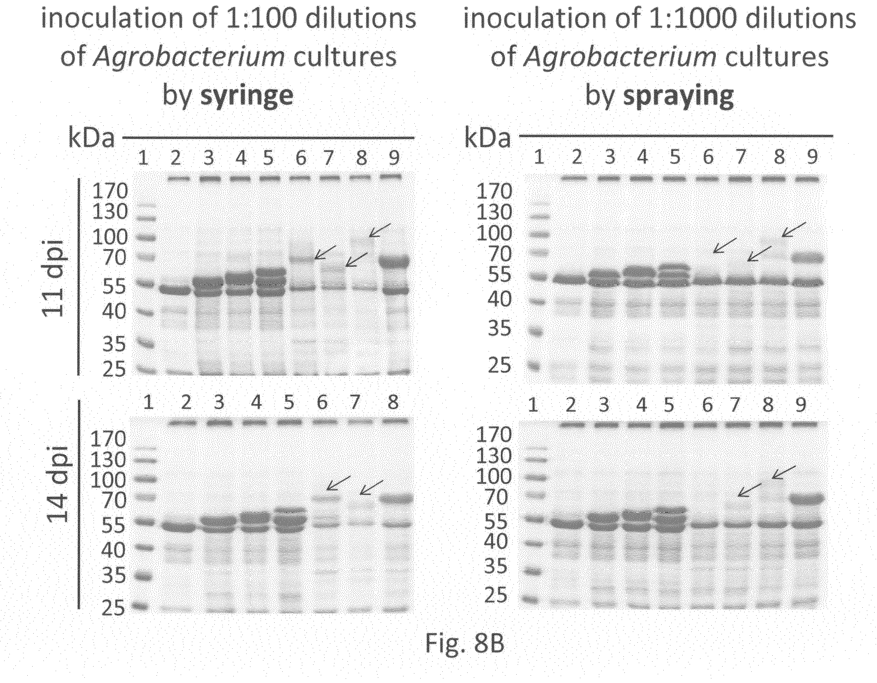

Fig. 8 depicts the analysis of different inoculation methods for highest cellulase expression of selected cellulase fusions: syringe infiltration vs spraying with suspension of agrobacteria harboring assembled TMV constructs. SDS-PAGE analysis of cellulase expression in crude extracts. Arrows indicate the bands of the cellulase expressed in cases where the cellulase is not strongly expressed compared to other bands.
   1 - PageRuler™ Prestained Protein Ladder (#SM0671, Fermentas);
   2 - uninfected leaf tissue;
   3 - β-glucosidase BGL4 from *Humicola grisea* expressed in cytosol (pNMD1201 construct);
   4 - Bgl4 expressed in cytosol as a fusion with His(6)-tag followed by enterokinase cleavage site (His-EK) (pNMD1213 construct);
   5 - Bgl4 targeted to chloroplast (pNMD1192 construct);
   6 - endoglucanase E1 from *Acidothermus cellulolyticus* targeted to the apoplast via fusion with rice amylase presequence (pNMD1231);
   7 - exoglucanase 1 (CBHI) from *Trichoderma reesei* targeted to apoplast via fusion with barley α-amylase presequence (pNMD1181);
   8 - exocellulase E3 from *Thermobifida fusca* targeted to apoplast via fusion with apple pectinase presequence (pNMD1161);
   9 - E3 targeted to chloroplast (chloroplast targeting presequence followed by His(6)-tag and enterokinase cleavage site (His-EK)) (pNMD1129).

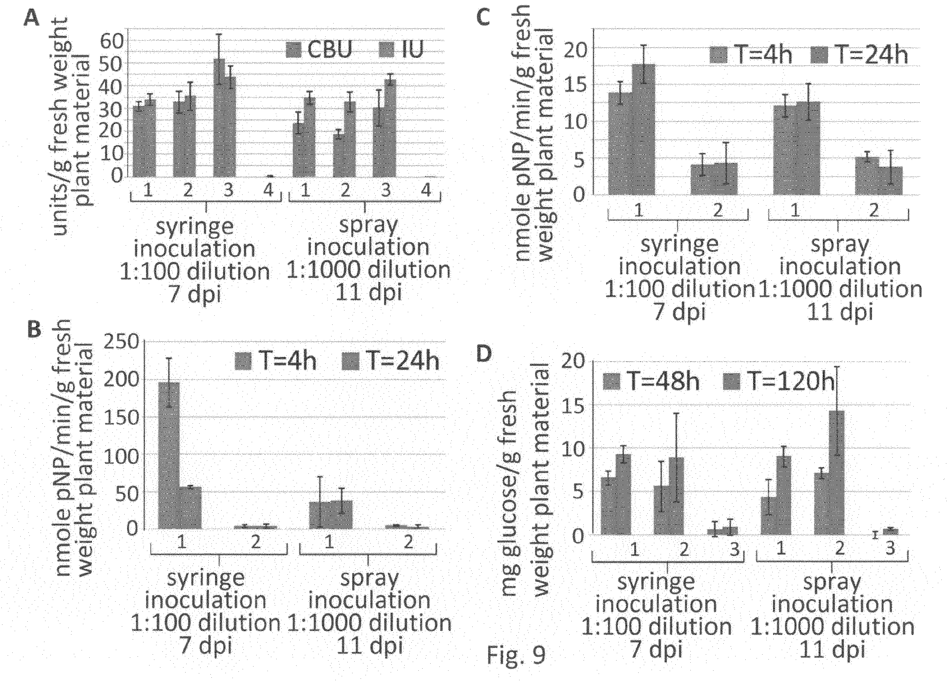

Fig. 9 shows results of a comparison of different inoculation methods for the highest cellulase expression of selected cellulase fusions: syringe infiltration vs. spraying with suspension of agrobacteria harboring assembled TMV constructs. Analysis of the enzymatic activity for the *Nicotiana benthamiana* leaves inoculated using a syringe (7 dpi, 1:100 dilution of agrofbacterial culture OD600=1.3) and by spraying (11 dpi, 1:1000 dilution of agrobacterialagrofbacterial culture OD600=1.3).
   (A) β-glucosidase BGL4 from *Humicola grisea* Bgl4. 1 - Bgl4 expressed in cytosol (pNMD1201); 2 - Bgl4 expressed in cytosol as a fusion with His(6)-tag followed by enterokinase cleavage site (His-EK) (pNMD1213); 3 - Bgl4 targeted to chloroplast (pNMD1192); 4 - uninfected leaf tissue.
   (B) endoglucanase E1 from *Acidothermus cellulolyticus.* 1 - E1 fusion with rice amylase presequence (pNMD1231); 2 - uninfected leaf tissue.
   (C) Exoglucanase 1 (CBHI) from *Trichoderma reesei.* 1 - CBHI fusion with barley α-amylase presequence (pNMD1181); 2 - uninfected leaf tissue.
   (*D*) Exocellulase E3 from *Thermobifida fusca.* 1 - E3 fusion with apple pectinase presequence (pNMD1161); 2 - E3 fusion with chloroplast targeting presequence followed by His(6)-tag and enterokinase cleavage site (His-EK) (pNMD1129); 3 - uninfected leaf tissue.

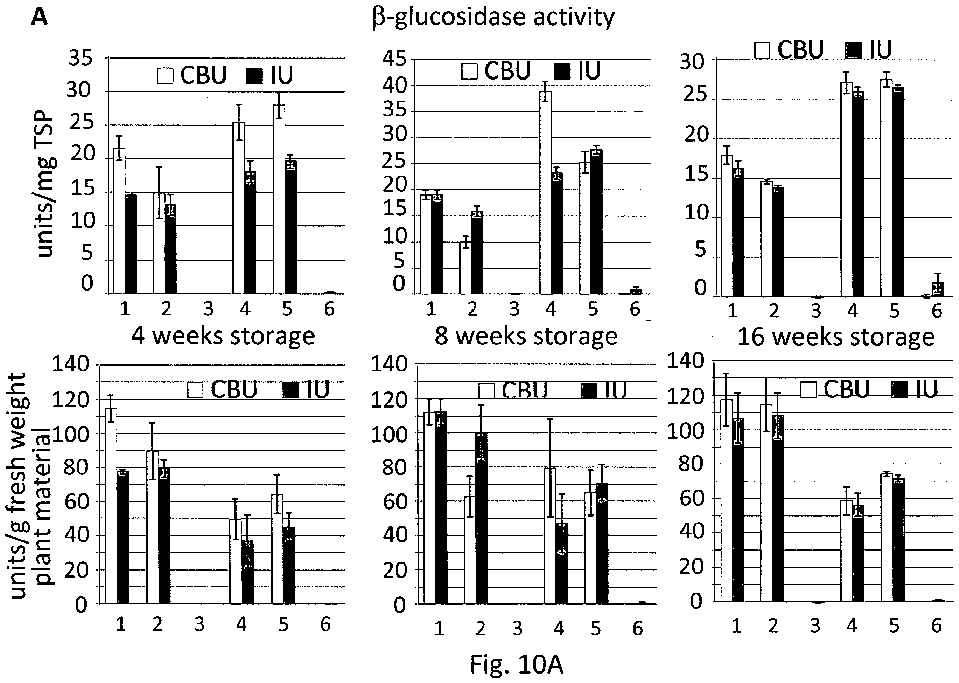

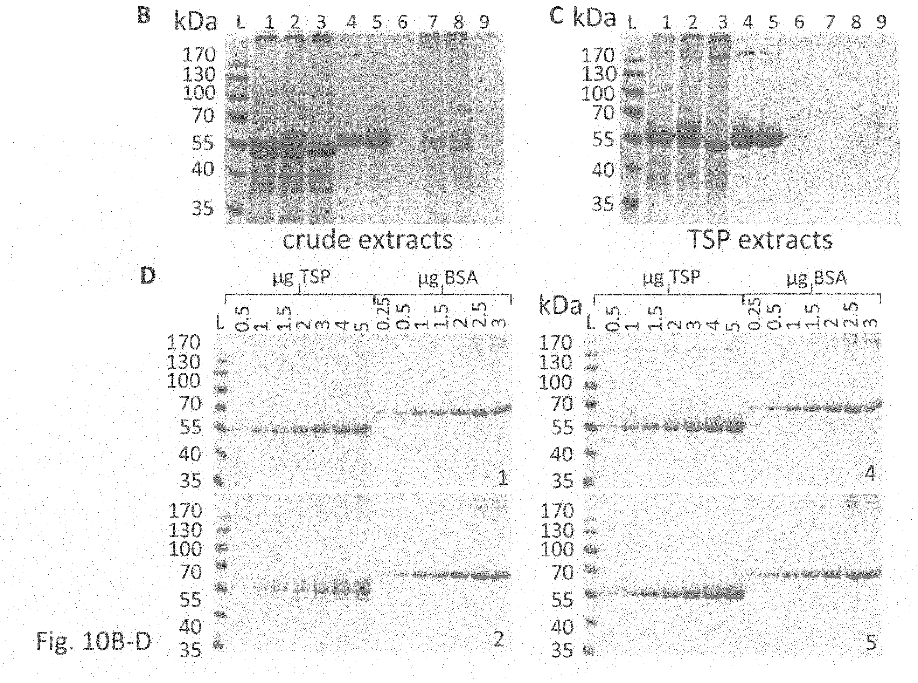

Fig. 10 shows results of an analysis of storage conditions for plant material expressing selected *Humicola grisea* β-glucosidase Bgl4 cellulase fusions.
   (*A*) Enzymatic activity of TSP samples containing *Humicola grisea* β-glucosidase Bgl4 fusions extracted from plant material and stored for different periods of time at different conditions; analysis of crude protein extracts (*B*) and TSP extracts (*C*) by SDS-PAGE with Coomassie staining;
   (*D*) estimation of percentage of TSP of recombinant Bgl4 fusions (4 weeks storage time). L - PageRuler™ Prestained Protein Ladder (#SM0671, Fermentas);
      1 to 3 - plant material stored at -80°C;
      4 to 6 - plant material stored as a silage;
      7 to 9 - plant material stored as a dry mass;
      1, 4 and 7 - plant material expressing cytosolic Bgl4;
      2, 5 and 8 - plant material expressing chloroplast targeted Bgl4;
      3, 6 and 9 - non-infected plant material.

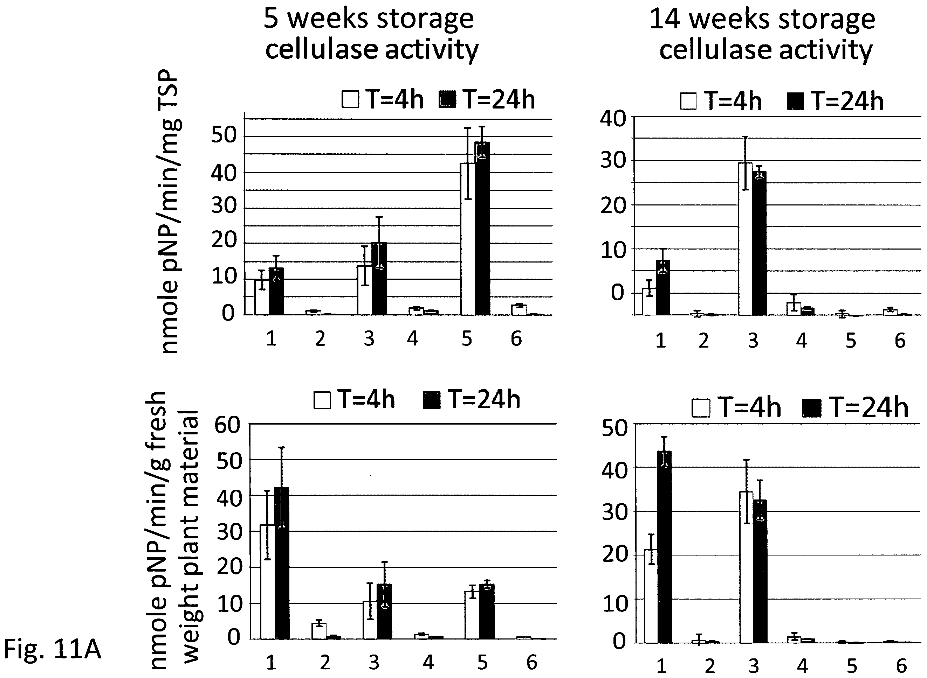

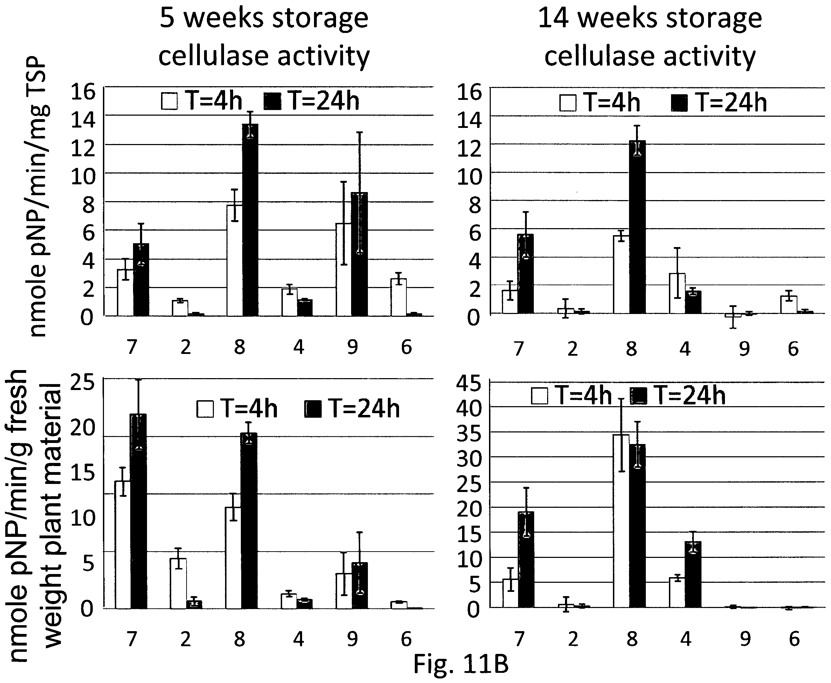

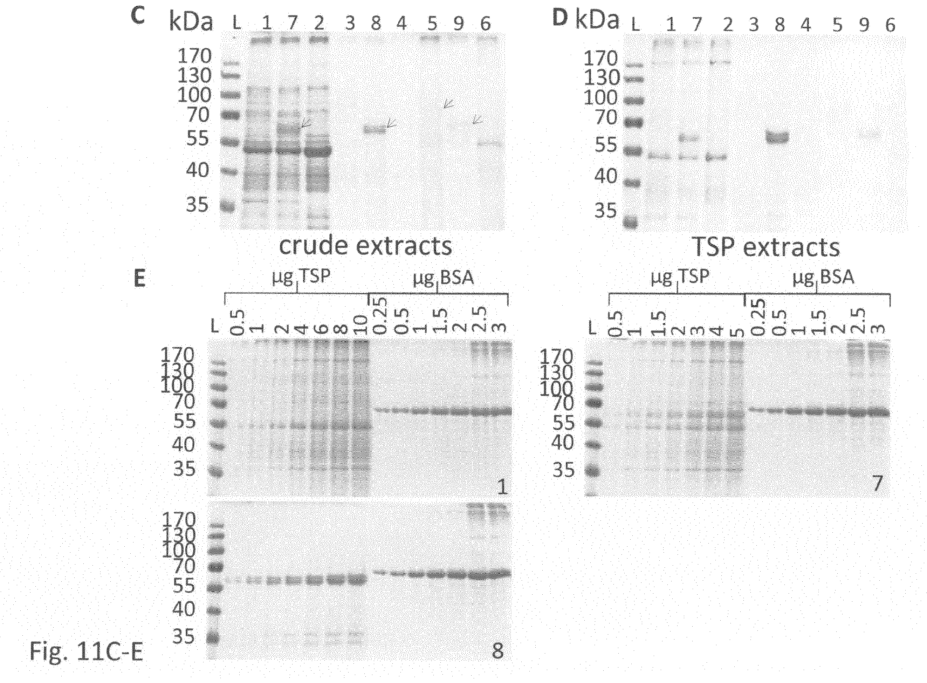

Fig. 11 shows the results of an analysis of storage conditions for plant material expressing selected *Acidothermus cellulolyticus* endoglucanase E1 and *Trichoderma reesei* exoglucanase CBHI fusions.
   (*A*) Enzymatic activity of TSP samples containing endoclucanase E1 fusions extracted from plant material and stored for different periods of time (left: 5 weeks; right: 14 weeks) at different conditions. 1, 3 and 5 - plant material expressing E1 from *Acidothermus cellulolyticus* targeted to the apoplast via fusion with rice amylase presequence (pNMD1231); 2, 4 and 6 - uninfected plant material;
   (*B*) Enzymatic activity of TSP samples containing containing *Trichoderma reesei* exoglucanase CBHI fusions extracted from plant material stored for different periods of time at different conditions. 7, 8 and 9 - plant material expressing apoplast targeted CBHI (fusion with barley α-amylase presequence, pNMD1181 construct); 2, 4 and 6 - non-infected plant material;
      Analysis of crude protein extracts (*C*) and TSP extracts (*D*) using SDS-PAGE;
   (*E*) estimation of percentage of TSP of recombinant E1 and CBHI fusions (4 weeks storage time);
      1, 2 and 7 - plant material stored at -80°C;
      3, 4 and 8 - plant material stored as silage;
      5, 6 and 9 - plant material stored as dry mass.

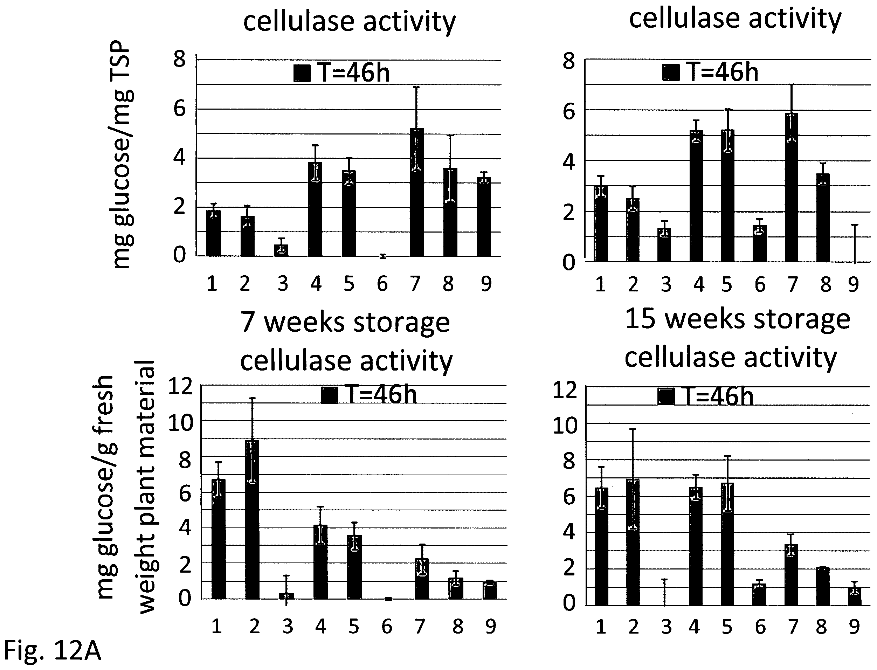

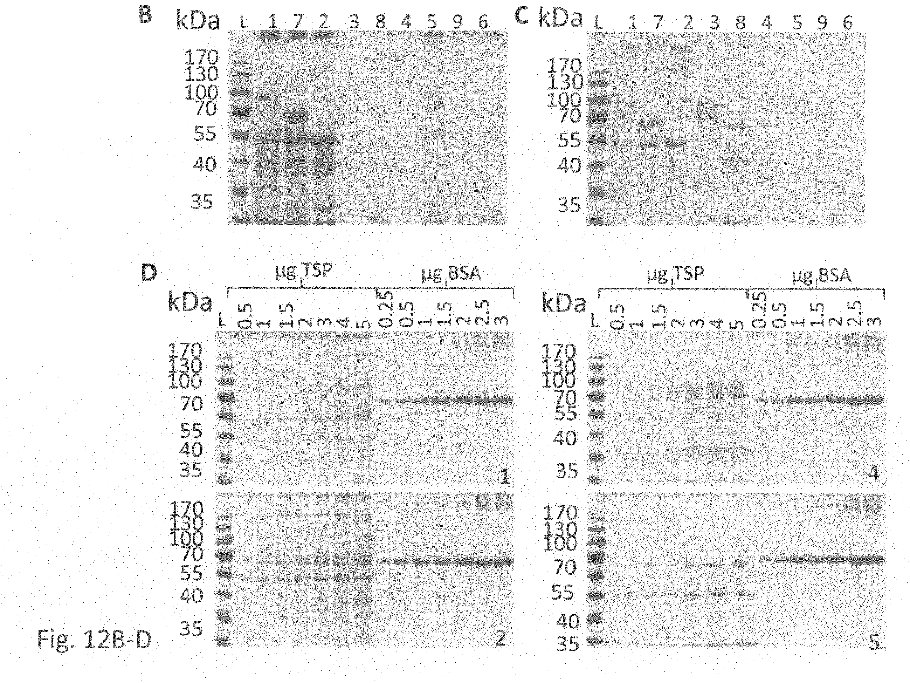

Fig. 12 shows the analysis of storage conditions for plant material expressing selected *Thermobifida fusca* exocellulase E3 fusions.
   (*A*) Enzymatic activity of TSP samples containing exoglucanase E3 fusions extracted from plant material stored for different periods of time at different conditions, 1, 4 and 7 - plant material expressing E3 targeted to apoplast with apple pectinase TP; 2, 5 and 8 - plant material expressing chloroplast TP-His-EK-E3 fusion; 3, 6 and 9 - uninfected plant material. Analysis of crude protein extracts (*B*) and TSP extracts (*C*) by SDS-PAGE with Coomassie staining (7 weeks storage time); (*E*) Estimation of percentage of TSP of recombinant E3 fusions (7 weeks storage time).
      1, 2 and 7 - plant material stored at -80°C;
      3, 4 and 8 - plant material stored as a silage;
      5, 6 and 9 - plant material stored as a dry mass.

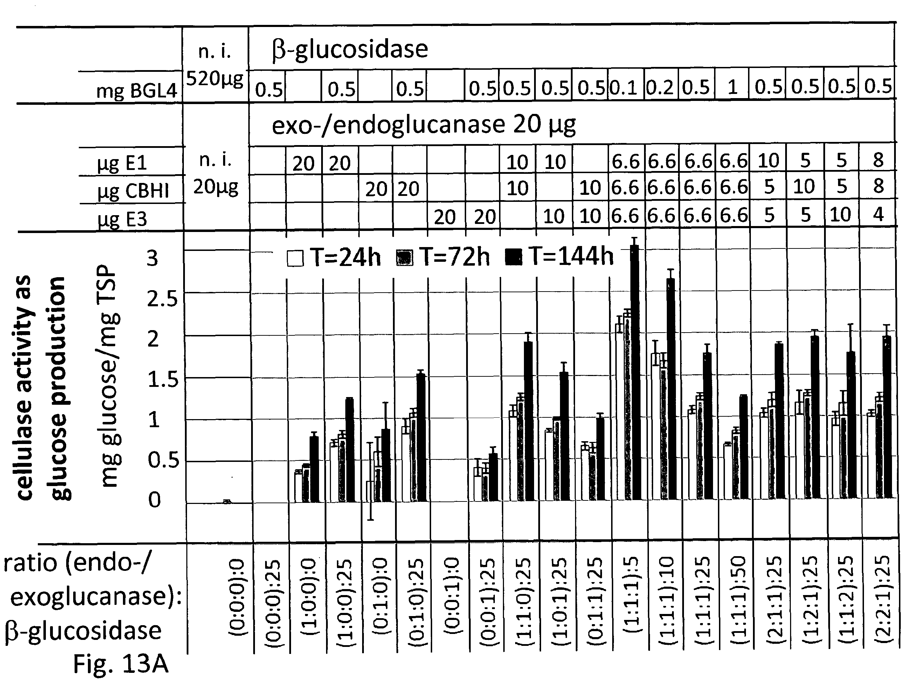

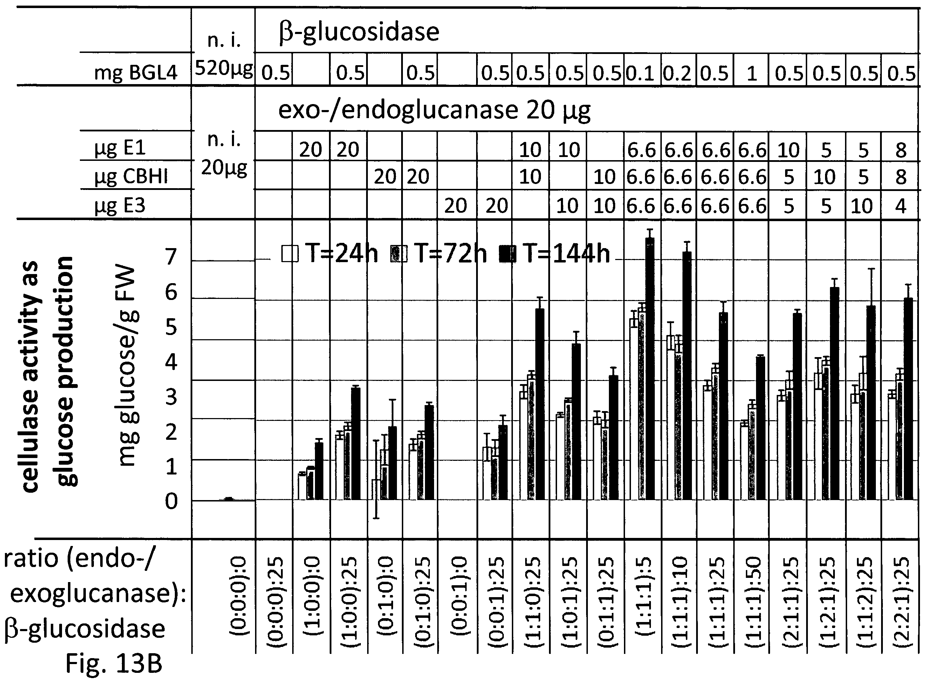

Fig. 13 shows results of screening for optimal ratios between plant-produced cellulases providing most efficient conversion of cellulose to glucose using a mixture of TSP samples containing recombinant β-glucosidase BGL4, endoglucanase E1, exoglucanase 1 (CBHI) and exocellulase E3. (*A*) Enzymatic activity is represented in mg glucose/mg TSP of enzyme-containing plant extracts. (*B*) Enzymatic activity is represented in mg glucose/g fresh weight of enzyme containing plant material. β-glucosidase BGL4 from *Humicola grisea* expressed in cytosol (pNMD1201), apoplast targeted endoglucanase E1 from *Acidothermus cellulolyticus* expressed as a fusion with rice amylase presequence (pNMD1231), apoplast targeted exoglucanase 1 (CBHI) from *Trichoderma reesei* expressed as a fusion with barley a-amylase presequence (pNMD1181) and chloroplast targeted exocellulase E3 from *Thermobifida fusca* (pNMD1129) were used for the screening.

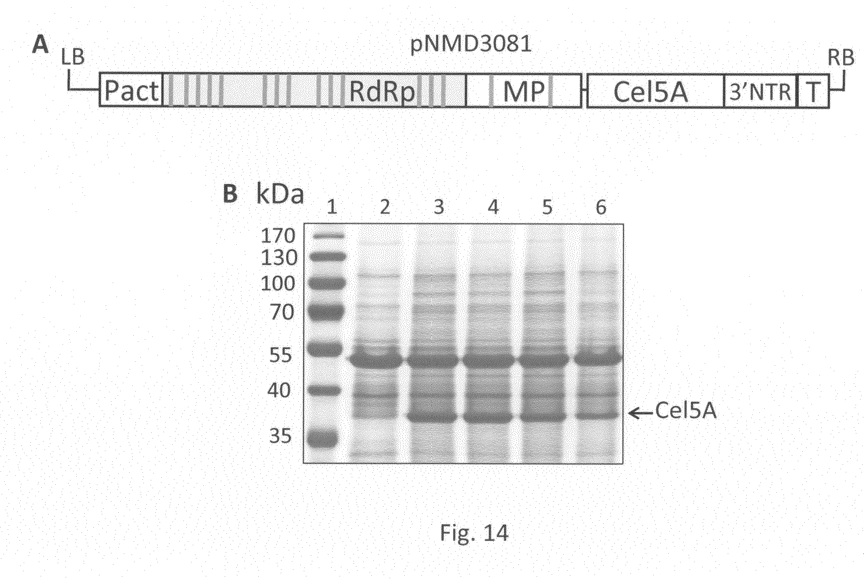

Fig. 14. shows the plasmid map of the TMV-based vector for the expression of cytosolic endoglucanase Cel5A from *Thermotoga maritima* (A) and Coomassie-stained SDS protein gel loaded with crude extracts, corresponding to 1.5 mg fresh weight, from *Nicotiana benthamiana* leaf tissue of plants transfected by *Agrobacterium tumefaciens* ICF320 strain carrying pNMD3081 construct (B). The lanes are as follows:
   1 - PageRulerTM Prestained Protein Ladder (#SM0671, Fermentas);
   2 - uninfected leaf tissue;
   3 - leaf tissue harvested at 11 days post spraying with 10-2 dilution of agrobacterial culture;
   4 - leaf tissue harvested at 11 days post spraying with 10-3 dilution of agrobacterial culture;
   3 - leaf tissue harvested at 14 days post spraying with 10-4 dilution of agrobacterial culture;
   4 - leaf tissue harvested at 18 days post spraying with 10-5 dilution of agrobacterial culture.

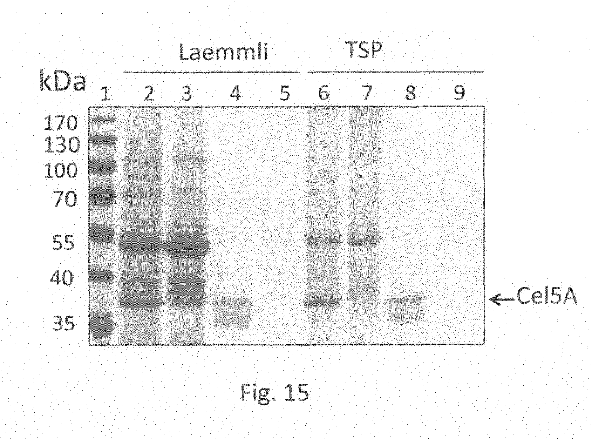

Fig. 15 shows the SDS-PAGE analysis of plant biomass containing heterologically expressed endoglucanase Cel5A from *Thermotoga maritima* upon storage as silage. For the expression of endoglucanase Cel5A, *Nicotiana benthamiana* plants were transfected by *Agrobacterium tumefaciens* ICF320 strain carrying the pNMD3081 construct using spraying with 1000-fold dilutions. Upon harvest at 11 dpi, plant material was stored at -80°C or as silage for 12 weeks.
   A Coomassie-stained SDS protein gel was loaded with crude Laemmli buffer extracts (Laemmli, corresponding to 1.5 mg fresh weight), or total soluble extracts (TSP, 5 µg) from *Nicotiana benthamiana* leaf tissue. The lanes are as follows:
   1 - PageRuler™ Prestained Protein Ladder (#SM0671, Fermentas);
   2 - Cel5A-containing leaf biomass after frozen storage at -80°C;
   3 - uninfected leaf biomass after frozen storage at -80°C;
   4 - Cel5A-containing leaf biomass after the storage as a silage;
   5 - uninfected leaf biomass after the storage as a silage;
   6 - Cel5A-containing leaf biomass after frozen storage at -80°C;
   7 - uninfected leaf biomass after frozen storage at -80°C;
   8 - Cel5A-containing leaf biomass after the storage as a silage;
   9 - uninfected leaf biomass after the storage as a silage.

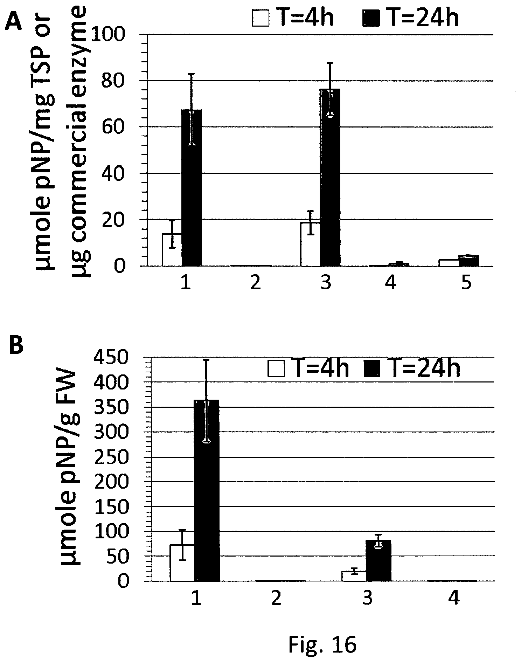

Fig. 16 shows the results of an analysis of storage conditions for plant material expressing cytosolic endoglucanase Cel5A from *Thermotoga maritima.* Upon harvest at 11 dpi, plant material was stored at -80°C or as silage for 12 weeks. Enzymatic activity is calculated per mg TSP (A) and per g fresh weight of plant material (B). The lanes are as follows:
   1 - Cel5A-containing leaf biomass after frozen storage at -80°C;
   2 - uninfected leaf biomass after frozen storage at -80°C;
   3 - Cel5A-containing leaf biomass after storage as a silage;
   4 - uninfected leaf biomass after storage as a silage;
   5 - cellulase (endo-1,4-β-D-glucanase) from *Trichoderma sp.* (Megazyme, E-CELTR).

### Detailed Description of the Invention

The process of producing a preparation of plant cell wall-degrading enzymes involves expression, in plants or parts thereof, the one or more than one plant cell wall-degrading enzyme. A part of a plant may be a plant part attached to a complete living plant such as leaves or may be detached from a complete plant, wherein the former possibility is preferred. It is generally desired to achieve high expression levels of these enzymes, since high expression levels can contribute to the stability of enzymatic activity of the expressed enzymes in the storing step. Methods of high level expression of proteins in plants have been established over the last two decades and are known to the plant biotechnologist. It is possible to use transgenic plants stably encoding the one or more than one plant cell wall-degrading enzyme in the nuclear or plastid genome. Inducible expression systems may be used to induce expression at a desired growth state of the plant. WO 2007137788 describes an inducible expression system using (viral) replicons expressed in transgenic plants. A method of controlling gene expression in plastids is disclosed in WO 2005/054481.

In one embodiment, transient expression is used, wherein plants or plant parts are transfected at a suitable point in time with a nucleic acid molecule (vector) comprising a nucleic acid construct containing a nucleic acid sequence encoding said one or more than one cell wall-degrading enzyme(s). This embodiment has the advantage that a decision on which enzyme to express can be delayed until shortly before the point of transfection, whereby the user can react on short notice to demands. The process of the invention therefore fits to modern market needs such as "trade on demand". The term "transient" means that no selection methods are used for selecting cells or plants transfected with the nucleic acid molecule (vector) or the nucleic acid construct in the background of non-transfected cells or plants using, e.g. selectable agents and selectable marker genes capable of detoxifying the selectable agents. As a result, the transfected nucleic acid is generally not stably introduced into plant chromosomal DNA. Instead, transient expression methods make use of the effect of transfection in the very plants transfected.

An established way of achieving high level expression is the use of self-replicating (viral) replicons containing a nucleotide sequence encoding said one or more cell wall-degrading enzyme(s). Plant viral expression systems have been described in many publications both for the use of DNA viral vectors and for the use of RNA viral vectors, such as in WO2008028661, WO2006003018, WO2005071090, WO2005049839, WO2006012906, WO02101006 or WO02068664 and many more publications are cited in these documents.

Various methods for introducing a nucleic acid molecule into a plant or plant part for transient expression are known. Agrobacteria can be used for transfecting plants with a DNA molecule (vector) or construct of interest e.g. by agroinfiltration. A system and method for large scale infiltration of plants by agrobacteria is described in WO2009095183. In another embodiment, plants or plant parts are sprayed with an aqueous suspension containing cells of an *Agrobacterium* strain, which is well suitable for large scale applications to many plants such as to plants on a farm field. Such spray transfection processes are described in detail in WO2012/019660.

The *Agrobacterium* strain may belong to the species *Agrobacterium tumefaciens* or *Agrobacterium rhizogenes* that are commonly used for plant transformation and transfection and which are known to the skilled person from general knowledge. The *Agrobacterium* strain comprises a DNA molecule (Ti-plasmid) comprising a nucleic acid construct containing a DNA sequence of interest. The DNA sequence of interest encodes one or more than one enzyme to be expressed in plants. The nucleic acid construct is typically present in T-DNA of Ti-plasmids for introduction of the nucleic construct into plant cells by the secretory system of the *Agrobacterium* strain. On at least one side or on both sides, the nucleic acid construct is flanked by a T-DNA border sequence for allowing transfection of said plant(s) and introduction into cells of said plant of said nucleic acid construct. In the nucleic acid construct, the DNA sequence of interest is present such as to be expressible in plant cells. For this purpose, the DNA sequence of interest may be, in said nucleic acid construct, under the control of a promoter active in plant cells. Examples of the DNA sequence of interest are a DNA sequence encoding a DNA viral replicon or an RNA viral replicon or a gene to be expressed. The gene encodes the enzyme to be expressed in cells of the plant(s). Also the viral replicons encode the enzyme to be expressed in cells of the plant(s). The DNA construct may comprise, in addition to the DNA sequence of interest, other sequences such as regulatory sequences for expression of the DNA sequence of interest, such as a transcription promoter and terminator. *Agrobacterium-*mediated gene transfer and vectors therefor are known to the skilled person, e.g. from the references cited above or from text books on plant biotechnology such as Slater, Scott and Fowler, Plant Biotechnology, second edition, Oxford University Press, 2008.

As used herein, the term "promoter active in plant cells" means a DNA sequence that is capable of controlling (initiating) transcription in a plant cell. This includes any promoter of plant origin, but also any promoter of non-plant origin which is capable of directing transcription in a plant cell, i.e., certain promoters of viral or bacterial origin such as the CaMV35S (Harpster et al. (1988) Mol Gen Genet. 212(1):182-90, the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al. (1996) Plant Cell 8(1): 15-30), stem-specific promoters (Keller et al., (1988) EMBO J. 7(12): 3625-3633), leaf specific promoters (Hudspeth et al. (1989) Plant Mol Biol. 12: 579-589), mesophyl-specific promoters (such as the light-inducible Rubisco promoters), root-specific promoters (Keller et al. (1989) Genes Dev. 3: 1639-1646), tuber-specific promoters (Keil et al. (1989) EMBO J. 8(5): 1323-1330), vascular tissue specific promoters (Peleman et al. (1989) Gene 84: 359-369), stamen-selective promoters (WO 89/10396, WO 92/13956), dehiscence zone specific promoters (WO 97/13865) and the like. For transient expression, constitutive promoters, i.e. promoters that are not developmentally regulated, are preferably used. However, constitutive promoters may be tissue-specific or organ-specific. Preferred promoters are those used in the Examples described below.

Herein, the term "construct" means a recombinant construct encoding the one or more than one cell wall-degrading enzyme.

In embodiments wherein strong expression of a protein is desired, the nucleic acid construct may encode a viral vector (referred to herein as "replicon") that can replicate in plant cells. In order to be replicating, the replicon contains an origin of replication that can be recognized by a nucleic acid polymerase present in plant cells, such as by the viral polymerase expressed from the replicon. In case of RNA viral vectors (referred to as "RNA replicons"), the replicons may be formed by transcription under the control of a promoter active in plant cells, from the DNA construct after the latter has been introduced into plant cell nuclei. In case of DNA replicons, the replicons may be formed by recombination between two recombination sites flanking the sequence encoding the viral relicon in the DNA construct, e.g. as described in WO00/17365 and WO 99/22003. If the replicon are encoded by the DNA construct, RNA replicons are preferred. Use of DNA and RNA viral vectors (DNA or RNA replicons) has been extensively described in the literature over the years. Some examples are the following patent publications: WO2008028661, WO2007137788, WO 2006003018, WO2005071090, WO2005049839, WO02097080, WO02088369, WO02068664. An example of DNA viral vectors are those based on geminiviruses. For the present invention, viral vectors or replicons based on plant RNA viruses, notably those based on plus-sense single-stranded RNA viruses are preferred. Accordingly, the viral replicon may be a plus-sense single-stranded RNA replicon. Examples of such viral vectors are tobacco mosaic virus (TMV) and potex virus X (PVX). Potexvirus-based viral vectors and expression systems are described in EP2061890 or WO2008/028661. Many other plant viral replicons are described in the patent publications mentioned above.

In one embodiment, step (i) comprises spraying aerial parts of said plant with an aqueous suspension containing cells of an *Agrobacterium* strain comprising a DNA molecule comprising a nucleic acid construct encoding a DNA or, preferably, an RNA replicon encoding said one or more than one cell wall-degrading enzyme.

The aqueous suspension that may be used for spraying plants or plant parts in the process of the invention may have a concentration of *Agrobacterium* cells of at most 1.1·10⁹ cfu/ml, which corresponds approximately to an *Agrobacterium* culture in LB-medium of an optical density at 600 nm of 1. Due to the high transfection efficiency achievable, notably if spraying is done with the use of an abrasive and/or a surfactant or wetting agent (see below), much lower concentrations may, however, be used, which allows treatment of many plants such as entire farm fields without the need for huge fermenters for *Agrobacterium* production. Thus, the concentration may be at most 2.2·10⁷ cfu/ml, preferably at most 1.1·10⁷ cfu/ml, more preferably at most 4.4·10⁶ cfu/ml. In one embodiment, the concentration is at most 1.1·10⁶ cfu/ml of the suspension. For avoiding determination of cell concentrations in terms of cfu/ml, concentrations of agrobacterial suspensions are frequently assessed by measuring the apparent optical density at 600 nm using a spectrophotometer. Herein, the concentration of 1.1·10⁷ cfu/ml corresponds to a calculated optical density at 600 nm of 0.01, whereby the calculated optical density is defined by a 100-fold dilution with water or buffer of a suspension having an optical density of 1.0 at 600 nm. Similarly, the concentrations of 4.4·10⁶ cfu/ml and 1.1·10⁶ cfu/ml correspond to a calculated optical density at 600 nm of 0.004 and 0.001, respectively, whereby the calculated optical densities are defined by a 250-fold or 1000-fold, respectively, dilution with water or buffer of a suspension having an optical density of 1.0 at 600 nm.

The aqueous suspension containing cells of an *Agrobacterium* strain used for spraying the plants or parts of a plant may contain at least one abrasive suspended in said suspension for improving transfection efficiency. The abrasive that may be used is a particulate material that is essentially insoluble in the aqueous suspension of *Agrobacterium* cells. The abrasive is believed to weaken, notably if used together with a wetting agent, the surface of plant tissue such as leaves, and thereby facilitates penetration of *Agrobacterium* cells into the intercellular space of plant tissue. As a result, the transfection efficiency is high.

The particulate material to be used as the abrasive may be carrier material as commonly used as carriers in wettable powder (WP) of pesticide formulations. In the context of wettable powders, these carriers are also referred to in the field of pesticide formulations as "fillers" or "inert fillers". Wettable powder formulations are part of the general knowledge in the field of plant protection. Reference is made to the handbook PESTICIDE SPECIFICATIONS, "Manual for Development and Use of FAO and WHO Specifications for Pesticides", edited by the World Health Organisation (WHO) and the FOOD and Agriculture Organization of the United States, Rome, 2002, ISBN 92-5-104857-6. Wettable powder formulations for plant protection are for example described in EP 1810569, EP1488697, EP1908348 and EP0789510. The abrasive may be a mineral material, typically an inorganic material. Examples of such carrier materials are diatomaceous earth, talc, clay, calcium carbonate, bentonite, acid clay, attapulgite, zeolite, sericite, sepiolite or calcium silicate. It is also possible to use quartz powder such as the highly pure quartz powder described in WO02/087324. Preferred examples are silica, such as precipitated and fumed hydrophilic silica, and carborundum. The abrasive properties of diluents or fillers such as silica used in wettable powders are known (see "Pesticide Application Methods" by G.A. Matthews, third edition, Blackwell Science, 2000, on page 52 thereof).

As commercial products of particulate inorganic materials for use as abrasives, the hydrophilic silica Sipernat™ 22S and Sipernat™ 50 S, manufactured by Evonic Degussa may be mentioned. Other products are "Hi-Sil™ 257", a synthetic, amorphous, hydrated silica produced by PPG Industries Taiwan Ltd. or "Hubersorb 600™", a synthetic calcium silicate, manufactured by Huber Corporation. A commercial sub-micron sized silica is Hi-Sil™ 233 (PPG Industries) having an average particle size of around 0.02 µm.

The abrasive may have a median particle size between 0.01 and 40, preferably between 0.015 and 30, more preferably between 0.05 and 30, even more preferably between 0.1 and 30, even more preferably between 0.1 and 20, even more preferably between 0.5 and 20, and most preferably between 1.0 and 16 µm. In one embodiment, the median particle size is between 0.015 and 1 or between 0.02 and 0.5 µm. The median particle size is the volume median particle size that can be measured by laser diffraction using a Mastersizer™ from Malvern Instruments, Ltd. In order to avoid clogging of spraying nozzles, the maximum particle size of the largest particles contained in the abrasive should be at most 45 µm, preferably at most 40 µm, which may be determined by sieving. This condition is considered fulfilled, if the sieve residue is below 1.5 % by weight (following ISO 3262-19). The abrasive may have a D90 value of at most 40 µm, preferably of at most 30 µm, measured by laser diffraction as described above. Typically, the particle sizes above relate to primary particle sizes. The content of the abrasive in the aqueous suspension may be between 0.01 and 3, preferably between 0.02 and 2, more preferably between 0.05 and 1 and even more preferably between 0.1 and 0.5 % by weight of said suspension.

The aqueous suspension usable for spraying parts of the plants preferably contains an agricultural spray adjuvant. The spray adjuvant may be a surfactant or wetting agent. The surfactant or wetting agent has multiple advantages. It reduces the surface tension of the water of the aqueous suspension and makes the waxy surface of plant leaves more permeable for agrobacteria. It further improves the stability of the suspension and reduces settling of the abrasive in the suspension. Surfactants usable are not particularly limited. However, nonionic surfactants are preferred. A measurement frequently used to describe surfactants is the HLB (hydrophilic/lipophilic balance). The HLB describes the ability of the surfactant to associate with hydrophilic and lipophilic compounds. Surfactants with a high HLB balance associate better with water soluble compounds than with oil soluble compounds. Herein, the HLB value should be 12 or greater, preferably at least 13. As noninionic surfactants, organo-silicone surfactants such as polyalkyleneoxide-modified heptamethyltrisiloxane are most preferred for use in the present invention. A commercial product is Silwet L77™ spray adjuvant from GE Advanced Materials.

The aqueous suspension of agrobacteria may be produced as follows. In one method, the *Agrobacterium* strain to be used in the process of the invention is inoculated into culture medium and grown to a high cell concentration. Larger cultures may be inoculated with small volumes of a highly concentrated culture medium for obtaining large amounts of the culture medium. Agrobacteria are generally grown up to a cell concentration corresponding to an OD at 600 nm of at least 1, typically of about 1.5. Such highly concentrated agrobacterial suspensions are then diluted to achieve the desired cell concentration. For diluting the highly concentrated agrobacterial suspensions, water is used. The water may contain a buffer. The water may further contain the surfactant mentioned above. Alternatively, the concentrated agrobacterial suspensions may be diluted with water, and any additives such as the surfactant and the optional buffer substances are added after or during the dilution process. The abrasive may be added before, during or after dilution. It is however preferred to agitate the suspension during addition of the abrasive to uniformly disperse the abrasive in the agrobacterial suspension. The step of diluting the concentrated agrobacterial suspension may be carried out in the spray tank of the sprayer used for spraying the diluted suspensions.

The sprayer to be used for transfecting plants or plant parts in step (i) of the process of the invention mainly depends on the number of plants or the area to be sprayed. For one or a small number of plants to be sprayed, pump sprayers as widely used in household and gardening can be used. These may have volumes of the spray tank of between 0.5 and 2 liters. For applications on a medium scale, manually operated hydraulic sprayers such as lever-operated knapsack sprayers or manually operated compression sprayers may be used. However, the high transfection efficiency achieved in the invention has its full potential in the transfection of many plants such as plants growing on a farm field or in a greenhouse. For this purpose, power-operated hydraulic sprayers such as tractor-mounted hydraulic sprayers equipped with spray booms can be used. Aerial application techniques using helicopters or airplanes are also possible for large fields. All these types of sprayers are known in the art and are described for example in the book "Pesticide Application Methods" by G.A. Matthews, third edition, Blackwell Science, 2000. In order to ensure a homogeneous suspension in the spray tanks of the sprayers, small or medium size sprayers may be shaken at regular intervals or continuously during spraying. Large sprayers such as the tractor-mounted sprayers should be equipped with an agitator in the spray tank.

Considering the presence of agrobacterial cells and optionally abrasive in the suspensions to be sprayed, sprayers used should produce spray of a droplet size at least of fine spray. Also, medium spray or coarse spray in the classification of sprays used in the above-mentioned book by G.A. Matthews, page 74, may be used. The main purpose of the spraying is wetting of plant tissue with the suspension. Thus, the exact droplet size is not critical. However, the transfection efficiency may be further improved by providing the spray to plant surfaces with increased pressure.

In another embodiment, the one or more than one cell-wall degrading enzyme is expressed in transgenic plants containing in their nuclear genome a recombinant construct comprising a recombinant expression cassette comprising a promoter active in plant cells and, operably linked thereto, a coding sequence encoding a plant cell wall-degrading enzyme. Such aspects are described for example in WO 98/16651 or WO 2007/100897

In the process of producing the preparation, the one or more than one cell wall degrading enzyme may be expressed in multi-cellular plants, notably, higher plants. Both monocot and dicot plants can be used. Common crop plants usable include alfalfa, barley, beans, canola, cowpeas, cotton, corn, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. The plant species preferred for practicing this invention include, but not restricted to, representatives of Gramineae, Compositeae, Solanaceae and Rosaceae. Preferred plants are plants that do not normally enter the food chain such as *Nicotiana* species such as *N. tabacum* and *N. benthamiana* may be used.

It is possible to express the one or more than one cell wall-degrading enzyme in the cytosol of said plants or plant parts. In this case, no signal peptide directing the enzyme into a particular compartment is added to the enzyme. A natural signal peptide present in the enzyme for secreting the enzyme from the native organism from which the enzyme originates may be omitted for expression in the process of the present invention. However, since signal peptides of secretory proteins from bacteria are generally not functional or of low reliability in plants, it is not necessary to omit the natural signal peptide when enzymes from bacteria or archaea are expressed in plants in the process of this invention.

In one embodiment, the enzyme expressed in the plants or parts thereof is targeted to a desired cell compartment. Preferred compartments are plastids or the apoplast. Targeting to these compartments has the additional advantage that the expressed and targeted cell wall-degrading enzyme(s) is (are) removed from the cytoplasm where most proteases reside, which can contributes to stability of the enzymes upon storage. Targeting of an enzyme expressed in a plant to a cell compartment can be achieved by fusing a suitable signal peptide to the enzyme to form a fusion protein comprising the signal peptide and the enzyme. Targeting of protein expressed in plants to the apoplast is known e.g. from WO03020938, and may be achieved by fusing a signal peptide (also referred to herein as "TP" for transit peptide) to the enzyme, wherein the signal peptide targets the enzyme into the endoplasmatic reticulum (ER) and the secretory pathway. The polypeptide capable of binding the fusion protein to a cell wall component used in WO03020938 may be omitted from the fusion protein of this reference when used in the processes of the present invention. All signal peptides of proteins known to be secreted or targeted by plants to the apoplast may be used for targeting the enzyme to the apoplast. Preferred examples are the signal peptides of tobacco calreticulin or of rice α-amylase. Further examples are the signal peptides of pectin methylesterase or of *N. tabacco* tyrosine and lysine rich protein (NtTLPR). A further example is the signal peptide of apoplastic isoperoxidase from zucchini (APRX) (Carpin et al., 2001, The Plant Cell, 13, 511-520). The signal peptide has to be comprised in the fusion protein such that it is functional for said targeting. This condition may be fulfilled at any location in the fusion protein. In general, however, the signal peptide is positioned at the N-terminus of the enzyme for efficient targeting of the enzyme to the apoplast. EP 1 867 724 A2 describes production of cell wall-degrading enzymes in plants and targeting to the apoplast or plastids.

Targeting of plant-expressed proteins to plastids by fusing a plastid transit peptide to the protein or enzyme is also known from many publications such as WO2004101797 and references cited therein. Details of the cleavage sites of natural plastid-targeted plant proteins have been investigated (Gavel & Von Heijne 1990, FEBS Lett., 261, 455-458). Various chloroplast transit peptide (TP) are used in the examples of this document. Examples of publications describing expression in plants of cellulolytic enzymes and targeting to plastids are US 6,013,860; WO 98/11235 and US 2002/0062502.

The cell wall-degrading enzymes expressed and used in the processes of the present invention are generally enzymes heterologous to the plant or plant part in which they are expressed, i.e. the plant or its part does not produce the enzyme naturally. In many cases, the enzymes may originate from bacteria, archaea or from fungi. It is possible to optimize codon usage of genes encoding these enzymes for expression in plants, notably in the plant employed for expressing them. Codon optimization is a standard method for increasing expression yields. Codon-optimized genes and nucleic acids can be ordered from commercial sources such as Entelechon GmbH, Regensburg, Germany.

Herein, a cell wall-degrading enzyme is an enzyme that can degrade at least one polymeric component of plant cell walls or an oligomeric degradation product of such polymeric component. Plant cell walls contain cellulose, hemicellulose, β-1,4-glycan, pectin and lignin, all of which are polymers. Except for lignin, these polymers are polysaccharides. Enzymes capable of degrading plant cell wall polymers or oligomeric degradation products thereof are produced by several organisms that can use these polymeric compounds as carbon source or that grow on decaying plants. Generally, these are microorganisms belonging to bacteria, archaea and fungi, but also some protozoa and termites produce cellulases. Ruminants rely on cellulase-expressing microorganisms to break down cellulose. Since plant cell wall biopolymers are polymeric, they cannot be degraded within cells of these organisms. Accordingly, cell wall-degrading enzymes are generally secreted into the environment and degrade plant cell wall components, and/or oligomeric degradation products of plant cell wall components, in the extracellular medium. Small molecular degradation products, mostly di- or monosaccharides such as D-glucose, may be taken up by the organisms and used as nutrients.

Herein, the terms "oligomer" or "oligomeric degradation product" refer to compounds made up of at least 2 monomer moieties. Thus, as far as saccharidic oligomers or oligomeric degradation product of polysaccharides are concerned, disaccharides are oligosaccharides. As an example, cellobiose is an oligosaccharide.

In contrast to many intracellular proteins expression and degradation of which is tightly regulated by organisms, secretory cell wall degrading enzymes are optimized by evolution to be rather stable enzymes that can relatively well withstand a variety of environmental conditions in the extracellular medium in order to be effective for extracellular plant cell wall degradation. The inventors assume that these circumstances are the reason for the surprisingly high stability of the cell wall degrading enzymes upon storage in silage, as found when the present invention was made.

Regarding cell wall degrading enzymes, the invention is not limited, provided the one or more than one cell wall-degrading enzyme is selected from endoglucanases, exoglucanases, exocellulases, β-glucosidases, and combinations thereof. Generally, the one or more than one cell wall degrading enzyme is heterologous to the plant or part in which it is expressed in the invention. The enzymes are generally of microbial origin, notably of bacterial, archaebacterial or fungal origin. Modifications may be made to such enzymes using general methods of genetic engineering. Enzymes involved in degradation of any of the main plant cell wall components may be used. Thus, cellulolytic enzymes are used in the invention. Hemicellulases, pectinases and/or lignin degrading enzymes may additionally be used. In an important embodiment, cellulolytic enzymes, i.e. enzymes that cleave β-1,4-glycosidic bonds between glucose moieties in compounds containing β-1,4-glycosidically linked glucose moieties are used. Cellulolytic enzymes are also referred to herein as "cellulases". Cellulose consists of D-glucose units that are linked by β-1,4-glycosidic bonds, and the one or more cell wall degrading enzymes used in the invention are cellulolytic enzymes (cellulases). Several enzymes are involved in the enzymatic breakdown of cellulose, and cellulolytic enzymes comprises multiple groups including:
- endoglucanases (β-1,4-glucanglucanohydrolases, EC3.2.1.4) that randomly cleave internal (away from chain ends) β-1,4-glycosidic bonds of cellulose;
- exoclucanases or cellobiohydrolases (EC3.2.1.91) that produce cellobiose by attacking cellulose from reducing chain ends (cellobiohydrolase I) or by attacking cellulose from the non-reducing ends of cellulose chains (cellobiohydrolase II); and
- β-glucosidases (EC 3.2.1.21) that convert cellobiose to glucose.

There is an enormous body of literature on all aspects of cellulolytic enzymes from many different sources, and the prospect of deriving fuel from cellulose has led to a boost of research in this field over the last decade. In an interesting embodiment, enzymes derived from extremophilic organisms such as thermophiles or acidophiles are used, since these are of particularly high stability. Table 1 shows enzymes from each of the above listed cellulase groups that were used in the examples.

**Table 1. Cellulases used for transient expression**

| **No.** | **Name** | **EC number** | **Source organism** | **Accession number** | **Molecular weight, kDa** | **pl** | **pH optimum** | **Temperature optimum, °C** |
|---|---|---|---|---|---|---|---|---|
| 1 | Endoglucanase E1 | 3.2.1.4 | Acidothermus cellulolyticus | Swiss-Prot: P54583.1 | 60.7 | 6.59 | 5-6 | 81 |
| 2 | Endoglucanase Cel5A | 3.2.1.4 | Thermotoga maritima | PDB: 3MMW-D | 37.4 | 5.89 | 5 | 80 |
| 3 | Exoglucanase 1 (CBH I) | 3.2.1.91 | Trichoderma reesei | Swiss-Prot: P62694.1 | 54 | 4.65 | 5 | 45-50 |
| 4 | Exoglucanase 1 (CBHI) | 3.2.1.91 | Humicola grisea | GenBank: BAA09785.1 | 54.1 | 4.84 | 5 | 60 |
| 5 | Exocellulase E3 | 3.2.1.91 | Thermobifida fusca (bacteria) | GenBank: AAA62211.1 | 63.5 | 4.46 | 7-8 | 65 |
| 6 | β-glucosidase BGL4 | 3.2.1.21 | Humicola grisea | GenBank: BAA74958.1 | 54 | 5.26 | 6 | 55 |

Since enzymes from several groups are required to degrade cellulose to glucose, it is preferred to provide at least one enzyme from each of the above groups when plant cell wall-containing plant material is to be degraded. This may be achieved by expressing two or more such enzymes in one plant, or by expressing one enzyme per plant. In the latter case, two or more plants having expressed different cellulolytic enzymes can be mixed in a desired ratio before the storing step (iii) and stored together in step (iii). Alternatively, two or more silages each obtained from a plant having expressed an enzyme can be combined when performing a process of degrading plant cell wall components of plant materials. In one embodiment, a silage containing an endoglucanase, a cellobiohydrolase I and a cellobiohydrolase II is used. In another embodiment, a silage containing an endoglucanase, a cellobiohydrolase I, a cellobiohydrolase II, and a β-glucosidase is used.

Hemicelluloses are polysaccharidic heteropolymers such as arabinoxylans, present along with cellulose in almost all plant cell walls. Hemicellulose has a random, amorphous structure with little strength. It is easily hydrolyzed by dilute acid or base as well as many hemicellulase enzymes. Hemicellulose contains several different sugar monomers. For instance, besides glucose, sugar monomers in hemicellulose can include xylose, mannose, galactose, rhamnose, and arabinose. Hemicelluloses contain most of the D-pentose sugars. Xylose is generally the sugar monomer present in the largest amount, but mannuronic acid and galacturonic acid also tend to be present. Unlike cellulose, hemicellulose consists of shorter chains, 500-3,000 sugar units as opposed to 7,000 - 15,000 glucose molecules per polymer generally seen in cellulose. In addition, hemicellulose is a branched polymer, while cellulose is unbranched. Microbial xylanases usable are reviewed by Beg et al. in Applied Microbiology and Biotechnology, Vol. 56, No. 3-4, 326-338. Hemicellulose conversion is described for example by Saba in J. Ind. Microbiol. Biotechnol. (2003) 30: 279-291.

Pectin is another major cell wall polymer found in all land plants (Willats et al., 2001, Plant Mol Biol, 47, 9-27). Pectin is a complex polymeric network of polysaccharides like homogalacturonan and rhamnogalacturonan. The main monomeric component of this network is galacturonic acid and not glucose like in the case of cellulose. Enzymes for degrading pectin are known in the art. An example is polygalacturonase, the most extensively studied among the family of pectinolytic enzymes. Polygalacturonases (PGases) are pectinolytic enzymes that catalyze the hydrolytic cleavage of the polygalacturonic acid chain with the introduction of water across the oxygen bridge. The PGases involved in the hydrolysis of pectic substances are endo-PGase (E.C. 3.2.1.15) and exo-PGase (E.C. 3.2.1.67). Endo-PGases are widely distributed among fungi, bacteria and many yeasts. They are also found in higher plants and some plant parasitic nematodes. They have been reported from many microorganisms, including *Aureobasidium pullulans, Rhizoctonia solani* Kuhn, *Fusarium moniliforme, Neurospora crassa*, *Rhizopus stolonifer*, *Aspergillus* sp., Thermomyces lanuginosus, Peacilomyces clavisporus. Endo-PGases have also been cloned and genetically studied in a large number of microbial species. In contrast, exo-PGases occur less frequently. They have been reported in *Erwinia carotovora, Agrobacterium tumefaciens, Bacteroides thetaiotamicron, Alternaria mali Fusarium oxysporum, Ralstonia solanacearum, Bacillus* sp. For review see Jayani et al., 2005, Process Biochemistry, 40, 2931-2944. Enzymes disclosed therein may be used in the present disclosure.

Lignin degrading secretory enzymes such as lignin peroxidase are expressed by white and brown rot fungi, such as *Phanerochaete chrysosporium*, and degrade the polymeric lignin network by radical reactions catalyzed by peroxidases.

As indicated above with regard to cellulolytic enzymes, cell wall degrading enzymes from thermophilic microorganisms or other thermostable enzymes can be advantageous with regard to stability in the storing step of the processes of the invention as well as in the subsequent use for degrading plant material. This also applies to cell wall degrading enzymes other than cellulolytic enzymes. According to Vieille, Biotechnol Annu Rev. 1996;2:1-83, enzymes synthesized by thermophiles (organisms with optimal growth temperatures > 60°C) and hyperthermophiles (optimal growth temperatures > 80°C) are typically thermostable (resistant to irreversible inactivation at high temperatures) and thermophilic (optimally active at high temperatures, i.e., > 60°C). Such thermostable enzymes are referred to as "thermozymes". Some thermophiles can grow at 90°C or above and some have maxima above 100°C. Procaryotes that have growth optima between 80°C and about 113°C are called hyperthermophiles. They usually do not grow well below 55°C (Microbiology, 5th edition, Lansing M Prescott, Donald A Klein, John P Harley). Many important biotechnological processes utilize thermophilic enzymes because of their ability to withstand intense heat (Madigan MT, Martino JM (2006). Brock Biology of Microorganisms (11th ed.). Pearson. pp. 136. ISBN 0-13-196893-9).

The one or more than one enzyme used in the invention may be thermostable and/or thermophilic. The enzymes used in the invention may have a temperature optimum of activity at >60°C. The temperature optimum of an enyzme can be determined by conducting activity assays at optimum pH at several temperatures to determine the temperature optimum, for example as described by Takashima et al., J. Biotechnol. 1996; 50(2-3):137-47.

Thermostable cellulases and hemicellulases are reviewed by Haki and Rakshit, Bioresource Technology, Vol. 89, Issue 1, 2003, pages 17-34. These enzymes, notably those listed in Tables 5 and 6 may be used in the processes of the present invention. Turner et al., Microbial Cell Factories 2007, 6:9 reviews thermophiles and thermostable enzymes, inter alia for biodegradation and modification of lignocellulose and for biofuel production. The enzymes mentioned in sections "Cellulose conversion by cellulases", "Hemicellulose conversions" and "Conversion of pectins" of Turner *et al.* may be used in the processes of the present invention. Taylor and Vaisman, BMC Structural Biology 2010, 10(Suppl 1):S5 describe discrimination of thermophilic and mesophilic proteins. Li et al., review structural features of thermoenzymes in Biotechnology Advances, 23 (2005) issue 4, 271-333. ΔG_{stab} of thermophilic proteins is 5 to 20 kcal/mol higher than that of mesophilic proteins.

Other examples of cell wall degrading enzymes that can be used in the present invention are the following endoglucanases mentioned in WO 2009/155601. Endoglucanase genes can be obtained from Aspergillus aculeatus (U.S. Pat. No. 6,623,949; WO 94/14953), Aspergillus kawachii (U.S. Pat. No. 6,623,949), Aspergillus oryzae (Kitamoto et al., Appl. Microbiol. Biotechnol, 1996, 46: 538-544; U.S. Pat. No. 6,635,465), Aspergillus nidulans (Lockington et al., Fungal Genet. Biol., 2002, 37: 190-196), Cellulomonas fimi (Wong et al., Gene, 1986, 44: 315-324), Bacillus subtilis (MacKay et al., Nucleic Acids Res., 1986, 14: 9159-9170), Cellulomonas pachnodae (Cazemier et al., Appl. Microbiol. Biotechnol., 1999, 52: 232-239), Fusarium equiseti (Goedegebuur et al., Curr. Genet., 2002, 41 : 89-98), Fusarium oxysporum (Hagen et al, Gene, 1994, 150: 163-167; Sheppard et al., Gene, 1994, 150: 163-167), Humicola insolens (U.S. Pat. No. 5,912,157; Davies et al., Biochem J., 2000, 348: 201-207), Hypocrea jecorina (Penttila et al., Gene, 1986, 45: 253-263), Humicola grisea (Goedegebuur et al., Curr. Genet., 2002, 41 : 89-98), Micromonospora cellulolyticum (Lin et al., J. Ind. Microbiol, 1994, 13: 344-350), Myceliophthora thermophila (U.S. Pat. No. 5,912,157), Rhizopus oryzae (Moriya et al., J. Bacteriol, 2003, 185: 1749-1756), Trichoderma reesei (Saloheimo et al., Mol. Microbiol., 1994, 13: 219-228), and Trichoderma viride (Kwon et al., Biosci. Biotechnol. Biochem., 1999, 63: 1714-1720; Goedegebuur et al., Curr. Genet., 2002, 41 :89-98) or the endo-1,4-β-glucanase E1 (GeneBank accession No. U33212, amino acid sequence given on page 13 of WO 2009/155601) described in US 5,275,944 and having a temperature optimum of 83°C.

Examples of cellobiohydrolases as cell wall degrading enzymes for use in the invention are mentioned in WO 2009/155601; they are encoded by cellobiohydrolase genes that can be obtained from Acidothermus cellulolyticus, Acremonium cellulolyticus (U.S. Pat. No. 6,127,160), Agaricus bisporus (Chow et al., Appl. Environ. Microbiol, 1994, 60: 2779-2785), Aspergillus aculeatus (Takada et al., J. Ferment. Bioeng., 1998, 85: 1-9), Aspergillus niger (Gielkens et al., Appl. Environ. Microbiol., 65: 1999, 4340-4345), Aspergillus oryzae (Kitamoto et al., Appl. Microbiol. Biotechnol, 1996, 46: 538-544), Athelia rolfsii (EMBL accession No. AB103461), Chaetomium thermophilum (EMBL accession Nos. AX657571 and CQ838150), Cullulomonas fimi (Meinke et al., Mol. Microbiol., 1994, 12: 413-422), Emericella nidulans (Lockington et al., Fungal Genet. Biol, 2002, 37: 190-196), Fusarium oxysporum (Hagen et al., Gene, 1994, 150: 163-167), Geotrichum sp. 128 (EMBL accession No. AB089343), Humicola grisea (de Oliviera and Radford, Nucleic Acids Res., 1990, 18: 668; Takashima et al, J. Biochem., 1998, 124: 717- 725), Humicola nigrescens (EMBL accession No. AX657571), Hypocrea koningii (Teeri et al., Gene, 1987, 51 : 43-52), Mycelioptera thermophila (EMBL accession No. AX657599), Neocallimastix patriciarum (Denman et al., Appl. Environ. Microbiol, 1996, 62: 1889-1896), Phanerochaete chrysosporium (Tempelaars et al., Appl. Environ. Microbiol., 1994, 60: 4387- 4393), Thermobifida fusca (Zhang, Biochemistry, 1995, 34: 3386-3395), Trichoderma reesei (Terri et al., BioTechnology, 1983, 1 : 696-699; Chen et al., BioTechnology, 1987, 5: 274-278), and Trichoderma viride (EMBL accession Nos. A4368686 and A4368688).

β-D-glucosidase genes that can be used in the present invention can be obtained from Aspergillus aculeatus (Kawaguchi et al, Gene, 1996, 173: 287-288), Aspergillus kawachi (Iwashita et al, Appl. Environ. Microbiol., 1999, 65: 5546-5553), Aspergillus oryzae (WO 2002/095014), Cellulomonas biazotea (Wong et al., Gene, 1998, 207: 79-86), Penicillium funiculosum (WO 200478919), Saccharomycopsis betabuligera (Machida et al, Appl. Environ. Microbiol., 1988, 54: 3147-3155), Schizosaccharomyces pombe (Wood et al., Nature, 2002, 415: 871-880), and Trichoderma reesei (Barnett et al., BioTechnology, 1991, 9: 562-567).

Several cell wall-degrading enzymes, coding sequences thereof, and methods for expression in plants are described in EP 1 867 724 A2.

Genes or coding sequences encoding cell wall-degrading enzymes for use in the present invention may be codon-optimised according to codon usage in the plant in which they are to be expressed according to generally known methods. The genes or coding sequences may be codon-optimised according to the codon usage in the plant genus in which they are to be expressed, preferably they are codon-optimised according to preferred codon usage in the plant species in which they are to be expressed. Such methods of codon optimisation are standard practice in the art and are commercially performed by oligonucleotide or gene synthesis companies such as Entelechon GmbH (Bad Abbach, Germany). Codon optimisation for gene expression in a particular plant genus or species may be combined with other gene optimisations inter alia for homogenous GC content, mRNA secondary structure, avoiding cryptic splice sites, codon and motif repeats, restriction sites etc., e.g. by Entelechon GmbH.

When the plants or part thereof have expressed sufficient amounts of cell wall-degrading enzyme in step (i), they are harvested. Harvesting of said plants or plant parts can be done by conventional means as known in the art for the plants employed. Harvesting may comprise elimination of undesired plant parts. For example, it may be desired to remove leaf material from potato tubers or beetles. With other plants, roots may be removed. Harvesting may also comprise shredding, hackling, chipping or chopping of desired plants or plant parts, as is generally done with maize to be ensiled. Then, the harvest is ensiled in the storing step of the process.

Ensiling of harvests is widely used by farmers for the preservation of forages as an animal feed supply for periods where fresh forage is limited or unavailable. Thus, ensiling is a well established technique that can be performed according to known procedures for the plants employed in the process of the invention. The ensiling process comprises an anaerobic microbial fermentation of water-soluble sugars to lactic acid, lowering the pH to a point that inhibits further microbial fermentation. The goal is a rapid pH drop to minimize fermentation losses of feedstuff nutrients, especially protein. Literature on ensiling plant harvests is abundant and knowledge on conditions such as suitable moisture content of the material to be ensiled is known and can be tested experimentally, see e.g.: "Preparation, Maintenance and Utilisation of Maize Silage: A Review" by Castenada, C., Birch, C. J. and Dryden, G. (2003); in: Birch, C. J. and Wilson, S. R. Versatile Maize - Golden Opportunities: 5th Australian Maize Conference Proceedings, City Golf Club, Toowoomba, (67-75). 18 - 20 February, 2003; and "Troubleshooting Silage Problems: How to identify potential problems" by Robert J. Van Saun and A. Jud Heinrichs, Department of Dairy and Animal Science; the Pennsylvania State University, http://www.das.psu.edu/research-extension/dairy/nutrition/pdf/ troubleshootsilage08125.pdf). A selection of literature references on ensiling plant material is as follows: US 5,053,233 discloses inter alia use of additives. Other publications on ensiling plants and use of additives are: Adesogan et al., J. Dairy Sci. 86 (2003) 1789-1796; Conaghan et al., J. Dairy Sci. 93 (2010) 628-643; Kung et al., J. Dairy Sci. 87 (2004) 1310-1316; Weinberg, "Preservation of forage crops by solid state lactic acid fermentation-ensiling" in: Current developments in solid state fermentation, 2008, pages 443-467; Woolford, M. K. (1984), Grass and Forage Science 39: 53-57; M.E. McCullough, Journal of Dairy Science, Vol. 52, Issue 10, October 1969, 1605-1608. Further, reference is made to the manual "Successful silage", eds.: Alan G. Kaiser, John W. Piltz, Helen M. Burns and Neil W. Griffiths, Top Fodder, published by Dairy Australia and New South Wales Department of Primary Industries, Australia, 2004. The manual is available online at: www.dpi.nsw.gov.au/agriculture/field/pastures-and-rangelands/silage/successful-silage. Conditions for ensiling immature corn are found in Shao et al., Biotechnology for Biofuels 2010, 3:12.

Lactic acid fermentation generally starts automatically by lactic acid bacteria present in the environment or on the plant that is to be stored. It is, however, also possible to inoculate the harvest to be ensiled by additional lactic acid-producing organisms. Commercial products may be used for this purpose, such as Silo-King, Agri-King Inc., Fulton, USA containing *Lactobacillus plantarum, Pediococcus pentosaceus* and *Enterococcus faecium.*

Alternatively or additionally to achieving the pH drop by lactic acid-producing organisms, acids, preferably organic acids such as acetic acid, propionic acid or butyric acid may be added to the harvest. In this way, a rapid pH drop can be achieved.

The harvest, optionally after having dried the harvested plant material to a suitable moisture content, is put into a container or silo, and optionally compacted as required to avoid excessive air in the container or silo. As mentioned above, lactic acid-producing organisms may be added to the silage. Further or alternatively, it is possible to add silage additives to the silage material. Silage additives are known. Examples of silage additives are nitrites, hexamine, sodium benzoate, sodium propionate, formic acid, propionic acid and ammonia, and mixtures thereof. Mixtures of sodium benzoate and sodium propionate or mixtures of sodium benzoate and propionic acid are conventional silage additives. Detailed guidance on the use of silage additives is provided in chapter 7 of the manual "Successful silage" cited above.

The container or silo is then covered or closed, so as to allow anaerobic conditions to develop. Containers/silos can be solid silos as conventionally used in agriculture. Alternatively, flexible plastic bags that may be made of polyethylene can be used for ensiling the harvest. Excessive air may be removed from plastic bags before sealing using a vacuum pump to reduce oxygen content.

Upon storage, the silage undergoes various phases, including an aerobic phase, a fermentation phase and a stable phase. In the aerobic phase, aerobic microorganisms consume the remaining oxygen, whereby anaerobic conditions are reached. In the fermentation phase, growth of anaerobic bacteria produce lactic acid that lowers the pH. Low pH reduces the activity of proteolytic enzymes and stops the growth of some anaerobic bacteria. The fermentation phase can last from 7 to 30 days. This phase ceases when fermentation of sugars by lactic acid bacteria ceases either due to low pH that stops bacterial growth or depletion of low molecular sugar. Possibly after a secondary fermentation phase due to growth of clostridial spores, a stable phase is reached that is characterized by little biological activity. In the stable phase, the silage can be stored with little further change for extended periods of time of, for example, more than 100 days, preferably more than 200 days and even more than 300 days. Although there is no strict upper limit, 365 days may be mentioned as such upper limit for storage of the silage. The high flexibility of the storage time in the silage is ideal for storing the silage until the silage is used for degrading plant cell wall components of plant material. The minimum storage time in step (iii) or in the process of storing plant cell wall-degrading enzymes is 21 days. The silage can be stored at ambient temperature without external heating or cooling. The silage internally undergoes temperature changes in the course of the silage phases mentioned above.

Storage conditions in silage can be tested on a small laboratory scale before performing a process on a large industrial scale. A model system for laboratory-scale silage fermentation is described by Johnson et al., J. Appl. Microbiol. 98 (2005) 106-113. This model system can be employed for testing silage conditions such as packing density, moisture content of the material to be ensiled etc. for plant harvests for which there may be less experience from conventional ensiling of animal feed. As described by Johnson *et al.,* silage conditions determined on a small scale can be used for determining silage conditions on larger scales.

Accordingly, when storing the harvest as a silage in step (iii), the harvest is placed in a container (described above) such that an atmosphere of reduced oxygen content can develop in the container. This may comprise covering or closing the container in order to reduce or prevent exchange of air with the environment outside the container. In large containers such as silos used on farms, a top layer of the harvest in the silo may function as a cover of the container that prevents exchange of air to harvest layers below the top layer of the harvest.

The harvest is stored in step (iii) for at least 21 days, preferably for at least 1 month in said silage, i.e. in an atmosphere of reduced oxygen content. Storage is generally done at ambient temperature, which means that the silage is neither heated from the outside nor cooled. The harvest may be stored in said silage for 2 or 3 months. However, it is generally not stored longer than 12 months, preferably not longer than 8 months, more preferably not longer than 5 months, since, in spite of the exceptional stability of the expressed cell wall-degrading enzymes in the silage, activity of the expressed enzymes generally decreases with increasing storage time in silage. Suitable storage times for a given cellulase expressed in a particular plant or plant part may be determined experimentally and followed over time, similarly as done in the Examples. Herein, storage times start when closing or covering the container containing the harvest.

In the process of storing plant cell wall-degrading enzymes, harvested plants or plant parts containing expressed one or more than one plant cell wall-degrading enzymes may be stored in a silage as described above for step (iii) of the process of the invention. Different plant or plant parts containing different expressed enzymes may be ensiled together to produce a silage containing two or more cell wall-degrading enzymes such as all enzymes required for degrading cellulose as described above.

The product obtained or obtainable in step (iii) of the process of the invention is the silage used in the invention. The silage contains one or more than one active plant cell wall-degrading enzymes. Activity of the enzyme can be determined according to generally known methods for the enzyme in question. Such activity tests for several enzymes are described in the examples. Generally, these methods require homogenization and/or extraction step to produce total soluble protein (TSP) from the silage. It is also possible to perform further purification steps before determination of activity of an enzyme in question.

The silage used in the invention contains active enzymes capable of degrading plant cell wall components. Thus, the silage can be used in a process of degrading plant cell wall components of plant material. The main application of the silage is in a process of producing bioalcohol, notably bioethanol, from lignocellulosic material. There is abundant literature on processes for degrading plant cell wall components of various plant lignocellulosic materials to oligosaccharides and, preferably, monosaccharides, a process also referred to as saccharification. Saccharification may be followed by fermentation, the process wherein monosaccharides and optionally small oligosaccharides obtained in saccharification are fermented to alcohol. Yeasts such as *Saccharomyces cerevisiae* are typically used for alcohol fermentation. In one embodiment, the steps of saccharification and fermentation are combined in a simultaneous process known as "SSF" that stands for "simultaneous saccharification and fermentation". Publications on SSF are, inter alia, the following: Kristensen et al., Biotechnology for Biofuels 2008, 1:5; Olofsson et al., Biotechnology for Biofuels 2008, 3:17; Doran-Peterson et al., Methods Mol Biol. 2009; 581:263-80. Accordingly, the process of degrading plant cell wall components of plant material and the process of producing a bioalcohol comprises a step of saccharification of plant material by treating the plant material with a silage described herein. The latter process also comprises a step of alcohol fermentation for producing bioalcohol from glucose produced in the saccharification step.

The plant material cell wall components of which are to be degraded may be the same or different from the silage of the invention. However, in the main application of the invention, the plant material is different from the silage of the invention obtained in step (iii). The plant material that is different from the silage differs from the plants from which the silage is obtained at least in that the plant material does not contain the cell wall-degrading enzyme expressed in the plant from which the silage is obtained, and does not contain a construct encoding such enzyme.

Plant material to be treated in the degradation and alcohol production processes of the invention may be any plant agricultural waste material such as straw. Plant material rich in cellulose is preferred. Mostly, however, these processes are used for treating plant material from plants specifically grown for producing bioalcohol. There are no strict limits for the origin of the plant material to be employed. The plant material may be from annual, biannual or perennial plants. Both monocot and dicot plants may be used, whereby cellulose-rich plants is preferred. Examples of annual plants are cerial plants such as barley, wheat, oat. A preferred plant is maize. Examples of perennial plants are woody plants, notably trees. Plant material from different plant sources may be mixed and treated as a mixture. The plant material is preferably milled to comminuted before subjecting to the processes of the invention to increase the surface area and ease attack by the degrading enzymes.

In the process for degrading cell wall components or in the process for producing the bioalcohol of the invention, the step of treatment of plant material with the silage may be preceded by a pretreatment step for changing the physical and/or chemical structure of the cell walls of the plant material. Such pretreatment steps are known in the art (see e.g. Sticklen, Nature Reviews 9 (2008), 433-443). Most process concepts for bioethanol production from lignocellulose start with a thermo-chemical hydrolysis of the hemicellulose part (pretreatment), followed by an enzymatic hydrolysis of the cellulose part. The purpose of the pretreatment is to alter the lignocellulosic structure and increase the rate of enzymatic hydrolysis of primarily the cellulose (Kristensen et al., Biotechnology for Biofuels 2008, 1 :5). This should be done with a minimum formation of compounds which inhibit the fermenting microorganisms. The accessible surface area is regarded as one of the most important factors affecting the effectiveness of enzymatic cellulose degradation. In native wood only a small fraction of the cell wall capillaries are accessible to the enzymes. Pretreatment, however, increases the available area in several ways: i) fragments and cracks are formed yielding increased area, ii) the hemicellulose fraction is hydrolysed which diminishes shielding effects, iii) the lignin also undergoes structural changes and the wood is delignified to various degrees, depending on the pretreatment technology. Thus, the shielding of microfibrils and occluding of pores, caused by lignin, can be removed. Other factors, believed to influence the digestibility in SSF, are the substrate crystallinity and the degree of polymerization. Pre-treated plant material is also considered "plant material" for the purposes of the present invention.

The pretreatment methods can be divided into physical and chemical methods, and combinations of these two are commonly used (see *e.g.* the review written by Mosier et al. Bioresour Technol 2005, 96(6):673-686). The type of feedstock affects the choice of pretreatment method. The hemicellulose is, for instance, acetylated to a high degree in xylan-rich materials. Since acetate is liberated during hydrolysis, the pretreatment of these materials is to some extent autocatalytic and require less added acid and milder process conditions. However, the liberated acetate adds to the toxicity of the hemicellulose hydrolyzates (see: Biotechnology for Biofuels 2008, 1:5). Ammonia fiber expansion/explosion (AFEX) pretreatment is an attractive method for pretreatment of agricultural residues, yielding highly digestible cellulose. AFEX depolymerizes the lignin, removes the hemicellulose and decrystallizes the cellulose. The moderate temperature and pH also minimize formation of sugar degradation products. Another method of pretreatment is the lime method, based on calcium (or sodium) hydroxide. Alkali pretreatments are run at lower temperatures for long residence times, and as for the AFEX method, a delignification of the biomass is obtained. AFEX is described inter alia in Shao et al., Biotechnology for Biofuels 2010, 3:12 and references 8-12 cited therein.

After the optional pretreatment, the optionally pretreated plant material is subjected to treatment with the silage for saccharification in step (iv) or step (b). This step is generally done in a aqueous reaction medium, preferably in stirred-tank reactors or fermentors. Temperature and pH of the reaction medium are set such that the enzyme(s) contained in the reaction medium have sufficient activity. Silage containing at least one cell wall degrading enzyme expressed as described herein is added. Other enzymes required for cell wall degradation may be added to the reaction medium from other sources. Preferably, silage containing multiple different enzymes involved in plant cell wall or cellulose degradation is added. Where multiple cell wall-degrading enzymes are used, they are preferably selected such that they have similar temperature and pH optima so that high enzymatic activity can be obtained for all the enzymes used under the same or similar pH and temperature conditions.

The silage is added such that cell wall degrading enzymes contained therein easily enter the reaction medium. Since storage in silage partially destroys the structure of the plants or parts thereof containing the enzymes, the silage may be added to the reaction medium as it is, notably if the enzymes are targeted to the apoplast. Alternatively, the silage may be milled or homogenized before addition to the reaction medium in order to promote entry of the enzymes into the reaction medium. In the reaction medium, the cell wall material from the ensiled plant material is also degraded by the enzymes. Thus, the plant material originating from the plants having expressed the enzymes is also used for monosaccharide and bioalcohol production, which contributes to the excellent efficiency and cost-effectiveness of the present invention.

Conditions for the saccharification step (step (iv) or step (b)) in the invention are similar as the conditions known in the art for saccharification using added cellulases, see e.g. Teymouri et al., Appl. Biochem. Biotechnol. (2004) 116, 1183-1192; Ransom et al., Appl. Biochem. Biotechnol (2007) 36, 207-220; Oraby et al., Transgenic Res. (2007) 16, 739-749. The mixing ratio of the amount of silage to the amount of plant material (different from the silage) is not particularly limited. The ratio may in a range of from 100:1 to 1:1000, preferably in a range from 10:1 to 1:100, more preferably from 1:1 to 1:10 by weight determined on the basis of dry matter. Saccharification is generally performed in reactors or fermentors under controlled pH, temperature, and mixing conditions. Duration of saccharification is chosen such that a desirable degree of saccharification is obtained. A saccharification step may last from 1 to 200 hours, preferably from 5 to 4 hours. Saccharification may be carried out at temperatures from about 30°C to about 70°C, preferably from about 35 to 60°C dependent on the cell wall degrading enzymes used and their concentration present. The pH is selected for high activity of the cell wall-degrading enzymes and may be in the range of from 4 and about 7, preferably from pH 4.5 to 6.

In the fermentation step (c) of the bioalcohol production process, monosaccharides produced in the saccharification step are converted to bioalcohol such as bioethanol. As mentioned above, saccharification and fermentation can be done simultaneously (see e.g. Biotechnology for Biofuels 2008, 1:5). Alternatively, these steps are done consecutively. For fermenting monosaccharides to alcohol, known microorganism as known from starch-based bioethanol production can be employed. Yeasts such as *Saccharomyces cerevisiae* may be used, and many suitable strains thereof for alcohol production are known. Conditions for alcohol fermentation are also known from the bioalcohol production processes based on starch-derived glucose. Produced bioalcohol may then be isolated and purified, e.g. using distillation.

### EXAMPLES

The invention is further described using examples that do not limit the scope of protection.

### Reference Example 1: Determination of Agrobacterium cell concentration in liquid culture in terms of colony forming units (cfu)

The concentration of *Agrobacterium* cells in liquid suspension in terms of colony forming units per ml (cfu/ml) of liquid suspensions can be determined using the following protocol. Cells of *Agrobacterium tumefaciens* strain ICF 320 transformed with construct pNMD620 were grown in 7.5 ml of liquid LBS medium containing 25 mg/L kanamycin (AppliChem, A1493) and 50 mg/L rifampicin (Carl Roth, 4163.2). The bacterial culture was incubated at 28°C with continuous shaking. Absorbance or optical density of bacterial culture expressed in absorbance units (AU) was monitored in 1-ml aliquots of the culture using a spectrophotometer at 600 nm wavelength (OD600). The cell concentration estimated as a number of colony-forming units per milliliter of liquid culture (cfu/ml) can be analyzed at OD600 values 1; 1.3; 1.5; 1.7 and 1.8. For this purpose 250-µl aliquots of liquid culture were diluted with LBS-medium to achieve a final volume of 25 ml (dilution 1:100). 2.5 ml of such 1:100 dilution were mixed with 22.5 ml of LBS to achieve the dilution 1:1000. Liquid culture dilutions 1:100; 1:1,000; 1:10,000; 1:100,000; 1:1,000,000; 1:10,000,000 and 1:100,000,000 were prepared similarly. Aliquots of last three dilutions were spread on agar-solidified LBS medium supplemented with 25 mg/L kanamycin and 50 mg/L rifampicin (250 µl of bacterial culture per plate of 90 mm diameter). Plating of aliquots for each dilution was performed in triplicate. After 2 days incubation at 28°C, bacterial colonies were counted for each plate. Plating of 1: 1,000,000 and 1:10,000,000 dilutions resulted in few hundred and few dozen colonies per plate, respectively. So far as dilution 1:100,000,000 provided just few colonies per plate, this dilution was not used for calculation of cell concentration. The cell concentration was estimated according to the formula: cfu/ml = 4 x number of colonies per plate x dilution factor.

For transforming cell concentrations as measured by absorbance measurements at 600 nm (in LB medium) and in terms of cell-forming units, the following relation is used herein: an OD600 of 1.0 corresponds to 1.1 x10⁹ cfu/ml.

### Example 1: Vectors used in the following examples

Tobacco mosaic virus (TMV) based viral vectors with cell-to-cell movement ability designed as pro-vectors containing complementary parts of viral replicon and assembled vectors containing full viral replicon were made. They were created on the basis of vectors described in Marillonnet et al. (2004), Proc. Natl. Acad. Sci. USA 101 (18) 6852-685), WO 02/88369.

The 5'-module TMV pro-vectors have a *Arabidopsis* actin 2 promoter-driven TMV RNA-dependent RNA-polymerase (RdRp) containing 14 introns, TMV movement protein (MP) with 2 introns, and different fusion sequences followed by an intron 5' half part, an attP recombination site and a nopalin synthase (Nos) terminator. The entire fragment is cloned between the T-DNA left and right borders of binary vector. Fusion sequences include His(6)-tag with enterokinase cleavage site (His-EK) (plCH22455 construct, the nucleotide sequence of its T-DNA is given as SEQ ID NO: 1), rice α-amylase 3A apoplast targeting presequence (TP) (pICH20155), *Nicotiana plumbaginifolia* calreticulin apoplast TP (pICH20188), apple pectinase apoplast TP (pICH20388), barley α-amylase apoplast TP (pICH20999), *Nicotiana plumbaginifolia* calreticulin apoplast TP-His(6)-EK (pICH22464), artificial dicot chloroplast TP (pICH20030) and artificial dicot chloroplast TP-His-EK (pICH22474) (Fig. 2A).

In 3'-provector modules of TMV pro-vectors, an attB recombination site is followed by an intron 3' half part, the gene of interest coding sequences and a 3'-TMV non-translated region (3'NTR) as well as a nopalin synthase (Nos) terminator. The entire fragment is cloned between the T-DNA left and right borders of binary vector. Genes of interest used in these constructs encoded endoglucanase E1 from *Acidothermus cellulolyticus* (pNMD231; the nucleotide sequence of the T-DNA region is given as SEQ ID NO: 2), Exoglucanase 1 (CBHI) from *Trichoderma reesei* (pNMD241; the nucleotide sequence of the T-DNA region is given as SEQ ID NO: 3), exocellulase E3 from *Thermobifida fusca* (pNMD251; the nucleotide sequence of T-DNA region is given as SEQ ID NO: 4), β-glucosidase BGL4 from *Humicola grisea* (pNMD910; the nucleotide sequence of T-DNA region is given as SEQ ID NO: 5) and GFP (pICH7410) (Fig. 2B). Coding sequences of all genes of interest except GFP were codon-optimized for *Nicotiana benthamiana* and synthesized by Entelechon GmbH (Bad Abbach, Germany).

The integrase vector module plCH14011 harbors a heat-shock-promoter-driven *PhiC31* integrase gene with nuclear localization signal (NLS) and a Nos terminator inserted between left and right borders of the T-DNA (Fig. 2C). The nucleotide sequence of the T-DNA region is given as SEQ ID NO: 6.

Assembled TMV vectors contain Actin 2-promoter-driven TMV RdRp with 14 introns, TMV MP with 2 introns, fusion sequence, and coding sequence of gene of interest, 3'TMV non-translated region (3' NTR) as well as a Nos terminator. The entire fragment is inserted between the T-DNA left and right borders of binary vector. pNMD1201 (nucleotide sequence of its T-DNA is given as SEQ ID NO: 7), pNMD1213 and pNMD1192 constructs contain β-glucosidase BGL4 from *Humicola grisea* without any fusion sequence, BGL4 fused with N-terminal His-EK and BGL4 fused with artificial dicot chloroplast TP, respectively. The pNMD1231 construct bears endoglucanase E1 from *Acidothermus cellulolyticus* fused with rice α-amylase 3A apoplast TP. pNMD1181 contains Exoglucanase 1 (CBHI) from *Trichoderma reesei* fused with barley α-amylase apoplast TP. pNMD1161 (nucleotide sequence of its T-DNA is given as SEQ ID NO: 8), and pNMD1129 contain exocellulase E3 from *Thermobifida fusca* fused with apple pectinase apoplast TP and artificial dicot chloroplast TP-His-EK, respectively. The pNMD560 vector contains GFP ORF without any fusion sequences (Fig. 7).

Endoglucanase Cel5A from *Thermotoga maritima* was expressed from construct pNMD3081. A plasmid map of the TMV-based vector for the expression of cytosolic endoglucanase Cel5A is shown in Fig. 14.

Details on the cellulolytic enzymes employed are as follows.
Endoglucanase E1 is from the thermophilic bacterium *Acidothermus cellulolyticus* 11B (Swiss-Prot: P54583; EC 3.2.1.4). It is a termostable enzyme with 81°C temperature optimum and pH optimum from about 5 to about 6 (Himmel et., 1994; Sakon et al., 1996). It is a secreted protein with cleavable N-terminal signal peptide. The protein precursor consists of 562 amino acid residues; the signal peptide spans the first 41 amino acids; the 42-562 aa region corresponds to the mature protein. A nucleotide sequence corresponding to the mature part of the protein (42-662 aa) without native signal peptide was codon optimized for *Nicotiana benthamiana* and synthesized by Entelechon GmbH (Bad Abbach, Germany). This sequence was subcloned into TMV expression vectors.

Exoglucanase 1 (CBH1) is from the mesophilic fungus *Trichoderma reesei* (also known as *Hypocrea jecorina*) (Swiss-Prot: P62694; EC 3.2.1.91). It is a secreted protein with cleavable N-terminal signal peptide. The protein precursor consists of 513 amino acids, signal peptides spans first 17 amino acids. It is a mesophilic enzyme with 45-50°C temperature optimum and pH optimum 5 (Baker et al., 1998). The nucleotide sequence corresponding to the mature part of the protein (18-662 aa) without native signal peptide codon optimized for *Nicotiana benthamiana* and synthesized by Entelechon GmbH (Bad Abbach, Germany) was used for subcloning into TMV expression vectors.

Beta-1,4-exocellulase (E3) is from the thermophilic bacterium *Thermobifida fusca* (GenBank: AAA62211; EC 3.2.1.91). It is a secreted protein; the protein precursor consists of 596 amino acid residues, including 38 aa N-terminal signal peptide and the mature part (39-596 aa). E3 is thermostable, retaining full activity after 16 h at 55°C. It also has a broad pH optimum around 7-8, retaining 90% of its maximal activity between pH 6 and 10 (Zhang et al., 1995, 2000). The nucleotide sequence corresponding to the mature part of the protein (39-596 aa) without native signal peptide codon optimized for *Nicotiana benthamiana* and synthesized by Entelechon GmbH (Bad Abbach, Germany) was used for subcloning into TMV expression vectors.

β-glucosidase BGL4 is from the thermophilic fungus *Humicola grisea* (GenBank: BAA74958; EC 3.2.1.21). It is a 476 amino acids polypeptide. The literature describing BGL4 does not contain any indication about the presence of cleavable N-terminal signal peptide; in addition, the TargetP 1.1 software (http://www.cbs.dtu.dk/services/TargetP/) did not identify any N-terminal signal peptide in this protein sequence. The BGL4 enzyme is thermostable, with a temperature optimum 60°C. The pH optima of this enzyme is 6.0, and it retains more than 80% of its relative activity within the range of pH 6.0-11.0 after 20 h at 4°C (Takashima et al. 1999). The nucleotide sequence corresponding to the full-length protein (39-596 aa) was codon optimized for *Nicotiana benthamiana* and synthesized by Entelechon GmbH (Bad Abbach, Germany). This sequence was used for subcloning into TMV expression vectors.

The endoglucanase Cel5A from *Thermotoga maritima* is described in Pereira JH et al. (2010) Biochemical characterization and crystal structure of endoglucanase Cel5A from the hyperthermophilic Thermotoga maritima. J Struct Biol. 172(3): 372-9; and Mahadevan SA et al. (2008) Site-directed mutagenesis and CBM engineering of Cel5A (Thermotoga maritima). FEMS Microbiol Lett. 287(2): 205-11. Some basic properties are given in Table 1. The exoglucanase 1 (CBHI) from *Humicola grisea* is described in Takashima, S. et al., (1996) Cloning, sequencing, and expression of the cellulase genes of Humicola grisea var. thermoidea. J Biotechnol. 50:137-147. Some basic properties of this enzyme are given in Table 1.

The nucleotide sequences of the codon-optimised coding sequences of the cell wall degrading enzymes used in the examples are as follows:
SEQ ID NO: 9: coding sequence of β-glucosidase Bgl4 from *Humicola grisea* (BAA74958.1);
SEQ ID NO: 10: coding sequence of the endoglucanase Cel5A from *Thermotoga maritima* (3MMW_D);
SEQ ID NO: 11: coding sequence of the endoglucanse E1 from *Acidothermus cellulolyticus* (P54583.1) mature part (without signal peptide);
SEQ ID NO: 12: coding sequence of the exoglucanase 1 (CBHI) from *Trichoderma reesei* (P62694.1) mature part (without signal peptide);
SEQ ID NO: 13: coding sequence of the Exoglucanase 1 (CBHI) from *Humicola grisea* (D63515) mature part (without signal peptide);
SEQ ID NO: 14: coding sequence of the exocellulase E3 from *Thermobifida fusca* (AAA62211.1) mature part (without signal peptide).

### Example 2: Targeting to the cell compartments improves the expression level and enzymatic activity of recombinant cell wall-degrading enzymes

Test on expression of cellulases in different cell compartments (cytosol, chloroplast and apoplast). For this purpose cellulolytic enzymes were expressed as translational fusions with corresponding targeting presequences using TMV pro-vector modules (Marillonnet et al., 2004, see above), WO 02/88369.

*Nicotiana benthamiana* plants were grown in the greenhouse (day and night temperatures of 19-23°C and 17-20°C, respectively, with 12 h light and 35-70% humidity). Six-week-old plants were used for inoculations.

For plant transfection, saturated *Agrobacterium* overnight cultures were adjusted to OD₆₀₀=1.3 (∼1.2x10⁹ cfu/ml) with *Agrobacterium* inoculation medium (AIM: 10 mM MES pH 5.5, 10 mM MgSO₄). Further dilutions of cultures were done with AIM and inoculation was carried out by infiltration using a needleless syringe.

For screening of plant material expressing cellulase fusions to different targeting presequences for cellulase activity, a dilution mixture of *Agrobacterium* strains carrying 5'-TMV provectors (100-fold dilution with respect to the final mixture), 3'-TMV provectors (100-fold dilution) or integrase construct (10-fold dilution), respectively, was inoculated by needleless syringe into 3 leaves of different age of *Nicotiana benthamiana.* An overview over agrobacterial inoculations for the optimisation of transient expression of recombinant cellulases is shown in Fig. 3.

Inoculated *Nicotiana benthamiana* plants were incubated in a greenhouse under the conditions described above for 11 days. Variable phenotypes were observed in inoculation zones ranging from strong necrosis (e.g. endoglucanase E1 expressed in cytosol) to practically unaffected tissue (all β-glucosidase BGL4 fusions) (Fig. 4).

The leaf material from 3 leaves of different age was harvested using the cork borer at 7 to 11 dpi (days post inoculation) in triplicate samples, frozen in liquid nitrogen and stored at -80°C. Harvested plant material was analyzed using the SDS-PAGE and enzymatic activity assays.

### SDS-PAGE analysis with Coomassie staining

For SDS-PAGE, about 50 mg fresh weight *Nicotiana benthamiana* leaf material were ground in liquid nitrogen and crude protein extracts were prepared with 5 volumes of 2 x Laemmli buffer. Total soluble protein (TSP) was extracted in small-scale from -150 mg fresh weight plant material ground in liquid nitrogen with 5 volumes pre-chilled extraction buffer (EB: 50 mM sodium acetate pH 5.5, 100 mM NaCl, 10% (v/v) glycerol in water). For extraction, samples were vortexed after addition of EB and incubated on ice for 30 min with vortexing from time to time. Cell debris was separated by centrifugation for 10 min at 13000 rpm and 4°C twice.

The protein concentration of TSP extracts was determined by the Bradford assay using Bio-Rad Protein Assay (Bio-Rad Laboratories, GmbH, Munich, Germany) and Bovine Serum Albumin (Sigma-Aldrich, Co., St- Louis, USA) as a standard. For analysis by SDS-PAGE, Laemmli buffer was added to TSP samples with a final concentration of 1x.

Protein samples were denatured at 95°C for 5 min, separated in 10% polyacrylamide gel and stained using PageBlue™ Protein Staining Solution (Fermentas GmbH, St. Leon-Rot, Germany). An estimation of the percentage of recombinant cellulase fusion of TSP was done by comparison of TSP extracts to known amounts of BSA (Sigma-Aldrich) on Coomassie-stained polyacrylamide gels gels.

Fig. 5 summarizes the SDS-PAGE analysis of expression for different translational fusions of Endoglucanase E1 from *Acidothermus cellulolyticus,* Exoglucanase 1 (CBHI) from *Trichoderma reesei,* Exocellulase E3 from *Thermobifida fusca* and β-glucosidase BGL4 from *Humicola grisea.* Significant variation of the expression level depending on the targeting presequence was found. High expression was demonstrated for all β-glucosidase BGL4 fusions with highest accumulation in cytosol and chloroplast, although in the latter case the cleavage of the targeting presequence was not complete. In case of Endoglucanase E1, the highest accumulation was shown for apoplast targeted forms; accumulation in cytosol and chloroplast was lower. A similar expression pattern was found for Exoglucanase 1 (CBHI) with significant differences between tested apoplast targeting presequences. Surprisingly, CBHI expressed as a fusion with barley α-amylase apoplast TP was not detectable after the Coomassie staining. All Exoglucanase E3 fusions were well expressed with best accumulation in cytosol and chloroplast. Electrophoretic migration shift was found for apoplast targeted forms suggesting that these proteins are glycosylated.

### Enzymatic activity measurements

### Endoglucanase E1 and cellobiohydrolase CBHI cellulase activity assay

Enzyme activity was determined using p-nitrophenyl-β-D-cellobioside as substrate. 100 µg of plant TSP extracts were incubated in 50 mM NaAc pH 5.5, 100 mM NaCl, 5% (v/v) glycerol, supplemented with or without 5 mM p-nitrophenyl-β-D-cellobioside as enzyme blanks, respectively, in a final volume of 1 ml at 50°C for 1 to 24 hours. Aliquots of the reaction mixture were removed at different time points and diluted 1:10 with 0.15 M glycine pH 10.0 to terminate the reaction. The concentration of p-nitrophenol (pNP) released from p-nitrophenyl-β-D-cellobioside was determined by measurement of the absorbance at 405 nm. The commercial enzymes cellulase (endo-1,4-β-D-glucanase) from *Trichoderma* sp. (Megazyme, Bray, Ireland) and cellobiohydrolase (CBHI) from *Trichoderma* sp. (Megazyme) served as positive controls. Enzyme activities were expressed on a gram fresh weight plant material or a TSP basis.

### β-glucosidase Bgl4 cellulase activity assay

For determination of β-glucosidase activity, cellobiose was used as a substrate. Different protein amounts of plant TSP extracts ranging from 1-40 µg were incubated in 50 mM NaAc pH 5.5, 100 mM NaCl, 5% (v/v) glycerol supplemented with or without 5 mM cellobiose as enzyme blanks (mixture with substrate but without enzyme), respectively, in a final volume of 1 ml at 50°C for 30 min. The concentration of released glucose in the samples was determined by D-glucose (GOPOD-Format) Kit (Megazyme). The commercial enzyme cellobiase from *Aspergillus niger* (synonym: Novozyme 188; Sigma-Aldrich) served as positive control. β-glucosidase activity was calculated in cellobiase units (CBU) according to IUPAC or international units (IU) as described in Ghose *et al.,* 1987, and enzyme activities were expressed on a gram fresh weight plant material or a TSP basis.

### Exoglucanase E3 cellulase activity assay

For determination of exoglucanase activity, the insoluble microcrystalline cellulose substrate Avicel (Sigmacell cellulose type 20; Sigma-Aldrich) was used. 100 µg of plant TSP extracts were incubated in 50 mM NaAc pH 5.5, 50 mM NaCl, 5% (v/v) glycerol, 0.02% sodium azide, 1.2 mg/ml Novozyme 188 (β-glucosidase; Sigma-Aldrich), supplemented with or without 1% (w/v) Avicel as enzyme blanks, respectively, in a final volume of 3 ml at 50°C and 90 rpm for 120 hours. Aliquots of the reaction mixture were removed at different time points (2 h, 4 h, 24 h, 48 h, 72 h, and 120 h) for determination of the glucose concentration using D-glucose (GOPOD-Format) Kit (Megazyme). The commercial enzyme cellobiohydrolase (CBHI) from *Trichoderma* sp. (Megazyme) served as positive control. Enzyme activities were expressed on a gram fresh weight plant material or a TSP basis.

Analysis of the enzymatic activities of expressed translational fusions is summarized in Fig. 6. Highest β-glucosidase BGL4 activity was found in cytosol and chloroplast, which correlates with recombinant protein accumulation data (Fig. 6A). In the case of Endoglucanase E1, the highest activity was detected for apoplast targeted forms, again correlating with recombinant protein accumulation (Fig. 6B). The same was found for Exoglucanase CBHI (Fig. 6C). In contrast, no clear correlation with protein accumulation was found for Exoglucanase E3 suggesting that proper folding was critical for enzymatic activity (Fig. 6D). Cytosolic form with high expression provided relatively low enzymatic activity; highest activity was found for chloroplast targeted form with His-tag (high recombinant protein accumulation) and apoplast targeted form with presequence from apple pectinase (relatively low accumulation). Interestingly, poor enzymatic activity was found for chloroplast targeted form without His-tag.

Fusions providing highest enzymatic activity were selected for cloning of assembled vectors to be used for the preparative large-scale production of cell wall-degrading enzymes (Fig. 7). They include two forms of β-glucosidase BGL4 from *Humicola grisea* for cytosolic expression (without any presequence (pNMD1201) and with N-terminal His-EK (pNMD1213)) as well as BGL4 with chloroplast targeting presequence (pNMD1192). For Endoglucanase E1 from *Acidothermus cellulolyticus,* fusion with apoplast targeting presequence from rice α-amylase was selected (pNMD1231 construct). In the case of Exoglucanase 1 (CBHI) from *Trichoderma reesei,* it was the fusion with α-barley amylase apoplast TP (pNMD1181 construct). Two constructs were created for *Thermobifida fusca* exocellulase E3 containing fusions with apple pectinase apoplast TP and chloroplast TP followed by His-EK (pNMD1161 and pNMD1129, respectively). In addition, the pNMD560 construct containing the GFP coding sequence insertion was used as an agrobacterial transfection control.

### Example 3: Spraying with agrobacteria provides similar expression levels of cell wall-degrading enzymes as infiltration

We analysed two different inoculation methods for highest cellulase expression of selected cellulase fusions: syringe infiltration vs. spraying with suspensions of agrobacteria harboring same assembled TMV vectors. For plant transfection, *Agrobacterium* overnight cultures (OD₆₀₀=1-3) were diluted with *Agrobacterium* inoculation medium (AIM, 10 mM MES pH 5.5, 10 mM MgSO₄). For spraying, 0.1% Silwet L-77 (v/v) (Kurt Obermeier GmbH & Co. KG, Bad Berleburg, Germany) was added to the solution. Dilutions of agrobacterial cultures 1:100 and 1:1000 were used for syringe infiltration and spraying, respectively, as providing the highest expression (data not shown). Six week old *Nicotiana benthamiana* plants were used for inoculation with agrobacterial cultures. Plant material was harvested at 4, 7, 11 and 14 dpi (days post infection) and used for the SDS-PAGE analysis and evaluation of enzymatic activity as described in Example 2.

SDS-PAGE analysis (Fig. 8) demonstrated that for most cellulase fusions, syringe infiltration and spraying with agrobacteria provide comparable expression levels. Few days delay of recombinant protein accumulation was observed for spraying delivery, which can be explained by the lower number of leaf cells initially targeted by *Agrobacterium.* Cytosolic β-glucosidase BGL4 from *Humicola grisea* expressed from an assembled TMV vector upon syringe inoculation of a 1:100 dilution of *Agrobacterium* culture (7 dpi) and spray inoculation of a 1:1000 dilution (11 dpi) is estimated to form about 55% and 50% of TSP, respectively. Similarly, chloroplast targeted BGL4 formed about 75% TSP after the infiltration and 60% of TSP after the spraying. In case of Exoglucanase 1 (CBHI) from *Trichoderma reesei* expressed as a fusion with barley-α-amylase apoplast TP, corresponding values were 6.5% and 3.5% of TSP. Chloroplast *TP-His-EK-Thermobifida fusca* exocellulase E3 fusion formed about 30% and 25% of TSP, respectively; and apple pectinase apoplast TP-E3 fusion provided about 7% and 2,5% of TSP, respectively. More significant difference was found for apoplast targeted Endoglucanase E1 from *Acidothermus cellulolyticus* (fusion with rice α-amylase) where syringe infiltration resulted in recombinant protein accumulation about 15% of TSP and by spraying of about 2% TSP.

Enzymatic activities of all four enzymes calculated on the basis of g fresh weight of plant material correlated with recombinant protein expression data (Fig. 9). BGL4, CBHI and E1 provided comparable enzymatic activities after infiltration and spraying; E3 activity after the spraying was lower.

### Example 4: Storage of cellulase-containing plant biomass as a silage prevents degradation of recombinant cellulase proteins and maintains their enzymatic activity

Plant material inoculated with 1:1000 dilutions of *Agrobacterium* cultures carrying assembled TMV constructs was harvested 11 dpi as a pool of plants. Midribs of leaves were removed and leaf blades were chopped into pieces of about 5 cm² in size using a scalpel blade. Leaf pieces were spread out and dehydrated either at 55°C for 20 h for storage as dry mass, or for 22 h at room temperature (RT) for storage as silage. Dehydrated leaf material (45-58% of fresh weight) was supplemented with 2% (w/dry weight) propionic acid, sodium salt (AppliChem, Darmstadt, Germany) and packed into plastic bags using a commercial vacuum food sealer (Food Saver V2040-I, Sunbeam Products, Inc., Boca Raton, USA) for conservation. This plant material was stored as silage at RT for 1-4 months.

150 mg fresh weight aliquots (9 leaf discs of 1 cm in diameter pooled from 3 comparable leaves) were harvested in triplicates (3 samples from leaves of different age) from identical plant material used for the preparation of silage. Samples were frozen in liquid nitrogen and stored at -80°C.

Silage stored for up to 16 weeks looked brown and had a pH of about 5.3. The determination of protein concentration of TSP samples revealed that TSP samples prepared from silage or from plant material dried at 55°C contained a reduced amount of extractable protein compared to plant material stored at -80°C.

For the analysis of β-glucosidase activity, 1, 2.5, 5 and 25 µg of TSP samples were incubated with 50 mM NaAc pH 5.5, 100 mM NaCl, 5% (v/v) glycerol, 5 mM cellobiose at 50°C for 30 min. The release of glucose was quantified using the GOPOD Kit (Megazyme) and used to calculate enzymatic activity in cellobiase units (CBU) or international units (IU) per mg protein amount analyzed or in CBU and IU per g fresh weight plant material or g commercial enzyme. Due to the low protein amount extractable from dried plant material, only 5 µg TSP of these samples could be analyzed which show only weak enzymatic activities (data not shown).

β-glucosidase activities of TSP samples prepared from plant material upon a storage period of 4, 8 or 16 weeks are compared in Fig. 10A and are very similar, suggesting that the extension of the storage period seems to have no significant negative effect on recombinant BGL4 cellulases. SDS-PAGE demonstrated that recombinant BGL4 remained stable in silage (Fig. 10B and C); in the same time period, native plant protein degrades nearly completely. The recovery of BGL4 proteins from dry biomass was very low due to the poor degradation or poor extractability.

For cytosolic BGL4, the percentage content in TSP extracted from plant material stored at -80°C was estimated to 60%, whereas the percentage content in TSP extracted from silage was 95%. For chloroplast targeted BGL4, the corresponding values are 60% and 90%, respectively (Fig. 10D). Such increase in the content in TSP observed during silage storage can be explained by degradation of native plant proteins.

*Acidothermus cellulolyticus* Endoglucanase E1 and *Trichoderma reesei* Exoglucanase 1 (CBHI) fusions remain stable and retain their enzymatic activity during storage of plant biomass as a silage as shown in Fig. 11A-D. Again, the percentage content of CBHI in TSP extracted from silage (35%) is higher than in TSP extracted from material stored at -80°C (12.5%) (Fig. 11E).

Good protein stability during storage of plant biomass as a silage was observed also for recombinant exocellulase E3 from *Thermobifida fusca* (Fig. 12A-C). For E3 fusion with apple pectinase apoplast TP, the percentage content in TSP extracted from plant material stored at -80°C was estimated to 5% and 18% in TSP extracted from silage. For chloroplast TP-His-EK-E3 fusion these values are 25% and 15%, respectively (Fig. 12D).

Similarly, the endoglucanase E1 from *Thermotoga maritima* shows high expression level after agrobacterial spraying transfection of *Nicotina benthamiana* plants using TMV-based viral vectors (Fig. 14). The recombinant protein remains stable and retains the enzymatic activity during storage of plant biomass as silage as shown in Fig. 14 and 15.

### Example 5: Mixture of plant-produced recombinant cellulases efficiently convert cellulose to glucose

In order to analyze the total enzymatic activity of a mixture of different cellulase classes expressed *in planta* on the conversion of cellulose to glucose, *N. benthamiana* plants were inoculated for the production of cellulases by spraying with 1:1000 dilutions of *Agrobacterium* cultures carrying assembled TMV vectors. For this experiment, each one fusion for β-glucosidase BGL4 (cytosolic), Endoglucanase E1 (fusion with rice α-amylase apoplast TP), exoglucanase CBHI (fusion with barley α-amylase apoplast TP) and exoglucanase E3 (chloroplast TP-His-EK fusion) was chosen on the basis of the following criteria: highest expression *in planta* and highest enzymatic activity (also upon storage of plant material).

To evaluate the total cellulase activity, the percentage of recombinant cellulases in TSP extracts was estimated and a mixture of 20 µg endo- and exocellulases (E1, CBHI and E3 in variable amounts) and 100-1000 µg β-glucosidase (Bgl4) was incubated in 50 mM NaAc pH 5.5, 80 mM NaCl, 8% (v/v) glycerol, 0.02% sodium azide, supplemented with or without 1% (w/v) Avicel as enzyme blanks, respectively, in a final volume of 5 ml at 50°C and 90 rpm in a shaker for 144 hours. Aliquots of the reaction mixture were removed at different time points (2 h, 24 h, 48 h, 72 h, and 144 h) for determination of the glucose concentration using D-glucose (GOPOD-Format) Kit (Megazyme). The activity of the cellulase mixtures was calculated as mg glucose release per mg TSP (Fig. 13A) or per a gram fresh weight plant material (Fig. 13B).

Samples containing 20 µg of a mixture of rice amylase apoplast TP-E1, barley α-amylase apoplast TP-CBHI and chloroplast-TP-His-EK-E3 and 500 µg Bgl4 (ratio (1:1:0):25; (2:1:1):25; and (2:2:1):25)) showed the highest total glucose release. Calculations of cellulase activity as glucose release in mg glucose/mg TSP or mg glucose/g fresh weight plant material indicates highest enzymatic activity for 20 µg of a mixture of rice amylase apo TP-E1, barley α-amylase apo TP-CBHI and chloroplast-TP-His-EK-E3 and 100 µg of Bgl4 (ratio (1:1:1):5) and no significant influence of the alteration of the ratios between endo- and exoglucanases on the amount of glucose production (ratio (1:1:1):25; (2:1:1):25; (1:2:1):25; (1:1:2):25 or (2:2:1):25). A 2:2:1 ratio for E1, CBHI and E3 gave highest sugar release in the analysis of Baker *et al.,* 1998, here a 2:1:1 ratio for E1, CBHI and E3 gives at least no significantly higher glucose production when calculated per g of fresh weight plant material or mg TSP. In summary for this experiment, using cellulases expressed *in planta,* the most efficient cellulose degradation is expected to be observed for a mixture of E1, CBHI, E3 and Bgl4 of (1:1:1):5 or (2:1:1):5 when referred to g of fresh weight plant material.

Concerning the amount of total glucose release, the cost effectiveness of a larger excess of Bgl4 should be taken into consideration, as the release of glucose is increased for samples with ratios (1:1:1):10, (1:1:1):25 and (1:1:1):50 compared to (1:1:1):5 (to minimize a putative inhibition of endo- and exoglucanases by cellobiose and for complete conversion of cellobiose to glucose). The concrete composition of a quarternary mixture of cellulases analyzed giving the highest glucose production will presumably vary with the degree of variation of enzyme expression *in planta* and the estimation of percentage of TSP.

Table 2 summarises experimental data obtained on expression and storage with several cellulolytic enzymes. Protein accumulation and activity levels for analyzed cellulases are given. Plant material was transfected for cellulase expression with 1000-fold dilutions of *Agrobacterium* cultures, harvested 11 dpi and stored under different conditions. Measurements were done on biological triplicates, means and standard deviations are given for analysis at 2 different time points of storage (6 weeks and 12 weeks.

**Table 2:**

| **cellulase** | **storage** | **protein accumulation** | | **activity level** | | |
|---|---|---|---|---|---|---|
| | | %age of TSP | mg recombinant soluble protein / g fresh weight plant material | per mg TSP | per g fresh weight plant material | unit of measurement |
| β-glucosidase Bgl4 from *Humicola grisea* (BAA74958.1) expressed in cytosol | -80°C | 50% ± 0 | 2.96 ± 0.31 | 16.64 ± 2.26 | 98.98 ± 18.71 | IU |
| | silage | 90% ± 0 | 1.88 ± 0.078 | 22.43 ± 4.03 | 46.66 ± 9.78 | |
| Endoglucanase Cel5A from *Thermotoga maritima* (3MMW_D) expressed in cytosol | -80°C | 22.5% ± 10.6 | 0.85 ± 0.24 | 66.44 ± 1.24 | 344.96 ± 25.82 | µmole pNP |
| | silage | 16.25% ± 5.3 | 0.24 ± 0.11 | 82.65 ± 9.09 | 87.98 ±9.29 | (T=24h) |
| Endoglucanse E1 from Acidothermus cellulolyticus (P54583.1) secreted to apoplast (fusion with rice amylase signal peptide) | -80°C | < 1% | < 0.03 | 18.35 ± 0.74 | 61.75 ± 1.68 | µmole pNP |
| | silage | <1% | < 0.008 | 38.062 ± 12.509 | 34.43 ± 17.62 | (T=24h) |
| Exoglucanase 1 (CBHI) from Trichoderma reesei (P62694.1) secretred to apoplast (fusion with barley-α-amylase signal peptide) | -80°C | 12.5% ± 0 | 0.43 ± 0.001 | 7.67 ± 0.552 | 25.87 ±2.15 | µmole pNP |
| | silage | 35% ± 0 | 0.39 ± 0.02 | 18.48 ± 1.12 | 20.45 ± 2.23 | (T=24h) |
| Exoglucanase 1 (CBHI) from Humicola grisea (BAA09785.1) secreted to apoplast (fusion with barley-α-amylase signal peptide) | -80°C | 17.5 ± 10.6 | 0.41 ± 0.25 | 7.17 ± 0.39 | 16.17 ± 0.81 | µmole pNP |
| | silage | 35 ± 21.21 | 0.4 ± 0.24 | 18.4 ± 1.87 | 20.99 ± 2.19 | (T=24h) |
| Exocellulase E3 (AAA62211.1) from Thermobifida fusca targeted to chloroplasts | -80°C | 25% ± 0 | 1.69 ± 0.136 | 1.06 ± 0.25 | 7.13 ± 2.19 | mg glucose |
| | silage | 15% ± 0 | 0.19 ± 0.04 | 3.33 ± 0.36 | 4.32 ± 1.46 | (T=46h) |

### References

Baker, J. O., Ehrman, C.I., Adney, W.S., Thomas, S.R. & Himmel, M.E. (1998) Hydrolysis of cellulose using ternary mixtures of purified celluloses. Appl Biochem Biotechnol 70-72: 395-403.
Himmel, M. E., Adney, W. S., Grohmann, K., & Tucker, M. P. (1994) Thermostable purified endoglucanase from *Acidothermus cellulolyticus* ATCC 43068. U.S. Patent 5.275.944. Sakon, J., Adney, W.S., Himmel, M.E., Thomas, S.R. & Karplus, P.A.(1996) Crystal structure of thermostable family 5 endocellulase E1 from Acidothermus cellulolyticus in complex with cellotetraose. Biochemistry 35(33):10648-60.
Shoemaker, S., Schweickart, V., Ladner, M., Gelfand, D., Kwok, S., Myambo, K. & Innis, M. (1983) Molecular cloning of Exo-Cellobiohydrolase I derived from Trichoderma reesei strain L27. Nature Biotechnology, 1: 691-696.
Takashima S, Nakamura A, Hidaka M, Masaki H, Uozumi T. (1999) Molecular cloning and expression of the novel fungal beta-glucosidase genes from Humicola grisea and Trichoderma reesei. J. Biochem 125, 728-736.
Zhang, S., Lao, G. & Wilson D.B. (1995) Characterization of a Thermomonospora fusca exocellulase. Biochemistry, 34 (10): 3386-95.
Zhang, S., Barr, B.K., & Wilson, D.B. (2000) Effects of noncatalytic residue mutations on substrate specificity and ligand binding of Thermobifida fusca endocellulase cel6A. Eur J Biochem. 67(1): 244-52.
Ghose, T. K. (1987) Measurement of cellulase activities. Pure & Appl. Chem. 59: 257-268.

## Claims

1. A process of degrading plant cell wall components of plant material, comprising:
(i) expressing in plants or parts thereof one or more than one heterologous cell wall-degrading enzyme or
transfecting plants or parts thereof with a nucleic acid molecule comprising a nucleic acid construct containing a nucleotide sequence encoding one or more than one cell wall-degrading enzyme and expressing in said plants or parts thereof said one or more than one cell wall-degrading enzyme;
(ii) harvesting said plants or parts containing said one or more than one cell wall-degrading enzyme;
(iii) storing the harvest as a silage, the minimum storage time being 21 days; and
(iv) treating said plant material with the silage of step (iii) for degrading plant cell wall components of the plant material, wherein said treating comprises adding the silage of step (iii) to said plant material;
wherein said one or more than one cell wall-degrading enzyme is selected from endoglucanases, exoglucanases, exocellulases, β-glucosidases, and combinations thereof.

2. The process according to claim 1, wherein said plant material is different from said silage of step (iii).

3. The process according to claim 1 or 2, wherein a silage containing an endoglucanase, a silage containing an exoglucanase, a silage containing an exocellulase, and a silage containing a β-glucosidase are mixed in desired ratio before treatment of said plant material to obtain a desired ratio of cellulolytic enzymes, and treating said plant material in step (iv) in an aqueous reaction medium with the obtained mixture of silages.

4. The process according to any one of claims 1 to 3, wherein step (i) comprises spraying aerial parts of said plant with an aqueous suspension containing cells of an *Agrobacterium* strain comprising a DNA molecule comprising a nucleic acid construct containing a DNA sequence encoding said one or more than one cell wall-degrading enzyme.

5. The process according to any one of claims 1 to 3, wherein step (i) comprises spraying aerial parts of said plant with an aqueous suspension containing cells of an *Agrobacterium* strain comprising a DNA molecule comprising a nucleic acid construct encoding a DNA or RNA replicon encoding said one or more than one cell wall-degrading enzyme.

6. The process according to any one of claims 1 to 5, wherein step (iii) comprises storing said harvested plants or parts thereof for at least 1 month in an atmosphere of reduced oxygen content.

7. The process according to any one of claims 1 to 6, wherein said plant material is treated with said silage in a reaction medium that is preferably an aqueous reaction medium.

8. A process of producing a bioalcohol such as bioethanol from plant material, comprising:
(a) providing or obtaining, according to steps (i) to (iii) of claim 1, a silage by fermenting plants or plant parts containing expressed one or more than one cell wall-degrading enzyme that may be heterologous to said plants or said plant parts,
(b) treating said plant material, or a product obtained by pre-treatment of said plant material, in an aqueous reaction medium with the silage of step (a) for producing glucose or other cellulose degradation products from cellulose contained in said plant material, wherein said treating comprises adding said silage to said plant material or to said product obtained by pre-treatment of said plant material, and
(c) fermenting the product obtained in the step (b) for producing said bioalcohol from said glucose,
wherein steps (b) and (c) may be performed simultaneously in the same reaction vessel or consecutively, and
wherein said one or more than one cell wall-degrading enzyme is selected from endoglucanases, exoglucanases, exocellulases, β-glucosidases, and combinations thereof.

9. The process according to claim 8, wherein step (a) comprises storing said plants or plant parts containing said one or more than one cell wall degrading enzyme for at least 1 month in an atmosphere of reduced oxygen content.

10. The process according to claim 1 or 8, wherein said plants or parts thereof contain a nucleic acid molecule comprising a nucleic acid construct containing a nucleotide sequence encoding said one or more than one cell wall-degrading enzyme expressed from said construct.

11. The process according to any one of claims 1 to 10, wherein step (iii) of claim 1, or step (a) of claim 8 comprises lactic acid bacteria fermentation and/or acidification by addition of an acid to produce acidic conditions in said silage.

12. The process according to any one of claims 1 to 11, wherein said one or more than one cell wall-degrading enzymes are secretory enzymes in the organism from which they are derived; and/or
wherein said cell wall-degrading enzyme(s) is(are) expressed in the cytosol of cells of said plants or parts thereof, or said cell wall-degrading enzyme(s) is(are) targeted to the apoplast or to plastids of said plant or plant parts.

13. A process of storing cell wall-degrading enzymes, comprising storing, as a silage, plants or plant parts containing a nucleic acid construct containing a nucleotide sequence encoding one or more than one cell wall-degrading enzyme and one or more than one cell wall-degrading enzyme expressed from said construct, said plant or plant parts containing said enzyme(s) targeted to the apoplast or to plastids of said plant or plant parts, wherein said one or more than one cell wall-degrading enzyme is selected from endoglucanases, exoglucanases, exocellulases, β-glucosidases, and combinations thereof.

14. Use of a silage of plants or parts thereof containing one or more than one cell wall-degrading enzyme capable of degrading a plant cell wall component for degrading plant cell wall components of a plant material or for producing a bioalcohol, said use comprising a step of adding said silage to said plant material, wherein said one or more than one cell wall-degrading enzyme is selected from endoglucanases, exoglucanases, exocellulases, β-glucosidases, and combinations thereof.

## Patentansprüche

1. Verfahren zum Abbau pflanzlicher Zellwand-Bestandteile eines Pflanzenmaterials, umfassend:
(i) Exprimieren, in Pflanzen oder in Teilen derselben, eines oder mehr als eines heterologen Zellwand-abbauenden Enzyms oder Transfizieren von Pflanzen oder Teilen derselben mit einem Nukleinsäure-Molekül, das ein Nukleinsäure-Konstrukt umfasst, das eine Nukleotidsequenz enthält, die für ein oder mehr als ein Zellwand-abbauendes Enzym codiert, und Exprimieren in den Pflanzen oder in Teilen derselben des einen oder des mehr als einen Zellwand-abbauenden Enzyms;
(ii) Ernten der Pflanzen oder der Teile, die das eine oder mehr als eine Zellwand-abbauende Enzym enthalten;
(iii) Lagern der Ernte als Silage, wobei die Mindestlagerdauer 21 Tage beträgt; und
(iv) Behandeln des Pflanzenmaterials mit der Silage aus Schritt (iii), um die pflanzlichen Zellwand-Bestandteile des Pflanzenmaterials abzubauen, wobei das Behandeln das Zugeben der Silage aus Schritt (iii) zu dem Pflanzenmaterial umfasst;
wobei das eine oder mehr als eine Zellwand-abbauende Enzym ausgewählt ist aus Endoglucanasen, Exoglucanasen, Exocellulasen, β-Glucosidasen und Kombinationen derselben.

2. Verfahren nach Anspruch 1, wobei sich das Pflanzenmaterial von der Silage aus Schritt (iii) unterscheidet.

3. Verfahren nach Anspruch 1 oder 2, wobei die Silage, die eine Endoglucanase enthält, die Silage, die eine Exoglucanase enthält, die Silage, die eine Exocellulase enthält, und die Silage, die eine β-Glucosidase enthält, vor der Behandlung des Pflanzenmaterials in einem gewünschten Verhältnis gemischt werden, um ein gewünschtes Verhältnis an Cellulose-abbauenden Enzymen zu erhalten und das Pflanzenmaterial in Schritt (iv) in einem wässrigen Reaktionsmedium mit der erhalten Mischung der Silagen behandelt wird.

4. Verfahren nach einem der Ansprüche 1 bis 3, wobei Schritt (i) das Besprühen der oberirdischen Teile der Pflanze mit einer wässrigen Suspension umfasst, die Zellen eines *Agrobacterium-Stamms* enthält, der ein DNA-Molekül umfasst, das ein Nukleinsäure-Konstrukt umfasst, das eine DNA-Sequenz enthält, die für das eine oder mehr als eine Zellwand-abbauende Enzym codiert.

5. Verfahren nach einem der Ansprüche 1 bis 3, wobei Schritt (i) das Besprühen der oberirdischen Teile der Pflanze mit einer wässrigen Suspension umfasst, die Zellen eines *Agrobacterium*-Stamms enthält, die ein DNA-Molekül umfassen, das ein Nukleinsäure-Konstrukt umfasst, das für eine DNA oder ein RNA-Replikon codiert, die (das) für das eine oder mehr als eine Zellwand-abbauende Enzym codiert.

6. Verfahren nach einem der Ansprüche 1 bis 5, wobei Schritt (iii) das Lagern der geernteten Pflanzen oder der Teile derselben für wenigstens einen Monat in einer Atmosphäre mit vermindertem Sauerstoffgehalt umfasst.

7. Verfahren nach einem der Ansprüche 1 bis 6, wobei das Pflanzenmaterial mit der Silage in einem Reaktionsmedium behandelt wird, das bevorzugt ein wässriges Reaktionsmedium ist.

8. Verfahren zur Herstellung eines Bioalkohols, wie Bioethanol, aus Pflanzenmaterial, umfassend:
(a) Bereitstellen oder Erhalten, gemäß den Schritten (i) bis (iii) nach Anspruch 1, einer Silage durch Fermentieren von Pflanzen oder Teilen von Pflanzen, die ein oder mehr als ein exprimiertes Zellwand-abbauendes Enzym enthalten, das zu den Pflanzen oder den Teilen der Pflanzen heterolog sein kann,
(b) Behandeln des Pflanzenmaterials oder eines Produkts, das durch eine Vorbehandlung des Pflanzenmaterials erhalten wurde, in einem wässrigen Reaktionsmedium mit der Silage des Schritts (a), um Glucose oder andere Cellulose-Abbauprodukte aus Cellulose, die in dem Pflanzenmaterial enthalten ist, zu erzeugen, wobei das Behandeln das Zugeben der Silage zu dem Pflanzenmaterial oder dem Produkt, das durch die Vorbehandlung des Pflanzenmaterials erhalten wurde, umfasst, und
(c) Fermentieren des in Schritt (b) erhaltenen Produkts, um Bioalkohol aus der Glucose herzustellen, wobei
die Schritte (b) und (c) gleichzeitig in dem gleichen Reaktionsgefäß oder aufeinanderfolgend durchgeführt werden können und wobei
das eine oder mehr als eine Zellwand-abbauende Enzym ausgewählt ist aus Endoglucanasen, Exoglucanasen, Exocellulasen, β-Glucosidasen und Kombinationen derselben.

9. Verfahren nach Anspruch 8, wobei Schritt (a) das Lagern der Pflanzen oder der Teile der Pflanzen, die das eine oder mehr als eine Zellwand-abbauende Enzym enthalten, für wenigstens einen Monat in einer Atmosphäre mit vermindertem Sauerstoffgehalt umfasst.

10. Verfahren nach Anspruch 1 oder 8, wobei die Pflanzen oder die Teile derselben ein Nukleinsäure-Molekül enthalten, das ein Nukleinsäure-Konstrukt umfasst, das eine Nukleotidsequenz enthält, die für das eine oder mehr als eine Zellwand-abbauende Enzym, das aus dem Konstrukt exprimiert wird, codiert.

11. Verfahren nach einem der Ansprüche 1 bis 10, wobei Schritt (iii) des Anspruchs 1 oder Schritt (a) des Anspruchs 8 eine Fermentation mit Milchsäurebakterien und/oder eine Ansäuerung durch die Zugabe einer Säure umfasst, um saure Bedingungen in der Silage zu erzeugen.

12. Verfahren nach einem der Ansprüche 1 bis 11, wobei das eine oder mehr als eine Zellwand-abbauende Enzym sekretorische Enzyme in dem Organismus sind, von dem diese stammen; und/oder wobei das Zellwand-abbauende Enzym (die Zellwand-abbauenden Enzyme) im Cytosol der Zellen der Pflanzen oder der Teile derselben exprimiert wird (werden) oder wobei das Zellwand-abbauende Enzym (die Zellwand-abbauenden Enzyme) auf die Apoplasten oder die Plastiden der Pflanzen oder der Teile der Pflanzen gerichtet ist (sind).

13. Verfahren zur Lagerung von Zellwand-abbauenden Enzymen, umfassend das Lagern, als Silage, der Pflanzen oder der Teile der Pflanzen, die ein Nukleinsäure-Konstrukt enthalten, das eine Nukleotidsequenz enthält, die für das eine oder mehr als eine Zellwand-abbauende Enzym codiert, und das eine oder mehr als eine Zellwand-abbauende Enzym aus dem Konstrukt exprimiert wird, wobei die Pflanzen oder die Teile der Pflanzen das (die) Enzym(e) enthalten, das (die) auf die Apoplasten oder die Plastiden der Pflanzen oder der Teile der Pflanzen gerichtet ist (sind), wobei das eine oder mehr als eine Zellwand-abbauende Enzym ausgewählt ist aus Endoglucanasen, Exoglucanasen, Exocellulasen, β-Glucosidasen und Kombinationen derselben.

14. Verwendung einer Silage von Pflanzen oder Teilen derselben, die ein oder mehr als ein Zellwand-abbauendes Enzym enthalten, das imstande ist einen pflanzlichen Zellwand-Bestandteil abzubauen, um pflanzliche Zellwand-Bestandteile eines Pflanzenmaterials abzubauen oder Bioalkohol zu erzeugen, wobei die Verwendung einen Schritt des Zugebens der Silage zu dem Pflanzenmaterial umfasst, wobei das eine oder mehr als eine Zellwand-abbauende Enzym ausgewählt ist aus Endoglucanasen, Exoglucanasen, Exocellulasen, β-Glucosidasen und Kombinationen derselben.

## Revendications

1. Procédé de dégradation de composants de parois cellulaires de plante d'un matériau végétal, comprenant :
(i) l'expression, dans des plantes ou des parties de celles-ci, d'une ou plusieurs enzyme(s) dégradant les parois cellulaires hétérologue(s) ou
le transfection de plantes ou de parties de celles-ci avec une molécule d'acide nucléique comprenant un produit d'assemblage d'acide nucléique contenant une séquence de nucléotides codant une ou plusieurs enzyme(s) dégradant les parois cellulaires et l'expression, dans lesdites plantes ou parties de celles-ci, desdites une ou plusieurs enzyme(s) dégradant les parois cellulaires ;
(ii) la récolte desdites plantes ou parties contenant lesdites une ou plusieurs enzyme(s) dégradant les parois cellulaires ;
(iii) le stockage de la récolte sous forme d'ensilage, le temps de stockage minimal étant de 21 jours ; et
(iv) le traitement dudit matériau végétal avec l'ensilage de l'étape (iii) pour dégrader des composants de parois cellulaires de plante du matériau végétal, lequel traitement comprend l'addition de l'ensilage de l'étape (iii) audit matériau végétal ;
dans lequel lesdites une ou plusieurs enzyme(s) dégradant les parois cellulaires est/sont choisie(s) parmi les endoglucanases, les exoglucanases, les exocellulases, les β-glucosidases, et leurs combinaisons.

2. Procédé selon la revendication 1, dans lequel ledit matériau végétal est différent dudit ensilage de l'étape (iii).

3. Procédé selon la revendication 1 ou 2, dans lequel un ensilage contenant une endoglucanase, un ensilage contenant une exoglucanase, un ensilage contenant une exocellulase, et un ensilage contenant une β-glucosidase, sont mélangés en des proportions souhaitées avant traitement dudit matériau végétal pour que soit obtenue une proportion souhaitée d'enzymes cellulolytiques, et le traitement dudit matériau végétal dans l'étape (iv) dans un milieu réactionnel aqueux avec le mélange obtenu d'ensilages.

4. Procédé selon l'une quelconque des revendications 1 à 3, dans lequel l'étape (i) comprend la pulvérisation de parties aériennes de ladite plante avec une suspension aqueuse contenant des cellules d'une souche d'*Agrobacterium* comprenant une molécule d'ADN comprenant un produit d'assemblage d'acide nucléique contenant une séquence d'ADN codant lesdites une ou plusieurs enzyme(s) dégradant les parois cellulaires.

5. Procédé selon l'une quelconque des revendications 1 à 3, dans lequel l'étape (i) comprend la pulvérisation de parties aériennes de ladite plante avec une suspension aqueuse contenant des cellules d'une souche d'*Agrobacterium* comprenant une molécule d'ADN comprenant un produit d'assemblage d'acide nucléique codant un réplicon d'ADN ou d'ARN codant lesdites une ou plusieurs enzyme(s) dégradant les parois cellulaires.

6. Procédé selon l'une quelconque des revendications 1 à 5, dans lequel l'étape (iii) comprend le stockage desdites plantes récoltées ou de parties de celles-ci pendant au moins 1 mois dans une atmosphère à teneur réduite en oxygène.

7. Procédé selon l'une quelconque des revendications 1 à 6, dans lequel ledit matériau végétal est traité avec ledit ensilage dans un milieu réactionnel qui est de préférence un milieu réactionnel aqueux.

8. Procédé pour produire un bioalcool tel que le bioéthanol à partir d'un matériau végétal, comprenant :
(a) la disposition ou l'obtention, conformément aux étapes (i) à (iii) de la revendication 1, d'un ensilage par fermentation de plantes ou de parties de plantes contenant une ou plusieurs enzyme(s) dégradant les parois cellulaires exprimée(s) qui peu(ven)t être hétérologue(s) vis-à-vis desdites plantes ou desdites parties de plantes,
(b) le traitement dudit matériau végétal, ou d'un produit obtenu par prétraitement dudit matériau végétal, dans un milieu réactionnel aqueux avec l'ensilage de l'étape (a) pour produire du glucose ou d'autres produits de dégradation de cellulose à partir de la cellulose contenue dans ledit matériau végétal, lequel traitement comprend l'addition dudit ensilage audit matériau végétal ou audit produit obtenu par prétraitement dudit matériau végétal, et
(c) la fermentation du produit obtenu dans l'étape (b) pour produire ledit bioalcool à partir dudit glucose,
dans lequel les étapes (b) et (c) peuvent être effectuées simultanément dans le même récipient réactionnel ou consécutivement, et
dans lequel lesdites une ou plusieurs enzyme(s) dégradant les parois cellulaires est/sont choisie(s) parmi les endoglucanases, les exoglucanases, les exocellulases, les β-glucosidases, et leurs combinaisons.

9. Procédé selon la revendication 8, dans lequel l'étape (a) comprend le stockage desdites plantes ou parties de plantes contenant lesdites une ou plusieurs enzyme(s) dégradant les parois cellulaires pendant au moins un mois dans une atmosphère à teneur réduite en oxygène.

10. Procédé selon la revendication 1 ou 8, dans lequel lesdites plantes ou parties de celles-ci contiennent une molécule d'acide nucléique comprenant un produit d'assemblage d'acide nucléique contenant une séquence de nucléotides codant lesdites une ou plusieurs enzyme(s) dégradant les parois cellulaires exprimée(s) à partir dudit produit d'assemblage.

11. Procédé selon l'une quelconque des revendications 1 à 10, dans lequel l'étape (iii) de la revendication 1 ou l'étape (a) de la revendication 8 comprend une fermentation avec des ferments lactiques et/ou une acidification par addition d'un acide pour produire des conditions acides dans ledit ensilage.

12. Procédé selon l'une quelconque des revendications 1 à 11, dans lequel lesdites une ou plusieurs enzyme(s) dégradant les parois cellulaires est/sont des enzymes sécrétoires dans l'organisme dont elles dérivent ; et/ou
dans lequel ladite ou lesdites enzyme(s) dégradant les parois cellulaires est/sont exprimée(s) dans le cytosol de cellules desdites plantes ou de parties de celles-ci, ou ladite ou lesdites enzyme(s) dégradant les parois cellulaires est/sont ciblée(s) vers l'apoplaste ou des plastides desdites plantes ou parties de plantes.

13. Procédé de stockage d'enzymes dégradant les parois cellulaires, comprenant le stockage, sous forme d'ensilage, de plantes ou de parties de plantes contenant un produit d'assemblage d'acide nucléique contenant une séquence de nucléotides codant une ou plusieurs enzyme(s) dégradant les parois cellulaires et une ou plusieurs enzyme(s) dégradant les parois cellulaires exprimée (s) à partir dudit produit d'assemblage, lesdites plantes ou parties de plante contenant ladite ou lesdites enzyme(s) ciblée(s) vers l'apoplaste ou les plastides desdites plantes ou parties de plantes, dans lequel lesdites une ou plusieurs enzyme(s) dégradant les parois cellulaires est/sont choisie(s) parmi les endoglucanases, les exoglucanases, les exocellulases, les β-glucosidases, et leurs combinaisons.

14. Utilisation d'un ensilage de plantes ou de parties de celles-ci contenant une ou plusieurs enzyme(s) dégradant les parois cellulaires capable(s) de dégrader un composant de paroi cellulaire de plante pour dégrader les composants de paroi cellulaire de plante d'un matériau végétal ou pour produire un bioalcool, ladite utilisation comprenant une étape d'addition dudit ensilage audit matériau végétal, dans lequel lesdites une ou plusieurs enzyme(s) dégradant les parois cellulaires est/sont choisie(s) parmi les endoglucanases, les exoglucanases, les exocellulases, les β-glucosidases, et leurs combinaisons.


### SEQUENCE LISTING

<110> Nomad Bioscience GmbH
<120> Production, storage and use of cell wall-degrading enzymes
<130> PCT-15987
<150> EP2011008353
   <151> 2011-10-17
<160> 14
<170> PatentIn version 3.3
<210> 1
   <211> 10921
   <212> DNA
   <213> Artificial
<220>
   <223> T-DNA of 5' vector module pICH22455
<400> 1
   tgatgggctg cctgtatcga gtggtgattt tgtgccgagc tgccggtcgg ggagctgttg     60
   gctggctggt ggcaggatat attgtggtgt aaacaaattg acgcttagac aacttaataa    120
   cacattgcgg acgtttttaa tgtactgggg tggatgcagg tcgatctagt aacatagatg    180
   acaccgcgcg cgataattta tcctagtttg cgcgctatat tttgttttct atcgcgtatt    240
   aaatgtataa ttgcgggact ctaatcataa aaacccatct cataaataac gtcatgcatt    300
   acatgttaat tattacatgc ttaacgtaat tcaacagaaa ttatatgata atcatcgcaa    360
   gaccggcaac aggattcaat cttaagaaac tttattgcca aatgtttgaa cgatctgctt    420
   gactctagat ccagagtccc gctcagaaga actcgtcaag aaggcgatag aaggcgatgc    480
   gctgcgaatc gggagcggcg ataccgtaaa gcacgaggaa gcggtcagcc cattcgccgc    540
   caagctcttc agcaatatca cgggtagcca acgctatgtc ctgatagcgg tccgccacac    600
   ccagccggcc acagtcgatg aatccagaaa agcggccatt ttccaccatg atattcggca    660
   agcaggcatc gccatgagtc acgacgagat cctcgccgtc gggcatacgc gccttgagcc    720
   tggcgaacag ttcggctggc gcgagcccct gatgctcttc gtccagatca tcctgatcga    780
   caagaccggc ttccatccga gtacgtgctc gctcgatgcg atgtttcgct tggtggtcga    840
   atgggcaggt agccggatca agcgtatgca gccgccgcat tgcatcagcc atgatggata    900
   ctttctcggc aggagcaagg tgagatgaca ggagatcctg ccccggcact tcgcccaata    960
   gcagccagtc ccttcccgct tcagtgacaa cgtcgagcac agctgcgcaa ggaacgcccg   1020
   tcgtggccag ccacgatagc cgcgctgcct cgtcctggag ttcattcagg gcaccggaca   1080
   ggtcggtctt gacaaaaaga accgggcgcc cctgcgctga cagccggaac acggcggcat   1140
   cagagcagcc gattgtctgt tgtgcccagt catagccgaa tagcctctcc acccaagcgg   1200
   ccggagaacc tgcgtgcaat ccatcttgtt caatcatgcg aaacgatcca gatccggtgc   1260
   agattatttg gattgagagt gaatatgaga ctctaattgg ataccgaggg gaatttatgg   1320
   aacgtcagtg gagcattttt gacaagaaat atttgctagc tgatagtgac cttaggcgac 1380
   ttttgaacgc gcaataatgg tttctgacgt atgtgcttag ctcattaaac tccagaaacc 1440
   cgcggctgag tggctccttc aacgttgcgg ttctgtcagt tccaaacgta aaacggcttg 1500
   tcccgcgtca tcggcggggg tcataacgtg actcccttaa ttctccgctc atggtaccac 1560
   gcgtttcgac aaaatttaga acgaacttaa ttatgatctc aaatacattg atacatatct 1620
   catctagatc taggttatca ttatgtaaga aagttttgac gaatatggca cgacaaaatg 1680
   gctagactcg atgtaattgg tatctcaact caacattata cttataccaa acattagtta 1740
   gacaaaattt aaacaactat tttttatgta tgcaagagtc agcatatgta taattgattc 1800
   agaatcgttt tgacgagttc ggatgtagta gtagccatta tttaatgtac atactaatcg 1860
   tgaatagtga atatgatgaa acattgtatc ttattgtata aatatccata aacacatcat 1920
   gaaagacact ttctttcacg gtctgaatta attatgatac aattctaata gaaaacgaat 1980
   taaattacgt tgaattgtat gaaatctaat tgaacaagcc aaccacgacg acgactaacg 2040
   ttgcctggat tgactcggtt taagttaacc actaaaaaaa cggagctgtc atgtaacacg 2100
   cggatcgagc aggtcacagt catgaagcca tcaaagcaaa agaactaatc caagggctga 2160
   gatgattaat tagtttaaaa attagttaac acgagggaaa aggctgtctg acagccaggt 2220
   cacgttatct ttacctgtgg tcgaaatgat tcgtgtctgt cgattttaat tatttttttg 2280
   aaaggccgaa aataaagttg taagagataa acccgcctat ataaattcat atattttcct 2340
   ctccgctttg aagttttagt tttattgcaa caacaacaac aaattacaat aacaacaaac 2400
   aaaatacaaa caacaacaac atggcacaat ttcaacaaac aattgacatg caaactctcc 2460
   aagccgctgc gggacgcaac agcttggtga atgatttggc atctcgtcgc gtttacgata 2520
   atgcagtcga ggagctgaat gctcgttcca gacgtcccaa ggtaatagga actttctgga 2580
   tctactttat ttgctggatc tcgatcttgt tttctcaatt tccttgagat ctggaattaa 2640
   ttcgtttaat ttggatctgt gaacctccac taaatctttt ggttttacta gaatcgatct 2700
   aagttgaccg atcagttagc tcgattatag ctaccagaat ttggcttgac cttgatggag 2760
   agatccatgt tcatgttacc tgggaaatga tttgtatatg tgaattgaaa tctgaactgt 2820
   tgaagttaga ttgaatctga acactgtcaa tgttagattg aatctgaaca ctgtttaagg 2880
   ttagatgaag tttgtgtata gattcttcga aactttagga tttgtagtgt cgtacgttga 2940
   acagaaagct atttctgatt caatcagggt ttatttgact gtattgaact ctttttgtgt 3000
   gtttgcaggt ccacttctcc aaggcagtgt ctacggaaca gaccctgatt gcaacaaacg 3060
   catatccgga gttcgagatt tcctttactc atacgcaatc cgctgtgcac tccttggccg 3120
   gaggccttcg gtcacttgag ttggagtatc tcatgatgca agttccgttc ggttctctga 3180
   cgtacgacat cggcggtaac ttttccgcgc accttttcaa agggcgcgat tacgttcact 3240
   gctgcatgcc taatctggat gtacgtgaca ttgctcgcca tgaaggacac aaggaagcta 3300
   tttacagtta tgtgaatcgt ttgaaaaggc agcagcgtcc tgtgcctgaa taccagaggg   3360
   cagctttcaa caactacgct gagaacccgc acttcgtcca ttgcgacaaa cctttccaac   3420
   agtgtgaatt gacgacagcg tatggcactg acacctacgc tgtagctctc catagcattt   3480
   atgatatccc tgttgaggag ttcggttctg cgctactcag gaagaatgtg aaaacttgtt   3540
   tcgcggcctt tcatttccat gagaatatgc ttctagattg tgatacagtc acactcgatg   3600
   agattggagc tacgttccag aaatcaggta acattcctta gttacctttc ttttcttttt   3660
   ccatcataag tttatagatt gtacatgctt tgagattttt ctttgcaaac aatctcaggt   3720
   gataacctga gcttcttctt ccataatgag agcactctca attacaccca cagcttcagc   3780
   aacatcatca agtacgtgtg caagacgttc ttccctgcta gtcaacgctt cgtgtaccac   3840
   aaggagttcc tggtcactag agtcaacact tggtactgca agttcacgag agtggatacg   3900
   ttcactctgt tccgtggtgt gtaccacaac aatgtggatt gcgaagagtt ttacaaggct   3960
   atggacgatg cgtggcacta caaaaagacg ttagcaatgc ttaatgccga gaggaccatc   4020
   ttcaaggata acgctgcgtt aaacttctgg ttcccgaagg tgctcttgaa attggaagtc   4080
   ttcttttgtt gtctaaacct atcaatttct ttgcggaaat ttatttgaag ctgtagagtt   4140
   aaaattgagt cttttaaact tttgtaggtg agagacatgg ttatcgtccc tctctttgac   4200
   gcttctatca caactggtag gatgtctagg agagaggtta tggtgaacaa ggacttcgtc   4260
   tacacggtcc taaatcacat caagacctat caagctaagg cactgacgta cgcaaacgtg   4320
   ctgagcttcg tggagtctat taggtctaga gtgataatta acggtgtcac tgccaggtaa   4380
   gttgttactt atgattgttt tcctctctgc tacatgtatt ttgttgttca tttctgtaag   4440
   atataagaat tgagttttcc tctgatgata ttattaggtc tgaatgggac acagacaagg   4500
   caattctagg tccattagca atgacattct tcctgatcac gaagctgggt catgtgcaag   4560
   atgaaataat cctgaaaaag ttccagaagt tcgacagaac caccaatgag ctgatttgga   4620
   caagtctctg cgatgccctg atgggggtta ttccctcggt caaggagacg cttgtgcgcg   4680
   gtggttttgt gaaagtagca gaacaagcct tagagatcaa ggttagtatc atatgaagaa   4740
   atacctagtt tcagttgatg aatgctattt tctgacctca gttgttctct tttgagaatt   4800
   atttcttttc taatttgcct gatttttcta ttaattcatt aggttcccga gctatactgt   4860
   accttcgccg accgattggt actacagtac aagaaggcgg aggagttcca atcgtgtgat   4920
   ctttccaaac ctctagaaga gtcagagaag tactacaacg cattatccga gctatcagtg   4980
   cttgagaatc tcgactcttt tgacttagag gcgtttaaga ctttatgtca gcagaagaat   5040
   gtggacccgg atatggcagc aaaggtaaat cctggtccac acttttacga taaaaacaca   5100
   agattttaaa ctatgaactg atcaataatc attcctaaaa gaccacactt ttgttttgtt   5160
   tctaaagtaa tttttactgt tataacaggt ggtcgtagca atcatgaagt cagaattgac   5220
   gttgcctttc aagaaaccta cagaagagga aatctcggag tcgctaaaac caggagaggg   5280
   gtcgtgtgca gagcataagg aagtgttgag cttacaaaat gatgctccgt tcccgtgtgt   5340
   gaaaaatcta gttgaaggtt ccgtgccggc gtatggaatg tgtcctaagg gtggtggttt 5400
   cgacaaattg gatgtggaca ttgctgattt ccatctcaag agtgtagatg cagttaaaaa 5460
   gggaactatg atgtctgcgg tgtacacagg gtctatcaaa gttcaacaaa tgaagaacta 5520
   catagattac ttaagtgcgt cgctggcagc tacagtctca aacctctgca aggtaagagg 5580
   tcaaaaggtt tccgcaatga tccctctttt tttgtttctc tagtttcaag aatttgggta 5640
   tatgactaac ttctgagtgt tccttgatgc atatttgtga tgagacaaat gtttgttcta 5700
   tgttttaggt gcttagagat gttcacggcg ttgacccaga gtcacaggag aaatctggag 5760
   tgtgggatgt taggagagga cgttggttac ttaaacctaa tgcgaaaagt cacgcgtggg 5820
   gtgtggcaga agacgccaac cacaagttgg ttattgtgtt actcaactgg gatgacggaa 5880
   agccggtttg tgatgagaca tggttcaggg tggcggtgtc aagcgattcc ttgatatatt 5940
   cggatatggg aaaacttaag acgctcacgt cttgcagtcc aaatggtgag ccaccggagc 6000
   ctaacgccaa agtaattttg gtcgatggtg ttcccggttg tggaaaaacg aaggagatta 6060
   tcgaaaaggt aagttctgca tttggttatg ctccttgcat tttaggtgtt cgtcgctctt 6120
   ccatttccat gaatagctaa gatttttttt ctctgcattc attcttcttg cctcagttct 6180
   aactgtttgt ggtatttttg ttttaattat tgctacaggt aaacttctct gaagacttga 6240
   ttttagtccc tgggaaggaa gcttctaaga tgatcatccg gagggccaac caagctggtg 6300
   tgataagagc ggataaggac aatgttagaa cggtggattc cttcttgatg catccttcta 6360
   gaagggtgtt taagaggttg tttatcgatg aaggactaat gctgcataca ggttgtgtaa 6420
   atttcctact gctgctatct caatgtgacg tcgcatatgt gtatggggac acaaagcaaa 6480
   ttccgttcat ttgcagagtc gcgaactttc cgtatccagc gcattttgca aaactcgtcg 6540
   ctgatgagaa ggaagtcaga agagttacgc tcaggtaaag caactgtgtt ttaatcaatt 6600
   tcttgtcagg atatatggat tataacttaa tttttgagaa atctgtagta tttggcgtga 6660
   aatgagtttg ctttttggtt tctcccgtgt tataggtgcc cggctgatgt tacgtatttc 6720
   cttaacaaga agtatgacgg ggcggtgatg tgtaccagcg cggtagagag atccgtgaag 6780
   gcagaagtgg tgagaggaaa gggtgcattg aacccaataa ccttaccgtt ggagggtaaa 6840
   attttgacct tcacacaagc tgacaagttc gagttactgg agaagggtta caaggtaaag 6900
   tttccaactt tcctttacca tatcaaacta aagttcgaaa ctttttattt gatcaacttc 6960
   aaggccaccc gatctttcta ttcctgatta atttgtgatg aatccatatt gacttttgat 7020
   ggttacgcag gatgtgaaca ctgtgcacga ggtgcaaggg gagacgtacg agaagactgc 7080
   tattgtgcgc ttgacatcaa ctccgttaga gatcatatcg agtgcgtcac ctcatgtttt 7140
   ggtggcgctg acaagacaca caacgtgttg taaatattac accgttgtgt tggacccgat 7200
   ggtgaatgtg atttcagaaa tggagaagtt gtccaatttc cttcttgaca tgtatagagt 7260
   tgaagcaggt ctgtctttcc tatttcatat gtttaatcct aggaatttga tcaattgatt 7320
   gtatgtatgt cgatcccaag actttcttgt tcacttatat cttaactctc tctttgctgt    7380
   ttcttgcagg tgtccaatag caattacaaa tcgatgcagt attcagggga cagaacttgt    7440
   ttgttcagac gcccaagtca ggagattggc gagatatgca attttactat gacgctcttc    7500
   ttcccggaaa cagtactatt ctcaatgaat ttgatgctgt tacgatgaat ttgagggata    7560
   tttccttaaa cgtcaaagat tgcagaatcg acttctccaa atccgtgcaa cttcctaaag    7620
   aacaacctat tttcctcaag cctaaaataa gaactgcggc agaaatgccg agaactgcag    7680
   gtaaaatatt ggatgccaga cgatattctt tcttttgatt tgtaactttt tcctgtcaag    7740
   gtcgataaat tttatttttt ttggtaaaag gtcgataatt tttttttgga gccattatgt    7800
   aattttccta attaactgaa ccaaaattat acaaaccagg tttgctggaa aatttggttg    7860
   caatgatcaa aagaaacatg aatgcgccgg atttgacagg gacaattgac attgaggata    7920
   ctgcatctct ggtggttgaa aagttttggg attcgtatgt tgacaaggaa tttagtggaa    7980
   cgaacgaaat gaccatgaca agggagagct tctccaggta aggacttctc atgaatatta    8040
   gtggcagatt agtgttgtta aagtctttgg ttagataatc gatgcctcct aattgtccat    8100
   gttttactgg ttttctacaa ttaaaggtgg ctttcgaaac aagagtcatc tacagttggt    8160
   cagttagcgg actttaactt tgtggatttg ccggcagtag atgagtacaa gcatatgatc    8220
   aagagtcaac caaagcaaaa gttagacttg agtattcaag acgaatatcc tgcattgcag    8280
   acgatagtct accattcgaa aaagatcaat gcgattttcg gtccaatgtt ttcagaactt    8340
   acgaggatgt tactcgaaag gattgactct tcgaagtttc tgttctacac cagaaagaca    8400
   cctgcacaaa tagaggactt cttttctgac ctagactcaa cccaggcgat ggaaattctg    8460
   gaactcgaca tttcgaagta cgataagtca caaaacgagt tccattgtgc tgtagagtac    8520
   aagatctggg aaaagttagg aattgatgag tggctagctg aggtctggaa acaaggtgag    8580
   ttcctaagtt ccattttttt gtaatccttc aatgttattt taacttttca gatcaacatc    8640
   aaaattaggt tcaattttca tcaaccaaat aatatttttc atgtatatat aggtcacaga    8700
   aaaacgacct tgaaagatta tacggccgga atcaaaacat gtctttggta tcaaaggaaa    8760
   agtggtgatg tgacaacctt tattggtaat accatcatca ttgccgcatg tttgagctca    8820
   atgatcccca tggacaaagt gataaaggca gctttttgtg gagacgatag cctgatttac    8880
   attcctaaag gtttagactt gcctgatatt caggcgggcg cgaacctcat gtggaacttc    8940
   gaggccaaac tcttcaggaa gaagtatggt tacttctgtg gtcgttatgt tattcaccat    9000
   gatagaggag ccattgtgta ttacgatccg cttaaactaa tatctaagtt aggttgtaaa    9060
   catattagag atgttgttca cttagaagag ttacgcgagt ctttgtgtga tgtagctagt    9120
   aacttaaata attgtgcgta tttttcacag ttagatgagg ccgttgccga ggttcataag    9180
   accgcggtag gcggttcgtt tgctttttgt agtataatta agtatttgtc agataagaga    9240
   ttgtttagag atttgttctt tgtttgataa tgtcgatagt ctcgtacgaa cctaaggtga    9300
   gtgatttcct caatctttcg aagaaggaag agatcttgcc gaaggctcta acgaggttaa    9360
   aaaccgtgtc tattagtact aaagatatta tatctgtcaa ggagtcggag actttgtgtg    9420
   atatagattt gttaatcaat gtgccattag ataagtatag atatgtgggt atcctaggag    9480
   ccgtttttac cggagagtgg ctagtgccag acttcgttaa aggtggagtg acgataagtg    9540
   tgatagataa gcgtctggtg aactcaaagg agtgcgtgat tggtacgtac agagccgcag    9600
   ccaagagtaa gaggttccag ttcaaattgg ttccaaatta ctttgtgtcc accgtggacg    9660
   caaagaggaa gccgtggcag gtaaggattt ttatgatata gtatgcttat gtattttgta    9720
   ctgaaagcat atcctgcttc attgggatat tactgaaagc atttaactac atgtaaactc    9780
   acttgatgat caataaactt gattttgcag gttcatgttc gtatacaaga cttgaagatt    9840
   gaggcgggtt ggcagccgtt agctctggaa gtagtttcag ttgctatggt caccaataac    9900
   gttgtcatga agggtttgag ggaaaaggtc gtcgcaataa atgatccgga cgtcgaaggt    9960
   ttcgaaggta agccatcttc ctgcttattt ttataatgaa catagaaata ggaagttgtg   10020
   cagagaaact aattaacctg actcaaaatc taccctcata attgttgttt gatattggtc   10080
   ttgtattttg caggtgtggt tgacgaattc gtcgattcgg ttgcagcatt taaagcggtt   10140
   gacaacttta aaagaaggaa aaagaaggtt gaagaaaagg gtgtagtaag taagtataag   10200
   tacagaccgg agaagtacgc cggtcctgat tcgtttaatt tgaaagaaga aaacgtctta   10260
   caacattaca aacccgaatc agtaccagta tttcgataag aaacaagaaa ccatgggcag   10320
   cagccaccac caccaccacc acagcagcgg cgacgacgac gacaaaggta agtttcattt   10380
   tcataattac acaaatttag atttgatttt tgttgtgccc caactggggt aacctttgag   10440
   ttctctcagt tgggggcgta gggcccagcg gccgctctag ctagagtcaa gcagatcgtt   10500
   caaacatttg gcaataaagt ttcttaagat tgaatcctgt tgccggtctt gcgatgatta   10560
   tcatataatt tctgttgaat tacgttaagc atgtaataat taacatgtaa tgcatgacgt   10620
   tatttatgag atgggttttt atgattagag tcccgcaatt atacatttaa tacgcgatag   10680
   aaaacaaaat atagcgcgca aactaggata aattatcgcg cgcggtgtca tctatgttac   10740
   tagatcgacc agcttagatc agattgtcgt ttcccgcctt cagtttaaac tatcagtgtt   10800
   tgacaggata tattggcggg taaacctaag agaaaagagc gtttattaga ataatcggat   10860
   atttaaaagg gcgtgaaaag gtttatccgt tcgtccattt gtatgtgcca accacagggt   10920
   t                                                                   10921
<210> 2
   <211> 2568
   <212> DNA
   <213> Artificial
<220>
   <223> T-DNA of 3' vector module pNMD231
<400> 2
   cctgtggttg gcacatacaa atggacgaac ggataaacct tttcacgccc ttttaaatat 60
   ccgattattc taataaacgc tcttttctct taggtttacc cgccaatata tcctgtcaaa     120
   cactgatagt ttaaactgaa ggcgggaaac gacaatctga tctaagctag ggtacctcga     180
   agccgcggtg cgggtgccag ggcgtgccct tgggctcccc gggcgcgtac tccacctcac     240
   ccatctttta ttacatgttt gaacttcaac aatttatgac tttttgttct tattgttgca     300
   ggtgcaggtg gcggatattg gcacacttct ggtagagaaa tcctagatgc aaacaatgtg     360
   cctgttcgca tagctgggat taactggttc ggcttcgaga cttgtaacta cgtagtccac     420
   ggactttgga gtcgggacta tagaagcatg ctcgatcaaa tcaaaagcct gggatacaac     480
   acgattcgac tgccatactc tgatgacatt ctgaaacctg gaaccatgcc gaactccatc     540
   aacttctatc aaatgaacca agatctgcaa gggctgacta gcttgcaagt gatggataag     600
   atagtagcct acgctggcca aattgggctt agaataatcc tcgatagaca tcgtcctgat     660
   tgctctggac aatctgcact ctggtataca tcctctgtct ctgaagctac ctggatttcc     720
   gatttgcaag cactagccca gagatacaaa ggaaatccaa ccgtagtcgg ctttgacttg     780
   cataacgaac cacatgatcc tgcttgttgg ggatgtggtg atccatcaat tgactggcgt     840
   ttagctgctg aaagagctgg aaacgctgtc ttatccgtga atcccaatct tctcatcttc     900
   gtagagggag ttcagtcata caatggcgat tcttactggt ggggagggaa tctacaaggc     960
   gctggacaat accctgtagt tctaaacgta cccaaccgat tggtgtattc tgcccatgac    1020
   tatgcaacat ccgtttatcc gcaaacctgg ttttctgatc ccacatttcc gaacaatatg    1080
   cctggcattt ggaacaagaa ctggggatat ctgttcaacc aaaacattgc tcccgtatgg    1140
   cttggcgagt ttggaacaac tttacaaagc acaacggacc agacctggct taagacatta    1200
   gtgcaatatc ttagaccgac cgcacaatat ggtgctgata gctttcaatg gacgttctgg    1260
   agttggaatc ctgattctgg cgatacgggt ggaatactta aggatgactg gcaaaccgtt    1320
   gacactgtaa aggacggata ccttgctccc ataaagtcta gcatctttga tcccgttggt    1380
   gcatctgctt ctccaagttc acaaccaagt ccctctgttt caccaagtcc atcaccatca    1440
   cctagtgctt ctagaacacc aactcctact ccaacaccaa ctgcatctcc aactcctaca    1500
   ttgacaccta ccgcaacacc tacacctact gcttcaccta ctccatcacc tactgccgct    1560
   tctggagcta gatgcactgc ttcttatcaa gtcaactctg attggggaaa cggctttact    1620
   gtgaccgttg ctgtcacgaa ttctggaagt gtggcaacaa agacttggac tgtttcttgg    1680
   acgtttgggg gaaatcaaac cattacgaac tcttggaatg ctgccgtaac acaaaatggg    1740
   caatccgtta ccgctcggaa catgtcatac aataacgtca tccaacctgg gcaaaacaca    1800
   acgttcggct ttcaagcctc ctataccggt tcaaatgctg cacctactgt tgcttgcgct    1860
   gcatcttaag cttgaagact aggcgtggtg cgcacgatag cgcatagtgt ttttctctcc    1920
   acttgaatcg aagagataga cttacggtgt aaatccgtag gggtggcgta aaccaaatta    1980
   cgcaatgttt tgggttccat ttaaatcgaa accccttatt tcctggatca cctgttaacg    2040
   cacgtttgac gtgtattaca gtgggaataa gtaaaagtga gaggttcgaa tcctccctaa    2100
   ccccgggtag gggcccagcg gccgctctag ctagagtcaa gcagatcgtt caaacatttg   2160
   gcaataaagt ttcttaagat tgaatcctgt tgccggtctt gcgatgatta tcatataatt   2220
   tctgttgaat tacgttaagc atgtaataat taacatgtaa tgcatgacgt tatttatgag   2280
   atgggttttt atgattagag tcccgcaatt atacatttaa tacgcgatag aaaacaaaat   2340
   atagcgcgca aactaggata aattatcgcg cgcggtgtca tctatgttac tagatcgact   2400
   agtgagctct gcatccaccc cagtacatta aaaacgtccg caatgtgtta ttaagttgtc   2460
   taagcgtcaa tttgtttaca ccacaatata tcctgccacc agccagccaa cagctccccg   2520
   accggcagct cggcacaaaa tcaccactcg atacaggcag cccatcag                 2568
<210> 3
   <211> 2493
   <212> DNA
   <213> Artificial
<220>
   <223> T-DNA of 3' vector module pNMD241
<400> 3
   cctgtggttg gcacatacaa atggacgaac ggataaacct tttcacgccc ttttaaatat     60
   ccgattattc taataaacgc tcttttctct taggtttacc cgccaatata tcctgtcaaa    120
   cactgatagt ttaaactgaa ggcgggaaac gacaatctga tctaagctag ggtacctcga    180
   agccgcggtg cgggtgccag ggcgtgccct tgggctcccc gggcgcgtac tccacctcac    240
   ccatctttta ttacatgttt gaacttcaac aatttatgac tttttgttct tattgttgca    300
   ggtcaatctg cttgcactct gcaatccgaa acgcatccac ctttgacttg gcaaaagtgt    360
   tcctctggtg gaacgtgtac ccagcaaacc ggttctgtag tgattgacgc aaattggaga    420
   tggacacacg ctaccaatag ctctacgaac tgctatgatg ggaatacctg gtctagcaca    480
   ctatgccctg ataacgaaac ctgtgcaaag aattgctgtc ttgatggtgc tgcctatgct    540
   tcaacttacg gtgtcacaac tagcggcaat tctctttcca tcggcttcgt cacacaaagt    600
   gcccaaaaga acgttggagc acggctttac cttatggctt ccgacacgac ctatcaagaa    660
   ttcacgctgc ttgggaatga gttttccttt gatgtggacg tttctcaact cccatgcgga    720
   cttaacggag cactttactt tgtgtctatg gacgctgatg gaggcgtttc taagtatccg    780
   actaacactg ctggtgctaa gtacggaacc ggttactgcg attcacaatg ccctagagat    840
   ctgaagttca tcaacggaca agcaaacgtc gagggctggg aaccttcttc caataacgca    900
   aacactggca ttggtggcca cggatcatgt tgttctgaga tggacatttg ggaagcaaac    960
   tcaatctccg aagcacttac cccacatccg tgtacaactg ttggccaaga aatctgtgag   1020
   ggtgatggat gtggtggcac ttactctgac aatcgctatg gtggaacctg tgatccagat   1080
   ggatgcgatt ggaaccccta taggcttgga aacacgtctt tctatggacc tggctctagc   1140
   tttacccttg ataccactaa gaaactcacc gtggtaacgc aattcgagac tagcggagcc   1200
   ataaaccgat attacgtaca gaatggcgtt acgtttcaac aacccaatgc cgagttgggc   1260
   tcatactctg ggaatgagtt gaatgatgac tactgtactg ctgaagaggc tgaattcggt   1320
   ggaagttcct ttagcgataa gggaggccta acccagttta agaaagcaac gtctggtggg   1380
   atggttctcg tcatgagtct atgggatgac tactatgcta acatgctgtg gctcgattcc   1440
   acatatccca ccaatgagac tagttcaact cctggagctg ttcgtggatc ctgttcaact   1500
   agctctggtg ttcctgccca agtagaatct caaagtccca acgcaaaggt gaccttttcc   1560
   aacataaagt tcggaccgat tggctcaact ggaaatccat ctgggggaaa tccacctggt   1620
   ggcaatagag gaacaactac aactagaagg cctgctacta ccactggttc atctcctggt   1680
   ccaactcaat ctcattatgg ccaatgcggt ggcattggct attctggtcc tactgtgtgt   1740
   gctagtggaa ccacatgcca agttctcaac ccctattact cccaatgcct ctaagcttga   1800
   agactaggcg tggtgcgcac gatagcgcat agtgtttttc tctccacttg aatcgaagag   1860
   atagacttac ggtgtaaatc cgtaggggtg gcgtaaacca aattacgcaa tgttttgggt   1920
   tccatttaaa tcgaaacccc ttatttcctg gatcacctgt taacgcacgt ttgacgtgta   1980
   ttacagtggg aataagtaaa agtgagaggt tcgaatcctc cctaaccccg ggtaggggcc   2040
   cagcggccgc tctagctaga gtcaagcaga tcgttcaaac atttggcaat aaagtttctt   2100
   aagattgaat cctgttgccg gtcttgcgat gattatcata taatttctgt tgaattacgt   2160
   taagcatgta ataattaaca tgtaatgcat gacgttattt atgagatggg tttttatgat   2220
   tagagtcccg caattataca tttaatacgc gatagaaaac aaaatatagc gcgcaaacta   2280
   ggataaatta tcgcgcgcgg tgtcatctat gttactagat cgactagtga gctctgcatc   2340
   caccccagta cattaaaaac gtccgcaatg tgttattaag ttgtctaagc gtcaatttgt   2400
   ttacaccaca atatatcctg ccaccagcca gccaacagct ccccgaccgg cagctcggca   2460
   caaaatcacc actcgataca ggcagcccat cag                                2493
<210> 4
   <211> 2679
   <212> DNA
   <213> Artificial
<220>
   <223> T-DNA of 3' vector module pNMD251
<400> 4
   cctgtggttg gcacatacaa atggacgaac ggataaacct tttcacgccc ttttaaatat     60
   ccgattattc taataaacgc tcttttctct taggtttacc cgccaatata tcctgtcaaa    120
   cactgatagt ttaaactgaa ggcgggaaac gacaatctga tctaagctag ggtacctcga    180
   agccgcggtg cgggtgccag ggcgtgccct tgggctcccc gggcgcgtac tccacctcac    240
   ccatctttta ttacatgttt gaacttcaac aatttatgac tttttgttct tattgttgca    300
   ggtgcaggct gttctgttga ttacaccgtg aattcttggg gaactggatt cacggctaac    360
   gttaccatca cgaatcttgg ttctgccata aacggatgga cattagagtg ggacttccct    420
   ggcaaccagc aagttaccaa cctgtggaat ggcacgtata cccagtctgg gcaacacgtt   480
   tctgtttcta atgctcctta caacgcatct atccctgcaa atggcactgt cgaattcggc   540
   tttaatggga gctattctgg aagcaacgac atacctagct cttttaagct gaacggtgta   600
   acatgtgacg ggagcgacga tccagatcct gaaccttctc catcaccttc tccatctccc   660
   agccctactg atcctgatga acctggtgga ccaaccaatc ctccaacaaa ccctggcgag   720
   aaagtggaca atcccttcga gggtgccaaa ctttacgtaa accctgtatg gagtgctaaa   780
   gctgcagctg aacctggtgg atctgctgta gccaatgaat ctactgctgt ttggctggat   840
   agaatcggtg ccatagaggg aaacgattct cctacaactg ggtctatggg tcttagagat   900
   catctcgagg aagccgtaag acaaagtggt ggcgatcccc ttaccatcca agttgtgatc   960
   tacaatctac ctggtcgcga ctgtgctgca cttgcttcta atggagaatt gggacctgat  1020
   gaactggata gatacaagtc cgagtacatt gatcccattg ctgacatcat gtgggacttc  1080
   gctgactatg agaacctacg aatcgtcgcc atcattgaga tagactccct ccctaatcta  1140
   gtcactaatg tcggaggcaa cggagggact gagctatgtg catacatgaa gcaaaacgga  1200
   ggctacgtca atggagttgg ctatgccctt agaaagttgg gtgagattcc taacgtctac  1260
   aactacatcg atgccgctca ccatggctgg ataggctggg attctaactt tggaccctcc  1320
   gtggacatct tctacgaagc tgcaaatgcc tctggtagca ccgttgacta tgtgcatgga  1380
   ttcatctcta acacggcaaa ctatagcgct actgttgaac cgtatctaga cgtaaacggc  1440
   accgtgaatg ggcaactgat aaggcaatct aagtgggtcg actggaacca atacgttgat  1500
   gagttgagtt tcgtccaaga cttgaggcaa gctctcatag ccaaagggtt tcgttctgat  1560
   atcggcatgc tcatagacac ttctagaaac ggatggggag gccctaatcg tccaactgga  1620
   cctagctcat ctacggacct taacacatac gttgacgagt cacgaattga tagacgcata  1680
   caccctggaa actggtgcaa ccaagctggt gctggcttgg gtgaaagacc taccgtgaac  1740
   ccagctcctg gtgttgacgc atacgtttgg gttaagcctc cgggagaatc tgatggagct  1800
   tccgaggaaa tccctaatga tgaggggaag ggcttcgata gaatgtgcga tcctacttac  1860
   caaggcaacg ctagaaacgg caacaatcca tctggtgctc tacctaatgc tcccatatct  1920
   gggcactggt ttctgcccaa atttcgggag cttctagcta atgcttaccc tcccctctaa  1980
   gcttgaagac taggcgtggt gcgcacgata gcgcatagtg tttttctctc cacttgaatc  2040
   gaagagatag acttacggtg taaatccgta ggggtggcgt aaaccaaatt acgcaatgtt  2100
   ttgggttcca tttaaatcga aaccccttat ttcctggatc acctgttaac gcacgtttga  2160
   cgtgtattac agtgggaata agtaaaagtg agaggttcga atcctcccta accccgggta  2220
   ggggcccagc ggccgctcta gctagagtca agcagatcgt tcaaacattt ggcaataaag  2280
   tttcttaaga ttgaatcctg ttgccggtct tgcgatgatt atcatataat ttctgttgaa  2340
   ttacgttaag catgtaataa ttaacatgta atgcatgacg ttatttatga gatgggtttt  2400
   tatgattaga gtcccgcaat tatacattta atacgcgata gaaaacaaaa tatagcgcgc 2460
   aaactaggat aaattatcgc gcgcggtgtc atctatgtta ctagatcgac tagtgagctc 2520
   tgcatccacc ccagtacatt aaaaacgtcc gcaatgtgtt attaagttgt ctaagcgtca 2580
   atttgtttac accacaatat atcctgccac cagccagcca acagctcccc gaccggcagc 2640
   tcggcacaaa atcaccactc gatacaggca gcccatcag 2679
<210> 5
   <211> 1978
   <212> DNA
   <213> Artificial
<220>
   <223> T-DNA of 3' vector module pNMD910
<400> 5
   cctgtggttg gcacatacaa atggacgaac ggataaacct tttcacgccc ttttaaatat     60
   ccgattattc taataaacgc tcttttctct taggtttacc cgccaatata tcctgtcaaa    120
   cactgatagt ttaaactgaa ggcgggaaac gacaatctga tctaagctag ggtacctcga    180
   agccgcggtg cgggtgccag ggcgtgccct tgggctcccc gggcgcgtac tccacctcac    240
   ccatctttta ttacatgttt gaacttcaac aatttatgac tttttgttct tattgttgca    300
   ggtatgagcc taccacctga ctttaagtgg ggttttgcta ccgccgctta tcagatagaa    360
   ggatctgtga atgaggatgg tagaggacct tctatttggg acaccttttg tgcaatccct    420
   ggtaaaatag ctgacggaag ctcaggagct gttgcctgtg attcttacaa gcggacaaag    480
   gaagatattg ccttgttaaa agagcttggt gctaactcct atagattttc catttcttgg    540
   tcccgaatta tccctctggg cggaagaaat gaccctatca accaaaaagg catcgatcat    600
   tacgtgaagt tcgtagacga tctaatcgag gccggaatta ctccattcat tactctcttc    660
   cactgggatc ttcccgatgc attggataag aggtacgggg gctttctaaa caaagaagag    720
   ttcgctgcag atttcgagaa ttatgcaagg attatgttta aagcaatccc taagtgcaag    780
   cactggatta cctttaacga accatggtgt agtgccattc tcggatataa tacaggctat    840
   ttcgcacctg gacacacttc tgatagatct aagagtccgg tgggtgactc agctcgtgaa    900
   ccgtggatag ttgggcacaa catactaatt gcccatgcta gggcagttaa ggcttatcgt    960
   gaagatttca aaccaacgca aggtggagaa ataggtataa cgctgaacgg tgatgctact   1020
   cttccgtggg atccagagga tcccgctgac attgaagcat gtgataggaa aatcgaattt   1080
   gccataagct ggttcgcaga tccgatctac ttcggcaaat atccggactc aatgagaaag   1140
   cagttagggg atcgactccc agaattcacg cctgaagagg tggctctagt gaaaggtagt   1200
   aacgatttct acgggatgaa tcactatacg gctaattaca tcaaacacaa gactggtgtt   1260
   cctcccgaag acgatttcct tggaaacctt gagacactct tttataacaa atatggtgat   1320
   tgcattggcc ctgagacaca gtcattctgg ttaagaccac atgctcaagg gtttcgagat   1380
   ttgctgaatt ggctttcaaa gaggtacggg taccctaaga tctacgtcac tgaaaacgga   1440
   accagtctga agggtgagaa tgacatgcca ctagaacaag tcctcgagga cgatttccga   1500
   gttaagtatt tcaatgatta cgttcgtgca atggccgctg cagtagctga agatggttgc   1560
   aacgtaagag gctacttggc ctggagtcta ctggataatt ttgaatgggc tgagggatac   1620
   gagacacggt tcggtgttac ttatgtcgac tatgccaacg atcagaagcg ctatccaaag   1680
   aaatctgcaa agtccttaaa gcccctgttt gatagcctta ttcgtaaaga gtaagcttga   1740
   agactaggcg tggtgcgcac gatagcgcat agtgtttttc tctccacttg aatcgaagag   1800
   atagacttac ggtgtaaatc cgtaggggtg gcgtaaacca aattacgcaa tgttttgggt   1860
   tccatttaaa tcgaaacccc ttatttcctg gatcacctgt taacgcacgt ttgacgtgta   1920
   ttacagtggg aataagtaaa agtgagaggt tcgaatcctc cctaaccccg ggtagggg     1978
<210> 6
   <211> 4807
   <212> DNA
   <213> Artificial
<220>
   <223> T-DNA of PhiC31 integrase expressing construct
<400> 6
   ctgatgggct gcctgtatcg agtggtgatt ttgtgccgag ctgccggtcg gggagctgtt      60
   ggctggctgg tggcaggata tattgtggtg taaacaaatt gacgcttaga caacttaata     120
   acacattgcg gacgttttta atgtactggg gtggatgcag gtcgatctag taacatagat     180
   gacaccgcgc gcgataattt atcctagttt gcgcgctata ttttgttttc tatcgcgtat     240
   taaatgtata attgcgggac tctaatcata aaaacccatc tcataaataa cgtcatgcat     300
   tacatgttaa ttattacatg cttaacgtaa ttcaacagaa attatatgat aatcatcgca     360
   agaccggcaa caggattcaa tcttaagaaa ctttattgcc aaatgtttga acgatctgct     420
   tgactctaga tccagagtcc cgctcagaag aactcgtcaa gaaggcgata gaaggcgatg     480
   cgctgcgaat cgggagcggc gataccgtaa agcacgagga agcggtcagc ccattcgccg     540
   ccaagctctt cagcaatatc acgggtagcc aacgctatgt cctgatagcg gtccgccaca     600
   cccagccggc cacagtcgat gaatccagaa aagcggccat tttccaccat gatattcggc     660
   aagcaggcat cgccatgagt cacgacgaga tcctcgccgt cgggcatacg cgccttgagc     720
   ctggcgaaca gttcggctgg cgcgagcccc tgatgctctt cgtccagatc atcctgatcg     780
   acaagaccgg cttccatccg agtacgtgct cgctcgatgc gatgtttcgc ttggtggtcg     840
   aatgggcagg tagccggatc aagcgtatgc agccgccgca ttgcatcagc catgatggat     900
   actttctcgg caggagcaag gtgagatgac aggagatcct gccccggcac ttcgcccaat     960
   agcagccagt cccttcccgc ttcagtgaca acgtcgagca cagctgcgca aggaacgccc    1020
   gtcgtggcca gccacgatag ccgcgctgcc tcgtcctgga gttcattcag ggcaccggac    1080
   aggtcggtct tgacaaaaag aaccgggcgc ccctgcgctg acagccggaa cacggcggca    1140
   tcagagcagc cgattgtctg ttgtgcccag tcatagccga atagcctctc cacccaagcg 1200
   gccggagaac ctgcgtgcaa tccatcttgt tcaatcatgc gaaacgatcc agatccggtg 1260
   cagattattt ggattgagag tgaatatgag actctaattg gataccgagg ggaatttatg 1320
   gaacgtcagt ggagcatttt tgacaagaaa tatttgctag ctgatagtga ccttaggcga 1380
   cttttgaacg cgcaataatg gtttctgacg tatgtgctta gctcattaaa ctccagaaac 1440
   ccgcggctga gtggctcctt caacgttgcg gttctgtcag ttccaaacgt aaaacggctt 1500
   gtcccgcgtc atcggcgggg gtcataacgt gactccctta attctccgct catgaattgg 1560
   taccgcttat gcagccgatg gtagattcga agacgcagct aaagctagaa aattgcttcg 1620
   agaccgaggg gaaaagaagg aaacagggct aagttgggtt gaagaaagga acaaggttca 1680
   tacatttgca gctggagaaa gacgtcatga gaaaagcaag gagatatatg agaagctggc 1740
   tgagttaggg gaagagatgg agaaagcggg ttacattgca gacacaagct atgttctgag 1800
   agaagtggat ggtgacgaga agaatcaaac cataaggtat catagtgaga gattagctat 1860
   tgcttttgga ctgatcacgt ttccagctga tagaccgatc cgagtaatga agaatctgcg 1920
   tgtttgcggt gattgccata acgcgatcaa gttcatgtcc gtatgtacga gacgagtgat 1980
   cattgtgaga gataataatc ggtttcatcg gtttgaagat ggcaagtgtt cttgtaatga 2040
   ctattggtga agaagacaaa tgagagttgg tttatattta accataattt cattcagttc 2100
   acactgaacc ggcgaaattt ctttgccaga cctattcgga attgaaacaa gtggagtctc 2160
   gaaacgaaaa gaactttctg gaattcgttt gctcacaaag ctaaaaacgg ttgatttcat 2220
   cgaaatacgg cgtcgttttc aaagaacaat ccagaaatca ctggttttcc tttatttcaa 2280
   aagaagagac tagaacttta tttctcctct ataaaatcac tttgtttttt cctctcttct 2340
   tcataaatca acaaaacaat cacaaatctc tcgaaacgct ctcgaagttc caaattttct 2400
   cttagcattc tctttcgttt ctcgtttgcg ttgaatcaaa gttcgttgcg atggctcgag 2460
   tctgcaggac aatggacacg tacgcgggtg cttacgaccg tcagtcgcgc gagcgcgaga 2520
   attcgagcgc agcaagccca gcgacacagc gtagcgccaa cgaagacaag gcggccgacc 2580
   ttcagcgcga agtcgagcgc gacgggggcc ggttcaggtt cgtcgggcat ttcagcgaag 2640
   cgccgggcac gtcggcgttc gggacggcgg agcgcccgga gttcgaacgc atcctgaacg 2700
   aatgccgcgc cgggcggctc aacatgatca ttgtctatga cgtgtcgcgc ttctcgcgcc 2760
   tgaaggtcat ggacgcgatt ccgattgtct cggaattgct cgccctgggc gtgacgattg 2820
   tttccactca ggaaggcgtc ttccggcagg gaaacgtcat ggacctgatt cacctgatta 2880
   tgcggctcga cgcgtcgcac aaagaatctt cgctgaagtc ggcgaagatt ctcgacacga 2940
   agaaccttca gcgcgaattg ggcgggtacg tcggcgggaa ggcgccttac ggcttcgagc 3000
   ttgtttcgga gacgaaggag atcacgcgca acggccgaat ggtcaatgtc gtcatcaaca 3060
   agcttgcgca ctcgaccact ccccttaccg gacccttcga gttcgagccc gacgtaatcc 3120
   ggtggtggtg gcgtgagatc aagacgcaca aacaccttcc cttcaagccg ggcagtcaag 3180
   ccgccattca cccgggcagc atcacggggc tttgtaagcg catggacgct gacgccgtgc 3240
   cgacccgggg cgagacgatt gggaagaaga ccgcttcaag cgcctgggac ccggcaaccg 3300
   ttatgcgaat ccttcgggac ccgcgtattg cgggcttcgc cgctgaggtg atctacaaga 3360
   agaagccgga cggcacgccg accacgaaga ttgagggtta ccgcattcag cgcgacccga 3420
   tcacgctccg gccggtcgag cttgattgcg gaccgatcat cgagcccgct gagtggtatg 3480
   agcttcaggc gtggttggac ggcagggggc gcggcaaggg gctttcccgg gggcaagcca 3540
   ttctgtccgc catggacaag ctgtactgcg agtgtggcgc cgtcatgact tcgaagcgcg 3600
   gggaagaatc gatcaaggac tcttaccgct gccgtcgccg gaaggtggtc gacccgtccg 3660
   cacctgggca gcacgaaggc acgtgcaacg tcagcatggc ggcactcgac aagttcgttg 3720
   cggaacgcat cttcaacaag atcaggcacg ccgaaggcga cgaagagacg ttggcgcttc 3780
   tgtgggaagc cgcccgacgc ttcggcaagc tcactgaggc gcctgagaag agcggcgaac 3840
   gggcgaacct tgttgcggag cgcgccgacg ccctgaacgc ccttgaagag ctgtacgaag 3900
   accgcgcggc aggcgcgtac gacggacccg ttggcaggaa gcacttccgg aagcaacagg 3960
   cagcgctgac gctccggcag caaggggcgg aagagcggct tgccgaactt gaagccgccg 4020
   aagccccgaa gcttcccctt gaccaatggt tccccgaaga cgccgacgct gacccgaccg 4080
   gccctaagtc gtggtggggg cgcgcgtcag tagacgacaa gcgcgtgttc gtcgggctct 4140
   tcgtagacaa gatcgttgtc acgaagtcga ctacgggcag ggggcaggga acgcccatcg 4200
   agaagcgcgc ttcgatcacg tgggcgaagc cgccgaccga cgacgacgaa gacgacgccc 4260
   aggacggcac ggaagacgta gcggcgtaga ccccctaaga agaagaggaa ggtttaggat 4320
   ccaccggaga tccctggccg gccccgatcg ttcaaacatt tggcaataaa gtttcttaag 4380
   attgaatcct gttgccggtc ttgcgatgat tatcatataa tttctgttga attacgttaa 4440
   gcatgtaata attaacatgt aatgcatgac gttatttatg agatgggttt ttatgattag 4500
   agtcccgcaa ttatacattt aatacgcgat agaaaacaaa atatagcgcg caaactagga 4560
   taaattatcg cgcgcggtgt catctatgtt actagatccg atgataagct gtcaaacatg 4620
   agaattgggc cccaagctta tgatcagatt gtcgtttccc gccttcagtt taaactatca 4680
   gtgtttgaca ggatatattg gcgggtaaac ctaagagaaa agagcgttta ttagaataat 4740
   cggatattta aaagggcgtg aaaaggttta tccgttcgtc catttgtatg tgcatgccaa 4800
   ccacagg 4807
<210> 7
   <211> 11053
   <212> DNA
   <213> Artificial
<220>
   <223> T-DNA of assembled vector pNMD1201
<400> 7
   cctgtggttg gcacatacaa atggacgaac ggataaacct tttcacgccc ttttaaatat     60
   ccgattattc taataaacgc tcttttctct taggtttacc cgccaatata tcctgtcaaa    120
   cactgatagt ttaaactgaa ggcgggaaac gacaatctga tctaagctag cttggaattg    180
   gtaccacgcg tttcgacaaa atttagaacg aacttaatta tgatctcaaa tacattgata    240
   catatctcat ctagatctag gttatcatta tgtaagaaag ttttgacgaa tatggcacga    300
   caaaatggct agactcgatg taattggtat ctcaactcaa cattatactt ataccaaaca    360
   ttagttagac aaaatttaaa caactatttt ttatgtatgc aagagtcagc atatgtataa    420
   ttgattcaga atcgttttga cgagttcgga tgtagtagta gccattattt aatgtacata    480
   ctaatcgtga atagtgaata tgatgaaaca ttgtatctta ttgtataaat atccataaac    540
   acatcatgaa agacactttc tttcacggtc tgaattaatt atgatacaat tctaatagaa    600
   aacgaattaa attacgttga attgtatgaa atctaattga acaagccaac cacgacgacg    660
   actaacgttg cctggattga ctcggtttaa gttaaccact aaaaaaacgg agctgtcatg    720
   taacacgcgg atcgagcagg tcacagtcat gaagccatca aagcaaaaga actaatccaa    780
   gggctgagat gattaattag tttaaaaatt agttaacacg agggaaaagg ctgtctgaca    840
   gccaggtcac gttatcttta cctgtggtcg aaatgattcg tgtctgtcga ttttaattat    900
   ttttttgaaa ggccgaaaat aaagttgtaa gagataaacc cgcctatata aattcatata    960
   ttttcctctc cgctttgaag ttttagtttt attgcaacaa caacaacaaa ttacaataac   1020
   aacaaacaaa atacaaacaa caacaacatg gcacaatttc aacaaacaat tgacatgcaa   1080
   actctccaag ccgctgcggg acgcaacagc ttggtgaatg atttggcatc tcgtcgcgtt   1140
   tacgataatg cagtcgagga gctgaatgct cgttccagac gtcccaaggt aataggaact   1200
   ttctggatct actttatttg ctggatctcg atcttgtttt ctcaatttcc ttgagatctg   1260
   gaattcgttt aatttggatc tgtgaacctc cactaaatct tttggtttta ctagaatcga   1320
   tctaagttga ccgatcagtt agctcgatta tagctaccag aatttggctt gaccttgatg   1380
   gagagatcca tgttcatgtt acctgggaaa tgatttgtat atgtgaattg aaatctgaac   1440
   tgttgaagtt agattgaatc tgaacactgt caatgttaga ttgaatctga acactgttta   1500
   aggttagatg aagtttgtgt atagattctt cgaaacttta ggatttgtag tgtcgtacgt   1560
   tgaacagaaa gctatttctg attcaatcag ggtttatttg actgtattga actctttttg   1620
   tgtgtttgca ggtccacttc tccaaggcag tgtctacgga acagaccctg attgcaacaa   1680
   acgcatatcc ggagttcgag atttccttta ctcatacgca atccgctgtg cactccttgg   1740
   ccggaggcct tcggtcactt gagttggagt atctcatgat gcaagttccg ttcggttctc   1800
   tgacgtacga catcggcggt aacttttccg cgcacctttt caaagggcgc gattacgttc   1860
   actgctgcat gcctaatctg gatgtacgtg acattgctcg ccatgaagga cacaaggaag   1920
   ctatttacag ttatgtgaat cgtttgaaaa ggcagcagcg tcctgtgcct gaataccaga   1980
   gggcagcttt caacaactac gctgagaacc cgcacttcgt ccattgcgac aaacctttcc   2040
   aacagtgtga attgacgaca gcgtatggca ctgacaccta cgctgtagct ctccatagca 2100
   tttatgatat ccctgttgag gagttcggtt ctgcgctact caggaagaat gtgaaaactt 2160
   gtttcgcggc ctttcatttc catgagaata tgcttctaga ttgtgataca gtcacactcg 2220
   atgagattgg agctacgttc cagaaatcag gtaacattcc ttagttacct ttcttttctt 2280
   tttccatcat aagtttatag attgtacatg ctttgagatt tttctttgca aacaatctca 2340
   ggtgataacc tgagcttctt cttccataat gagagcactc tcaattacac ccacagcttc 2400
   agcaacatca tcaagtacgt gtgcaagacg ttcttccctg ctagtcaacg cttcgtgtac 2460
   cacaaggagt tcctggtcac tagagtcaac acttggtact gcaagttcac gagagtggat 2520
   acgttcactc tgttccgtgg tgtgtaccac aacaatgtgg attgcgaaga gttttacaag 2580
   gctatggacg atgcgtggca ctacaaaaag acgttagcaa tgcttaatgc cgagaggacc 2640
   atcttcaagg ataacgctgc gttaaacttc tggttcccga aggtgctctt gaaattggaa 2700
   gtcttctttt gttgtctaaa cctatcaatt tctttgcgga aatttatttg aagctgtaga 2760
   gttaaaattg agtcttttaa acttttgtag gtgagagaca tggttatcgt ccctctcttt 2820
   gacgcttcta tcacaactgg taggatgtct aggagagagg ttatggtgaa caaggacttc 2880
   gtctacacgg tcctaaatca catcaagacc tatcaagcta aggcactgac gtacgcaaac 2940
   gtgctgagct tcgtggagtc tattaggtct agagtgataa ttaacggtgt cactgccagg 3000
   taagttgtta cttatgattg ttttcctctc tgctacatgt attttgttgt tcatttctgt 3060
   aagatataag aattgagttt tcctctgatg atattattag gtctgaatgg gacacagaca 3120
   aggcaattct aggtccatta gcaatgacat tcttcctgat cacgaagctg ggtcatgtgc 3180
   aagatgaaat aatcctgaaa aagttccaga agttcgacag aaccaccaat gagctgattt 3240
   ggacaagtct ctgcgatgcc ctgatggggg ttattccctc ggtcaaggag acgcttgtgc 3300
   gcggtggttt tgtgaaagta gcagaacaag ccttagagat caaggttagt atcatatgaa 3360
   gaaataccta gtttcagttg atgaatgcta ttttctgacc tcagttgttc tcttttgaga 3420
   attatttctt ttctaatttg cctgattttt ctattaattc attaggttcc cgagctatac 3480
   tgtaccttcg ccgaccgatt ggtactacag tacaagaagg cggaggagtt ccaatcgtgt 3540
   gatctttcca aacctctaga agagtcagag aagtactaca acgcattatc cgagctatca 3600
   gtgcttgaga atctcgactc ttttgactta gaggcgttta agactttatg tcagcagaag 3660
   aatgtggacc cggatatggc agcaaaggta aatcctggtc cacactttta cgataaaaac 3720
   acaagatttt aaactatgaa ctgatcaata atcattccta aaagaccaca cttttgtttt 3780
   gtttctaaag taatttttac tgttataaca ggtggtcgta gcaatcatga agtcagaatt 3840
   gacgttgcct ttcaagaaac ctacagaaga ggaaatctcg gagtcgctaa aaccaggaga 3900
   ggggtcgtgt gcagagcata aggaagtgtt gagcttacaa aatgatgctc cgttcccgtg 3960
   tgtgaaaaat ctagttgaag gttccgtgcc ggcgtatgga atgtgtccta agggtggtgg 4020
   tttcgacaaa ttggatgtgg acattgctga tttccatctc aagagtgtag atgcagttaa   4080
   aaagggaact atgatgtctg cggtgtacac agggtctatc aaagttcaac aaatgaagaa   4140
   ctacatagat tacttaagtg cgtcgctggc agctacagtc tcaaacctct gcaaggtaag   4200
   aggtcaaaag gtttccgcaa tgatccctct ttttttgttt ctctagtttc aagaatttgg   4260
   gtatatgact aacttctgag tgttccttga tgcatatttg tgatgagaca aatgtttgtt   4320
   ctatgtttta ggtgcttaga gatgttcacg gcgttgaccc agagtcacag gagaaatctg   4380
   gagtgtggga tgttaggaga ggacgttggt tacttaaacc taatgcgaaa agtcacgcgt   4440
   ggggtgtggc agaagacgcc aaccacaagt tggttattgt gttactcaac tgggatgacg   4500
   gaaagccggt ttgtgatgag acatggttca gggtggcggt gtcaagcgat tccttgatat   4560
   attcggatat gggaaaactt aagacgctca cgtcttgcag tccaaatggt gagccaccgg   4620
   agcctaacgc caaagtaatt ttggtcgatg gtgttcccgg ttgtggaaaa acgaaggaga   4680
   ttatcgaaaa ggtaagttct gcatttggtt atgctccttg cattttaggt gttcgtcgct   4740
   cttccatttc catgaatagc taagattttt tttctctgca ttcattcttc ttgcctcagt   4800
   tctaactgtt tgtggtattt ttgttttaat tattgctaca ggtaaacttc tctgaagact   4860
   tgattttagt ccctgggaag gaagcttcta agatgatcat ccggagggcc aaccaagctg   4920
   gtgtgataag agcggataag gacaatgtta gaacggtgga ttccttcttg atgcatcctt   4980
   ctagaagggt gtttaagagg ttgtttatcg atgaaggact aatgctgcat acaggttgtg   5040
   taaatttcct actgctgcta tctcaatgtg acgtcgcata tgtgtatggg gacacaaagc   5100
   aaattccgtt catttgcaga gtcgcgaact ttccgtatcc agcgcatttt gcaaaactcg   5160
   tcgctgatga gaaggaagtc agaagagtta cgctcaggta aagcaactgt gttttaatca   5220
   atttcttgtc aggatatatg gattataact taatttttga gaaatctgta gtatttggcg   5280
   tgaaatgagt ttgctttttg gtttctcccg tgttataggt gcccggctga tgttacgtat   5340
   ttccttaaca agaagtatga cggggcggtg atgtgtacca gcgcggtaga gagatccgtg   5400
   aaggcagaag tggtgagagg aaagggtgca ttgaacccaa taaccttacc gttggagggt   5460
   aaaattttga ccttcacaca agctgacaag ttcgagttac tggagaaggg ttacaaggta   5520
   aagtttccaa ctttccttta ccatatcaaa ctaaagttcg aaacttttta tttgatcaac   5580
   ttcaaggcca cccgatcttt ctattcctga ttaatttgtg atgaatccat attgactttt   5640
   gatggttacg caggatgtga acactgtgca cgaggtgcaa ggggagacgt acgagaagac   5700
   tgctattgtg cgcttgacat caactccgtt agagatcata tcgagtgcgt cacctcatgt   5760
   tttggtggcg ctgacaagac acacaacgtg ttgtaaatat tacaccgttg tgttggaccc   5820
   gatggtgaat gtgatttcag aaatggagaa gttgtccaat ttccttcttg acatgtatag   5880
   agttgaagca ggtctgtctt tcctatttca tatgtttaat cctaggaatt tgatcaattg   5940
   attgtatgta tgtcgatccc aagactttct tgttcactta tatcttaact ctctctttgc   6000
   tgtttcttgc aggtgtccaa tagcaattac aaatcgatgc agtattcagg ggacagaact   6060
   tgtttgttca gacgcccaag tcaggagatt ggcgagatat gcaattttac tatgacgctc 6120
   ttcttcccgg aaacagtact attctcaatg aatttgatgc tgttacgatg aatttgaggg 6180
   atatttcctt aaacgtcaaa gattgcagaa tcgacttctc caaatccgtg caacttccta 6240
   aagaacaacc tattttcctc aagcctaaaa taagaactgc ggcagaaatg ccgagaactg 6300
   caggtaaaat attggatgcc agacgatatt ctttcttttg atttgtaact ttttcctgtc 6360
   aaggtcgata aattttattt tttttggtaa aaggtcgata attttttttt ggagccatta 6420
   tgtaattttc ctaattaact gaaccaaaat tatacaaacc aggtttgctg gaaaatttgg 6480
   ttgcaatgat caaaagaaac atgaatgcgc cggatttgac agggacaatt gacattgagg 6540
   atactgcatc tctggtggtt gaaaagtttt gggattcgta tgttgacaag gaatttagtg 6600
   gaacgaacga aatgaccatg acaagggaga gcttctccag gtaaggactt ctcatgaata 6660
   ttagtggcag attagtgttg ttaaagtctt tggttagata atcgatgcct cctaattgtc 6720
   catgttttac tggttttcta caattaaagg tggctttcga aacaagagtc atctacagtt 6780
   ggtcagttag cggactttaa ctttgtggat ttgccggcag tagatgagta caagcatatg 6840
   atcaagagtc aaccaaagca aaagttagac ttgagtattc aagacgaata tcctgcattg 6900
   cagacgatag tctaccattc gaaaaagatc aatgcgattt tcggtccaat gttttcagaa 6960
   cttacgagga tgttactcga aaggattgac tcttcgaagt ttctgttcta caccagaaag 7020
   acacctgcac aaatagagga cttcttttct gacctagact caacccaggc gatggaaatt 7080
   ctggaactcg acatttcgaa gtacgataag tcacaaaacg agttccattg tgctgtagag 7140
   tacaagatct gggaaaagtt aggaattgat gagtggctag ctgaggtctg gaaacaaggt 7200
   gagttcctaa gttccatttt tttgtaatcc ttcaatgtta ttttaacttt tcagatcaac 7260
   atcaaaatta ggttcaattt tcatcaacca aataatattt ttcatgtata tataggtcac 7320
   agaaaaacga ccttgaaaga ttatacggcc ggaatcaaaa catgtctttg gtatcaaagg 7380
   aaaagtggtg atgtgacaac ctttattggt aataccatca tcattgccgc atgtttgagc 7440
   tcaatgatcc ccatggacaa agtgataaag gcagcttttt gtggagacga tagcctgatt 7500
   tacattccta aaggtttaga cttgcctgat attcaggcgg gcgcgaacct catgtggaac 7560
   ttcgaggcca aactcttcag gaagaagtat ggttacttct gtggtcgtta tgttattcac 7620
   catgatagag gagccattgt gtattacgat ccgcttaaac taatatctaa gttaggttgt 7680
   aaacatatta gagatgttgt tcacttagaa gagttacgcg agtctttgtg tgatgtagct 7740
   agtaacttaa ataattgtgc gtatttttca cagttagatg aggccgttgc cgaggttcat 7800
   aagaccgcgg taggcggttc gtttgctttt tgtagtataa ttaagtattt gtcagataag 7860
   agattgttta gagatttgtt ctttgtttga taatgtcgat agtctcgtac gaacctaagg 7920
   tgagtgattt cctcaatctt tcgaagaagg aagagatctt gccgaaggct ctaacgaggt 7980
   taaaaaccgt gtctattagt actaaagata ttatatctgt caaggagtcg gagactttgt 8040
   gtgatataga tttgttaatc aatgtgccat tagataagta tagatatgtg ggtatcctag 8100
   gagccgtttt taccggagag tggctagtgc cagacttcgt taaaggtgga gtgacgataa 8160
   gtgtgataga taagcgtctg gtgaactcaa aggagtgcgt gattggtacg tacagagccg 8220
   cagccaagag taagaggttc cagttcaaat tggttccaaa ttactttgtg tccaccgtgg 8280
   acgcaaagag gaagccgtgg caggtaagga tttttatgat atagtatgct tatgtatttt 8340
   gtactgaaag catatcctgc ttcattggga tattactgaa agcatttaac tacatgtaaa 8400
   ctcacttgat gatcaataaa cttgattttg caggttcatg ttcgtataca agacttgaag 8460
   attgaggcgg gttggcagcc gttagctctg gaagtagttt cagttgctat ggtcaccaat 8520
   aacgttgtca tgaagggttt gagggaaaag gtcgtcgcaa taaatgatcc ggacgtcgaa 8580
   ggtttcgaag gtaagccatc ttcctgctta tttttataat gaacatagaa ataggaagtt 8640
   gtgcagagaa actaattaac ctgactcaaa atctaccctc ataattgttg tttgatattg 8700
   gtcttgtatt ttgcaggtgt ggttgacgaa ttcgtcgatt cggttgcagc atttaaagcg 8760
   gttgacaact ttaaaagaag gaaaaagaag gttgaagaaa agggtgtagt aagtaagtat 8820
   aagtacagac cggagaagta cgccggtcct gattcgttta atttgaaaga agaaaacgtc 8880
   ttacaacatt acaaacccga atcagtacca gtatttcgat aagaaacaag aaaccatgag 8940
   cctaccacct gactttaagt ggggttttgc taccgccgct tatcagatag aaggatctgt 9000
   gaatgaggat ggtagaggac cttctatttg ggacaccttt tgtgcaatcc ctggtaaaat 9060
   agctgacgga agctcaggag ctgttgcctg tgattcttac aagcggacaa aggaagatat 9120
   tgccttgtta aaagagcttg gtgctaactc ctatagattt tccatttctt ggtcccgaat 9180
   tatccctctg ggcggaagaa atgaccctat caaccaaaaa ggcatcgatc attacgtgaa 9240
   gttcgtagac gatctaatcg aggccggaat tactccattc attactctct tccactggga 9300
   tcttcccgat gcattggata agaggtacgg gggctttcta aacaaagaag agttcgctgc 9360
   agatttcgag aattatgcaa ggattatgtt taaagcaatc cctaagtgca agcactggat 9420
   tacctttaac gaaccatggt gtagtgccat tctcggatat aatacaggct atttcgcacc 9480
   tggacacact tctgatagat ctaagagtcc ggtgggtgac tcagctcgtg aaccgtggat 9540
   agttgggcac aacatactaa ttgcccatgc tagggcagtt aaggcttatc gtgaagattt 9600
   caaaccaacg caaggtggag aaataggtat aacgctgaac ggtgatgcta ctcttccgtg 9660
   ggatccagag gatcccgctg acattgaagc atgtgatagg aaaatcgaat ttgccataag 9720
   ctggttcgca gatccgatct acttcggcaa atatccggac tcaatgagaa agcagttagg 9780
   ggatcgactc ccagaattca cgcctgaaga ggtggctcta gtgaaaggta gtaacgattt 9840
   ctacgggatg aatcactata cggctaatta catcaaacac aagactggtg ttcctcccga 9900
   agacgatttc cttggaaacc ttgagacact cttttataac aaatatggtg attgcattgg 9960
   ccctgagaca cagtcattct ggttaagacc acatgctcaa gggtttcgag atttgctgaa 10020
   ttggctttca aagaggtacg ggtaccctaa gatctacgtc actgaaaacg gaaccagtct 10080
   gaagggtgag aatgacatgc cactagaaca agtcctcgag gacgatttcc gagttaagta 10140
   tttcaatgat tacgttcgtg caatggccgc tgcagtagct gaagatggtt gcaacgtaag 10200
   aggctacttg gcctggagtc tactggataa ttttgaatgg gctgagggat acgagacacg 10260
   gttcggtgtt acttatgtcg actatgccaa cgatcagaag cgctatccaa agaaatctgc 10320
   aaagtcctta aagcccctgt ttgatagcct tattcgtaaa gagtaagctt actagagcgt 10380
   ggtgcgcacg atagcgcata gtgtttttct ctccacttga atcgaagaga tagacttacg 10440
   gtgtaaatcc gtaggggtgg cgtaaaccaa attacgcaat gttttgggtt ccatttaaat 10500
   cgaaacccct tatttcctgg atcacctgtt aacgcacgtt tgacgtgtat tacagtggga 10560
   ataagtaaaa gtgagaggtt cgaatcctcc ctaaccccgg gtaggggccc agcggccgct 10620
   ctagctagag tcaagcagat cgttcaaaca tttggcaata aagtttctta agattgaatc 10680
   ctgttgccgg tcttgcgatg attatcatat aatttctgtt gaattacgtt aagcatgtaa 10740
   taattaacat gtaatgcatg acgttattta tgagatgggt ttttatgatt agagtcccgc 10800
   aattatacat ttaatacgcg atagaaaaca aaatatagcg cgcaaactag gataaattat 10860
   cgcgcgcggt gtcatctatg ttactagatc gacctgcatc caccccagta cattaaaaac 10920
   gtccgcaatg tgttattaag ttgtctaagc gtcaatttgt ttacaccaca atatatcctg 10980
   ccaccagcca gccaacagct ccccgaccgg cagctcggca caaaatcacc actcgataca 11040
   ggcagcccat cag                                                    11053
<210> 8
   <211> 11377
   <212> DNA
   <213> Artificial
<220>
   <223> T-DNA of assembled viral vector pNMD1161
<400> 8
   cctgtggttg gcacatacaa atggacgaac ggataaacct tttcacgccc ttttaaatat      60
   ccgattattc taataaacgc tcttttctct taggtttacc cgccaatata tcctgtcaaa     120
   cactgatagt ttaaactgaa ggcgggaaac gacaatctga tctaagctag cttggaattg     180
   gtaccacgcg tttcgacaaa atttagaacg aacttaatta tgatctcaaa tacattgata     240
   catatctcat ctagatctag gttatcatta tgtaagaaag ttttgacgaa tatggcacga     300
   caaaatggct agactcgatg taattggtat ctcaactcaa cattatactt ataccaaaca     360
   ttagttagac aaaatttaaa caactatttt ttatgtatgc aagagtcagc atatgtataa     420
   ttgattcaga atcgttttga cgagttcgga tgtagtagta gccattattt aatgtacata     480
   ctaatcgtga atagtgaata tgatgaaaca ttgtatctta ttgtataaat atccataaac     540
   acatcatgaa agacactttc tttcacggtc tgaattaatt atgatacaat tctaatagaa     600
   aacgaattaa attacgttga attgtatgaa atctaattga acaagccaac cacgacgacg     660
   actaacgttg cctggattga ctcggtttaa gttaaccact aaaaaaacgg agctgtcatg 720
   taacacgcgg atcgagcagg tcacagtcat gaagccatca aagcaaaaga actaatccaa 780
   gggctgagat gattaattag tttaaaaatt agttaacacg agggaaaagg ctgtctgaca 840
   gccaggtcac gttatcttta cctgtggtcg aaatgattcg tgtctgtcga ttttaattat 900
   ttttttgaaa ggccgaaaat aaagttgtaa gagataaacc cgcctatata aattcatata 960
   ttttcctctc cgctttgaag ttttagtttt attgcaacaa caacaacaaa ttacaataac 1020
   aacaaacaaa atacaaacaa caacaacatg gcacaatttc aacaaacaat tgacatgcaa 1080
   actctccaag ccgctgcggg acgcaacagc ttggtgaatg atttggcatc tcgtcgcgtt 1140
   tacgataatg cagtcgagga gctgaatgct cgttccagac gtcccaaggt aataggaact 1200
   ttctggatct actttatttg ctggatctcg atcttgtttt ctcaatttcc ttgagatctg 1260
   gaattcgttt aatttggatc tgtgaacctc cactaaatct tttggtttta ctagaatcga 1320
   tctaagttga ccgatcagtt agctcgatta tagctaccag aatttggctt gaccttgatg 1380
   gagagatcca tgttcatgtt acctgggaaa tgatttgtat atgtgaattg aaatctgaac 1440
   tgttgaagtt agattgaatc tgaacactgt caatgttaga ttgaatctga acactgttta 1500
   aggttagatg aagtttgtgt atagattctt cgaaacttta ggatttgtag tgtcgtacgt 1560
   tgaacagaaa gctatttctg attcaatcag ggtttatttg actgtattga actctttttg 1620
   tgtgtttgca ggtccacttc tccaaggcag tgtctacgga acagaccctg attgcaacaa 1680
   acgcatatcc ggagttcgag atttccttta ctcatacgca atccgctgtg cactccttgg 1740
   ccggaggcct tcggtcactt gagttggagt atctcatgat gcaagttccg ttcggttctc 1800
   tgacgtacga catcggcggt aacttttccg cgcacctttt caaagggcgc gattacgttc 1860
   actgctgcat gcctaatctg gatgtacgtg acattgctcg ccatgaagga cacaaggaag 1920
   ctatttacag ttatgtgaat cgtttgaaaa ggcagcagcg tcctgtgcct gaataccaga 1980
   gggcagcttt caacaactac gctgagaacc cgcacttcgt ccattgcgac aaacctttcc 2040
   aacagtgtga attgacgaca gcgtatggca ctgacaccta cgctgtagct ctccatagca 2100
   tttatgatat ccctgttgag gagttcggtt ctgcgctact caggaagaat gtgaaaactt 2160
   gtttcgcggc ctttcatttc catgagaata tgcttctaga ttgtgataca gtcacactcg 2220
   atgagattgg agctacgttc cagaaatcag gtaacattcc ttagttacct ttcttttctt 2280
   tttccatcat aagtttatag attgtacatg ctttgagatt tttctttgca aacaatctca 2340
   ggtgataacc tgagcttctt cttccataat gagagcactc tcaattacac ccacagcttc 2400
   agcaacatca tcaagtacgt gtgcaagacg ttcttccctg ctagtcaacg cttcgtgtac 2460
   cacaaggagt tcctggtcac tagagtcaac acttggtact gcaagttcac gagagtggat 2520
   acgttcactc tgttccgtgg tgtgtaccac aacaatgtgg attgcgaaga gttttacaag 2580
   gctatggacg atgcgtggca ctacaaaaag acgttagcaa tgcttaatgc cgagaggacc 2640
   atcttcaagg ataacgctgc gttaaacttc tggttcccga aggtgctctt gaaattggaa 2700
   gtcttctttt gttgtctaaa cctatcaatt tctttgcgga aatttatttg aagctgtaga 2760
   gttaaaattg agtcttttaa acttttgtag gtgagagaca tggttatcgt ccctctcttt 2820
   gacgcttcta tcacaactgg taggatgtct aggagagagg ttatggtgaa caaggacttc 2880
   gtctacacgg tcctaaatca catcaagacc tatcaagcta aggcactgac gtacgcaaac 2940
   gtgctgagct tcgtggagtc tattaggtct agagtgataa ttaacggtgt cactgccagg 3000
   taagttgtta cttatgattg ttttcctctc tgctacatgt attttgttgt tcatttctgt 3060
   aagatataag aattgagttt tcctctgatg atattattag gtctgaatgg gacacagaca 3120
   aggcaattct aggtccatta gcaatgacat tcttcctgat cacgaagctg ggtcatgtgc 3180
   aagatgaaat aatcctgaaa aagttccaga agttcgacag aaccaccaat gagctgattt 3240
   ggacaagtct ctgcgatgcc ctgatggggg ttattccctc ggtcaaggag acgcttgtgc 3300
   gcggtggttt tgtgaaagta gcagaacaag ccttagagat caaggttagt atcatatgaa 3360
   gaaataccta gtttcagttg atgaatgcta ttttctgacc tcagttgttc tcttttgaga 3420
   attatttctt ttctaatttg cctgattttt ctattaattc attaggttcc cgagctatac 3480
   tgtaccttcg ccgaccgatt ggtactacag tacaagaagg cggaggagtt ccaatcgtgt 3540
   gatctttcca aacctctaga agagtcagag aagtactaca acgcattatc cgagctatca 3600
   gtgcttgaga atctcgactc ttttgactta gaggcgttta agactttatg tcagcagaag 3660
   aatgtggacc cggatatggc agcaaaggta aatcctggtc cacactttta cgataaaaac 3720
   acaagatttt aaactatgaa ctgatcaata atcattccta aaagaccaca cttttgtttt 3780
   gtttctaaag taatttttac tgttataaca ggtggtcgta gcaatcatga agtcagaatt 3840
   gacgttgcct ttcaagaaac ctacagaaga ggaaatctcg gagtcgctaa aaccaggaga 3900
   ggggtcgtgt gcagagcata aggaagtgtt gagcttacaa aatgatgctc cgttcccgtg 3960
   tgtgaaaaat ctagttgaag gttccgtgcc ggcgtatgga atgtgtccta agggtggtgg 4020
   tttcgacaaa ttggatgtgg acattgctga tttccatctc aagagtgtag atgcagttaa 4080
   aaagggaact atgatgtctg cggtgtacac agggtctatc aaagttcaac aaatgaagaa 4140
   ctacatagat tacttaagtg cgtcgctggc agctacagtc tcaaacctct gcaaggtaag 4200
   aggtcaaaag gtttccgcaa tgatccctct ttttttgttt ctctagtttc aagaatttgg 4260
   gtatatgact aacttctgag tgttccttga tgcatatttg tgatgagaca aatgtttgtt 4320
   ctatgtttta ggtgcttaga gatgttcacg gcgttgaccc agagtcacag gagaaatctg 4380
   gagtgtggga tgttaggaga ggacgttggt tacttaaacc taatgcgaaa agtcacgcgt 4440
   ggggtgtggc agaagacgcc aaccacaagt tggttattgt gttactcaac tgggatgacg 4500
   gaaagccggt ttgtgatgag acatggttca gggtggcggt gtcaagcgat tccttgatat 4560
   attcggatat gggaaaactt aagacgctca cgtcttgcag tccaaatggt gagccaccgg 4620
   agcctaacgc caaagtaatt ttggtcgatg gtgttcccgg ttgtggaaaa acgaaggaga 4680
   ttatcgaaaa ggtaagttct gcatttggtt atgctccttg cattttaggt gttcgtcgct 4740
   cttccatttc catgaatagc taagattttt tttctctgca ttcattcttc ttgcctcagt 4800
   tctaactgtt tgtggtattt ttgttttaat tattgctaca ggtaaacttc tctgaagact 4860
   tgattttagt ccctgggaag gaagcttcta agatgatcat ccggagggcc aaccaagctg 4920
   gtgtgataag agcggataag gacaatgtta gaacggtgga ttccttcttg atgcatcctt 4980
   ctagaagggt gtttaagagg ttgtttatcg atgaaggact aatgctgcat acaggttgtg 5040
   taaatttcct actgctgcta tctcaatgtg acgtcgcata tgtgtatggg gacacaaagc 5100
   aaattccgtt catttgcaga gtcgcgaact ttccgtatcc agcgcatttt gcaaaactcg 5160
   tcgctgatga gaaggaagtc agaagagtta cgctcaggta aagcaactgt gttttaatca 5220
   atttcttgtc aggatatatg gattataact taatttttga gaaatctgta gtatttggcg 5280
   tgaaatgagt ttgctttttg gtttctcccg tgttataggt gcccggctga tgttacgtat 5340
   ttccttaaca agaagtatga cggggcggtg atgtgtacca gcgcggtaga gagatccgtg 5400
   aaggcagaag tggtgagagg aaagggtgca ttgaacccaa taaccttacc gttggagggt 5460
   aaaattttga ccttcacaca agctgacaag ttcgagttac tggagaaggg ttacaaggta 5520
   aagtttccaa ctttccttta ccatatcaaa ctaaagttcg aaacttttta tttgatcaac 5580
   ttcaaggcca cccgatcttt ctattcctga ttaatttgtg atgaatccat attgactttt 5640
   gatggttacg caggatgtga acactgtgca cgaggtgcaa ggggagacgt acgagaagac 5700
   tgctattgtg cgcttgacat caactccgtt agagatcata tcgagtgcgt cacctcatgt 5760
   tttggtggcg ctgacaagac acacaacgtg ttgtaaatat tacaccgttg tgttggaccc 5820
   gatggtgaat gtgatttcag aaatggagaa gttgtccaat ttccttcttg acatgtatag 5880
   agttgaagca ggtctgtctt tcctatttca tatgtttaat cctaggaatt tgatcaattg 5940
   attgtatgta tgtcgatccc aagactttct tgttcactta tatcttaact ctctctttgc 6000
   tgtttcttgc aggtgtccaa tagcaattac aaatcgatgc agtattcagg ggacagaact 6060
   tgtttgttca gacgcccaag tcaggagatt ggcgagatat gcaattttac tatgacgctc 6120
   ttcttcccgg aaacagtact attctcaatg aatttgatgc tgttacgatg aatttgaggg 6180
   atatttcctt aaacgtcaaa gattgcagaa tcgacttctc caaatccgtg caacttccta 6240
   aagaacaacc tattttcctc aagcctaaaa taagaactgc ggcagaaatg ccgagaactg 6300
   caggtaaaat attggatgcc agacgatatt ctttcttttg atttgtaact ttttcctgtc 6360
   aaggtcgata aattttattt tttttggtaa aaggtcgata attttttttt ggagccatta 6420
   tgtaattttc ctaattaact gaaccaaaat tatacaaacc aggtttgctg gaaaatttgg 6480
   ttgcaatgat caaaagaaac atgaatgcgc cggatttgac agggacaatt gacattgagg 6540
   atactgcatc tctggtggtt gaaaagtttt gggattcgta tgttgacaag gaatttagtg 6600
   gaacgaacga aatgaccatg acaagggaga gcttctccag gtaaggactt ctcatgaata 6660
   ttagtggcag attagtgttg ttaaagtctt tggttagata atcgatgcct cctaattgtc 6720
   catgttttac tggttttcta caattaaagg tggctttcga aacaagagtc atctacagtt 6780
   ggtcagttag cggactttaa ctttgtggat ttgccggcag tagatgagta caagcatatg 6840
   atcaagagtc aaccaaagca aaagttagac ttgagtattc aagacgaata tcctgcattg 6900
   cagacgatag tctaccattc gaaaaagatc aatgcgattt tcggtccaat gttttcagaa 6960
   cttacgagga tgttactcga aaggattgac tcttcgaagt ttctgttcta caccagaaag 7020
   acacctgcac aaatagagga cttcttttct gacctagact caacccaggc gatggaaatt 7080
   ctggaactcg acatttcgaa gtacgataag tcacaaaacg agttccattg tgctgtagag 7140
   tacaagatct gggaaaagtt aggaattgat gagtggctag ctgaggtctg gaaacaaggt 7200
   gagttcctaa gttccatttt tttgtaatcc ttcaatgtta ttttaacttt tcagatcaac 7260
   atcaaaatta ggttcaattt tcatcaacca aataatattt ttcatgtata tataggtcac 7320
   agaaaaacga ccttgaaaga ttatacggcc ggaatcaaaa catgtctttg gtatcaaagg 7380
   aaaagtggtg atgtgacaac ctttattggt aataccatca tcattgccgc atgtttgagc 7440
   tcaatgatcc ccatggacaa agtgataaag gcagcttttt gtggagacga tagcctgatt 7500
   tacattccta aaggtttaga cttgcctgat attcaggcgg gcgcgaacct catgtggaac 7560
   ttcgaggcca aactcttcag gaagaagtat ggttacttct gtggtcgtta tgttattcac 7620
   catgatagag gagccattgt gtattacgat ccgcttaaac taatatctaa gttaggttgt 7680
   aaacatatta gagatgttgt tcacttagaa gagttacgcg agtctttgtg tgatgtagct 7740
   agtaacttaa ataattgtgc gtatttttca cagttagatg aggccgttgc cgaggttcat 7800
   aagaccgcgg taggcggttc gtttgctttt tgtagtataa ttaagtattt gtcagataag 7860
   agattgttta gagatttgtt ctttgtttga taatgtcgat agtctcgtac gaacctaagg 7920
   tgagtgattt cctcaatctt tcgaagaagg aagagatctt gccgaaggct ctaacgaggt 7980
   taaaaaccgt gtctattagt actaaagata ttatatctgt caaggagtcg gagactttgt 8040
   gtgatataga tttgttaatc aatgtgccat tagataagta tagatatgtg ggtatcctag 8100
   gagccgtttt taccggagag tggctagtgc cagacttcgt taaaggtgga gtgacgataa 8160
   gtgtgataga taagcgtctg gtgaactcaa aggagtgcgt gattggtacg tacagagccg 8220
   cagccaagag taagaggttc cagttcaaat tggttccaaa ttactttgtg tccaccgtgg 8280
   acgcaaagag gaagccgtgg caggtaagga tttttatgat atagtatgct tatgtatttt 8340
   gtactgaaag catatcctgc ttcattggga tattactgaa agcatttaac tacatgtaaa 8400
   ctcacttgat gatcaataaa cttgattttg caggttcatg ttcgtataca agacttgaag 8460
   attgaggcgg gttggcagcc gttagctctg gaagtagttt cagttgctat ggtcaccaat 8520
   aacgttgtca tgaagggttt gagggaaaag gtcgtcgcaa taaatgatcc ggacgtcgaa 8580
   ggtttcgaag gtaagccatc ttcctgctta tttttataat gaacatagaa ataggaagtt 8640
   gtgcagagaa actaattaac ctgactcaaa atctaccctc ataattgttg tttgatattg 8700
   gtcttgtatt ttgcaggtgt ggttgacgaa ttcgtcgatt cggttgcagc atttaaagcg   8760
   gttgacaact ttaaaagaag gaaaaagaag gttgaagaaa agggtgtagt aagtaagtat   8820
   aagtacagac cggagaagta cgccggtcct gattcgttta atttgaaaga agaaaacgtc   8880
   ttacaacatt acaaacccga atcagtacca gtatttcgat aagaaacaag aaaccatggc   8940
   actcaagaca cagttgttgt ggtcattcgt ggttgtgttc gttgtgtcct tcagtacaac   9000
   ttcatgctca ggtgcaggct gttctgttga ttacaccgtg aattcttggg gaactggatt   9060
   cacggctaac gttaccatca cgaatcttgg ttctgccata aacggatgga cattagagtg   9120
   ggacttccct ggcaaccagc aagttaccaa cctgtggaat ggcacgtata cccagtctgg   9180
   gcaacacgtt tctgtttcta atgctcctta caacgcatct atccctgcaa atggcactgt   9240
   cgaattcggc tttaatggga gctattctgg aagcaacgac atacctagct cttttaagct   9300
   gaacggtgta acatgtgacg ggagcgacga tccagatcct gaaccttctc catcaccttc   9360
   tccatctccc agccctactg atcctgatga acctggtgga ccaaccaatc ctccaacaaa   9420
   ccctggcgag aaagtggaca atcccttcga gggtgccaaa ctttacgtaa accctgtatg   9480
   gagtgctaaa gctgcagctg aacctggtgg atctgctgta gccaatgaat ctactgctgt   9540
   ttggctggat agaatcggtg ccatagaggg aaacgattct cctacaactg ggtctatggg   9600
   tcttagagat catctcgagg aagccgtaag acaaagtggt ggcgatcccc ttaccatcca   9660
   agttgtgatc tacaatctac ctggtcgcga ctgtgctgca cttgcttcta atggagaatt   9720
   gggacctgat gaactggata gatacaagtc cgagtacatt gatcccattg ctgacatcat   9780
   gtgggacttc gctgactatg agaacctacg aatcgtcgcc atcattgaga tagactccct   9840
   ccctaatcta gtcactaatg tcggaggcaa cggagggact gagctatgtg catacatgaa   9900
   gcaaaacgga ggctacgtca atggagttgg ctatgccctt agaaagttgg gtgagattcc   9960
   taacgtctac aactacatcg atgccgctca ccatggctgg ataggctggg attctaactt  10020
   tggaccctcc gtggacatct tctacgaagc tgcaaatgcc tctggtagca ccgttgacta  10080
   tgtgcatgga ttcatctcta acacggcaaa ctatagcgct actgttgaac cgtatctaga  10140
   cgtaaacggc accgtgaatg ggcaactgat aaggcaatct aagtgggtcg actggaacca  10200
   atacgttgat gagttgagtt tcgtccaaga cttgaggcaa gctctcatag ccaaagggtt  10260
   tcgttctgat atcggcatgc tcatagacac ttctagaaac ggatggggag gccctaatcg  10320
   tccaactgga cctagctcat ctacggacct taacacatac gttgacgagt cacgaattga  10380
   tagacgcata caccctggaa actggtgcaa ccaagctggt gctggcttgg gtgaaagacc  10440
   taccgtgaac ccagctcctg gtgttgacgc atacgtttgg gttaagcctc cgggagaatc  10500
   tgatggagct tccgaggaaa tccctaatga tgaggggaag ggcttcgata gaatgtgcga  10560
   tcctacttac caaggcaacg ctagaaacgg caacaatcca tctggtgctc tacctaatgc  10620
   tcccatatct gggcactggt tttctgccca atttcgggag cttctagcta atgcttaccc  10680
   tcccctctaa gcttactaga gcgtggtgcg cacgatagcg catagtgttt ttctctccac  10740
   ttgaatcgaa gagatagact tacggtgtaa atccgtaggg gtggcgtaaa ccaaattacg 10800
   caatgttttg ggttccattt aaatcgaaac cccttatttc ctggatcacc tgttaacgca 10860
   cgtttgacgt gtattacagt gggaataagt aaaagtgaga ggttcgaatc ctccctaacc 10920
   ccgggtaggg gcccagcggc cgctctagct agagtcaagc agatcgttca aacatttggc 10980
   aataaagttt cttaagattg aatcctgttg ccggtcttgc gatgattatc atataatttc 11040
   tgttgaatta cgttaagcat gtaataatta acatgtaatg catgacgtta tttatgagat 11100
   gggtttttat gattagagtc ccgcaattat acatttaata cgcgatagaa aacaaaatat 11160
   agcgcgcaaa ctaggataaa ttatcgcgcg cggtgtcatc tatgttacta gatcgacctg 11220
   catccacccc agtacattaa aaacgtccgc aatgtgttat taagttgtct aagcgtcaat 11280
   ttgtttacac cacaatatat cctgccacca gccagccaac agctccccga ccggcagctc 11340
   ggcacaaaat caccactcga tacaggcagc ccatcag                          11377
<210> 9
   <211> 1431
   <212> DNA
   <213> Artificial
<220>
   <223> optimised coding sequence of β-glucosidase Bg14 from Humicola grisea (BAA74958.1)
<400> 9
   atgagcctac cacctgactt taagtggggt tttgctaccg ccgcttatca gatagaagga     60
   tctgtgaatg aggatggtag aggaccttct atttgggaca ccttttgtgc aatccctggt    120
   aaaatagctg acggaagctc aggagctgtt gcctgtgatt cttacaagcg gacaaaggaa    180
   gatattgcct tgttaaaaga gcttggtgct aactcctata gattttccat ttcttggtcc    240
   cgaattatcc ctctgggcgg aagaaatgac cctatcaacc aaaaaggcat cgatcattac    300
   gtgaagttcg tagacgatct aatcgaggcc ggaattactc cattcattac tctcttccac    360
   tgggatcttc ccgatgcatt ggataagagg tacgggggct ttctaaacaa agaagagttc    420
   gctgcagatt tcgagaatta tgcaaggatt atgtttaaag caatccctaa gtgcaagcac    480
   tggattacct ttaacgaacc atggtgtagt gccattctcg gatataatac aggctatttc    540
   gcacctggac acacttctga tagatctaag agtccggtgg gtgactcagc tcgtgaaccg    600
   tggatagttg ggcacaacat actaattgcc catgctaggg cagttaaggc ttatcgtgaa    660
   gatttcaaac caacgcaagg tggagaaata ggtataacgc tgaacggtga tgctactctt    720
   ccgtgggatc cagaggatcc cgctgacatt gaagcatgtg ataggaaaat cgaatttgcc    780
   ataagctggt tcgcagatcc gatctacttc ggcaaatatc cggactcaat gagaaagcag    840
   ttaggggatc gactcccaga attcacgcct gaagaggtgg ctctagtgaa aggtagtaac    900
   gatttctacg ggatgaatca ctatacggct aattacatca aacacaagac tggtgttcct    960
   cccgaagacg atttccttgg aaaccttgag acactctttt ataacaaata tggtgattgc   1020
   attggccctg agacacagtc attctggtta agaccacatg ctcaagggtt tcgagatttg   1080
   ctgaattggc tttcaaagag gtacgggtac cctaagatct acgtcactga aaacggaacc   1140
   agtctgaagg gtgagaatga catgccacta gaacaagtcc tcgaggacga tttccgagtt   1200
   aagtatttca atgattacgt tcgtgcaatg gccgctgcag tagctgaaga tggttgcaac   1260
   gtaagaggct acttggcctg gagtctactg gataattttg aatgggctga gggatacgag   1320
   acacggttcg gtgttactta tgtcgactat gccaacgatc agaagcgcta tccaaagaaa   1380
   tctgcaaagt ccttaaagcc cctgtttgat agccttattc gtaaagagta a             1431
<210> 10
   <211> 954
   <212> DNA
   <213> Artificial
<220>
   <223> optimised coding sequence of endoglucanase Cel5A from Thermotoga maritima (3MMW_D)
<400> 10
   atgggcgttg atccatttga acggaataaa atcctgggac gcggaattaa tattggcaat      60
   gcactggaag caccaaatga aggtgattgg ggcgtagtca tcaaggatga gttctttgat     120
   attataaaag aagcgggttt ctcccacgtt aggataccta ttcgctggtc tacacacgca     180
   tacgccttcc caccttataa gataatggat agattcttta agcgtgttga cgaagtcatc     240
   aacggagctt tgaaaagagg attagccgtg gttattaaca tccatcatta tgaagagctc     300
   atgaacgacc cagaggaaca taaagagaga tttctcgctt tatggaagca aatagctgat     360
   aggtacaagg actatcctga gactttgttc ttcgagattc tcaatgagcc tcatggaaat     420
   ctaacgcctg agaagtggaa tgaacttttg gaagaggcgc taaaggttat tagaagcatc     480
   gataagaagc atacgataat cataggtact gcagaatggg gagggatctc tgctcttgag     540
   aagttgagtg ttcccaagtg ggagaaaaac tcaattgtga ctatccacta ctacaatcca     600
   ttcgagttta ctcaccaagg tgctgaatgg gtcgaaggat cagaaaagtg gcttggaaga     660
   aaatggggat ctccggatga tcagaaacac ctcatagagg aattcaactt tatcgaagag     720
   tggtcgaaaa agaacaaacg gccgatttat attggggaat ttggggctta cagaaaagct     780
   gacctggaaa gccgaattaa gtggacatcc tttgtggtcc gagagatgga gaagcgtcgt     840
   tggagttggg cctattggga gttctgtagt ggctttgggg tgtatgatac actaagaaag     900
   acctggaata aagacctgct tgaagccttg attggcgggg atagtatcga ataa           954
<210> 11
   <211> 1566
   <212> DNA
   <213> Artificial
<220>
   <223> optimised coding sequence of endoglucanse E1 from Acidothermus cellulolyticus (P54583.1) mature part without signal peptide
<400> 11
   gcaggtggcg gatattggca cacttctggt agagaaatcc tagatgcaaa caatgtgcct      60
   gttcgcatag ctgggattaa ctggttcggc ttcgagactt gtaactacgt agtccacgga     120
   ctttggagtc gggactatag aagcatgctc gatcaaatca aaagcctggg atacaacacg     180
   attcgactgc catactctga tgacattctg aaacctggaa ccatgccgaa ctccatcaac     240
   ttctatcaaa tgaaccaaga tctgcaaggg ctgactagct tgcaagtgat ggataagata     300
   gtagcctacg ctggccaaat tgggcttaga ataatcctcg atagacatcg tcctgattgc     360
   tctggacaat ctgcactctg gtatacatcc tctgtctctg aagctacctg gatttccgat     420
   ttgcaagcac tagcccagag atacaaagga aatccaaccg tagtcggctt tgacttgcat     480
   aacgaaccac atgatcctgc ttgttgggga tgtggtgatc catcaattga ctggcgttta     540
   gctgctgaaa gagctggaaa cgctgtctta tccgtgaatc ccaatcttct catcttcgta     600
   gagggagttc agtcatacaa tggcgattct tactggtggg gagggaatct acaaggcgct     660
   ggacaatacc ctgtagttct aaacgtaccc aaccgattgg tgtattctgc ccatgactat     720
   gcaacatccg tttatccgca aacctggttt tctgatccca catttccgaa caatatgcct     780
   ggcatttgga acaagaactg gggatatctg ttcaaccaaa acattgctcc cgtatggctt     840
   ggcgagtttg gaacaacttt acaaagcaca acggaccaga cctggcttaa gacattagtg     900
   caatatctta gaccgaccgc acaatatggt gctgatagct ttcaatggac gttctggagt     960
   tggaatcctg attctggcga tacgggtgga atacttaagg atgactggca aaccgttgac    1020
   actgtaaagg acggatacct tgctcccata aagtctagca tctttgatcc cgttggtgca    1080
   tctgcttctc caagttcaca accaagtccc tctgtttcac caagtccatc accatcacct    1140
   agtgcttcta gaacaccaac tcctactcca acaccaactg catctccaac tcctacattg    1200
   acacctaccg caacacctac acctactgct tcacctactc catcacctac tgccgcttct    1260
   ggagctagat gcactgcttc ttatcaagtc aactctgatt ggggaaacgg ctttactgtg    1320
   accgttgctg tcacgaattc tggaagtgtg gcaacaaaga cttggactgt ttcttggacg    1380
   tttgggggaa atcaaaccat tacgaactct tggaatgctg ccgtaacaca aaatgggcaa    1440
   tccgttaccg ctcggaacat gtcatacaat aacgtcatcc aacctgggca aaacacaacg    1500
   ttcggctttc aagcctccta taccggttca aatgctgcac ctactgttgc ttgcgctgca    1560
   tcttaa                                                               1566
<210> 12
   <211> 1491
   <212> DNA
   <213> Artificial
<220>
   <223> optimised coding sequence of exoglucanase 1 (CBHI) from Trichoderma reesei (P62694.1) mature part without signal peptide
<400> 12
   caatctgctt gcactctgca atccgaaacg catccacctt tgacttggca aaagtgttcc      60
   tctggtggaa cgtgtaccca gcaaaccggt tctgtagtga ttgacgcaaa ttggagatgg     120
   acacacgcta ccaatagctc tacgaactgc tatgatggga atacctggtc tagcacacta     180
   tgccctgata acgaaacctg tgcaaagaat tgctgtcttg atggtgctgc ctatgcttca     240
   acttacggtg tcacaactag cggcaattct ctttccatcg gcttcgtcac acaaagtgcc     300
   caaaagaacg ttggagcacg gctttacctt atggcttccg acacgaccta tcaagaattc     360
   acgctgcttg ggaatgagtt ttcctttgat gtggacgttt ctcaactccc atgcggactt     420
   aacggagcac tttactttgt gtctatggac gctgatggag gcgtttctaa gtatccgact     480
   aacactgctg gtgctaagta cggaaccggt tactgcgatt cacaatgccc tagagatctg     540
   aagttcatca acggacaagc aaacgtcgag ggctgggaac cttcttccaa taacgcaaac     600
   actggcattg gtggccacgg atcatgttgt tctgagatgg acatttggga agcaaactca     660
   atctccgaag cacttacccc acatccgtgt acaactgttg gccaagaaat ctgtgagggt     720
   gatggatgtg gtggcactta ctctgacaat cgctatggtg gaacctgtga tccagatgga     780
   tgcgattgga acccctatag gcttggaaac acgtctttct atggacctgg ctctagcttt     840
   acccttgata ccactaagaa actcaccgtg gtaacgcaat tcgagactag cggagccata     900
   aaccgatatt acgtacagaa tggcgttacg tttcaacaac ccaatgccga gttgggctca     960
   tactctggga atgagttgaa tgatgactac tgtactgctg aagaggctga attcggtgga    1020
   agttccttta gcgataaggg aggcctaacc cagtttaaga aagcaacgtc tggtgggatg    1080
   gttctcgtca tgagtctatg ggatgactac tatgctaaca tgctgtggct cgattccaca    1140
   tatcccacca atgagactag ttcaactcct ggagctgttc gtggatcctg ttcaactagc    1200
   tctggtgttc ctgcccaagt agaatctcaa agtcccaacg caaaggtgac cttttccaac    1260
   ataaagttcg gaccgattgg ctcaactgga aatccatctg ggggaaatcc acctggtggc    1320
   aatagaggaa caactacaac tagaaggcct gctactacca ctggttcatc tcctggtcca    1380
   actcaatctc attatggcca atgcggtggc attggctatt ctggtcctac tgtgtgtgct    1440
   agtggaacca catgccaagt tctcaacccc tattactccc aatgcctcta a              1491
<210> 13
   <211> 1524
   <212> DNA
   <213> Artificial
<220>
   <223> optimised coding sequence of exoglucanase 1 (CBHI) from Humicola grisea (D63515) mature part without signal peptide
<400> 13
   cagcaggctt gttcactcac cacagaacgg catccaagcc tatcatggaa gaaatgtact     60
   gctggcggtc agtgtcaaac tgttcaggcc tcgatcacct tagatagcaa ttggcggtgg    120
   acacatcaag ttagtggctc aacgaattgt tatacgggaa ataagtggga cacctctatt    180
   tgtaccgatg ctaaatcatg cgctcaaaat tgctgcgttg acggtgctga ctacacctcc    240
   acttatggga ttaccaccaa cggagatagt ctctctctga agttcgtcac caagggtcag    300
   tattctacga acgtaggctc gaggacgtat ctgatggatg gagaggacaa ataccaaaca    360
   ttcgagcttt tggggaacga gtttacgttc gacgtggacg ttagtaacat cggttgtggc    420
   cttaacggcg ctctctattt tgtgagcatg gacgcagatg ggggattgag cagatatcca     480
   gggaacaaag ccggagctaa gtatggtact gggtattgcg acgcgcaatg cccgagggac    540
   attaagttca taaatgggga agcgaacatc gaaggctgga ctggatcaac gaacgatccg    600
   aacgctggag ctggtcgata cgggacctgt tgttctgaga tggatatttg ggaagcgaac    660
   aatatggcaa ctgcgtttac tcctcacccg tgtaccatta taggtcagtc ccgctgtgaa    720
   ggcgatagtt gcggagggac ttactctaat gagaggtatg ctggcgtttg tgaccctgat    780
   ggctgcgatt tcaactccta cagacagggg aataagacgt tctacggaaa ggggatgact    840
   gtcgacacca cgaaaaagat tacggtggtc actcagttct tgaaagacgc aaatggcgat    900
   ctgggggaga taaagcgttt ctatgtgcaa gatgggaaga ttatcccgaa ttctgagagc    960
   acgatacctg gagtagaagg gaactcaatt actcaagatt ggtgcgatcg gcaaaaagtg   1020
   gcatttggcg acatcgacga cttcaatcgc aagggaggta tgaagcagat ggggaaagct   1080
   ctagccggtc caatggtact tgtcatgtca atctgggatg accacgcttc taacatgctt   1140
   tggcttgact caacctttcc ggttgacgca gccggaaaac ctggagccga acgaggcgct   1200
   tgcccaacta catctggagt tcctgccgaa gtggaagcag aggcacccaa ttcgaacgta   1260
   gttttctcca acattcgatt cggcccgata ggttctaccg tcgccggctt acctggagct   1320
   ggaaatggag gcaataatgg agggaatcca cccccaccta ctacaacgac gagttctgca   1380
   cctgccacta ccacaactgc tagcgccggt ccaaaagctg ggcgttggca acaatgcggg   1440
   ggaattggat tcactggacc tacccagtgc gaagagccct acacctgtac aaaactgaat   1500
   gattggtact cccagtgctt ataa                                          1524
<210> 14
   <211> 1677
   <212> DNA
   <213> Artificial
<220>
   <223> optimised coding sequence of exocellulase E3 from Thermobifida fusca (AAA62211.1) mature part without signal peptide
<400> 14
   gcaggctgtt ctgttgatta caccgtgaat tcttggggaa ctggattcac ggctaacgtt     60
   accatcacga atcttggttc tgccataaac ggatggacat tagagtggga cttccctggc    120
   aaccagcaag ttaccaacct gtggaatggc acgtataccc agtctgggca acacgtttct    180
   gtttctaatg ctccttacaa cgcatctatc cctgcaaatg gcactgtcga attcggcttt    240
   aatgggagct attctggaag caacgacata cctagctctt ttaagctgaa cggtgtaaca    300
   tgtgacggga gcgacgatcc agatcctgaa ccttctccat caccttctcc atctcccagc    360
   cctactgatc ctgatgaacc tggtggacca accaatcctc caacaaaccc tggcgagaaa    420
   gtggacaatc ccttcgaggg tgccaaactt tacgtaaacc ctgtatggag tgctaaagct    480
   gcagctgaac ctggtggatc tgctgtagcc aatgaatcta ctgctgtttg gctggataga    540
   atcggtgcca tagagggaaa cgattctcct acaactgggt ctatgggtct tagagatcat    600
   ctcgaggaag ccgtaagaca aagtggtggc gatcccctta ccatccaagt tgtgatctac    660
   aatctacctg gtcgcgactg tgctgcactt gcttctaatg gagaattggg acctgatgaa    720
   ctggatagat acaagtccga gtacattgat cccattgctg acatcatgtg ggacttcgct    780
   gactatgaga acctacgaat cgtcgccatc attgagatag actccctccc taatctagtc    840
   actaatgtcg gaggcaacgg agggactgag ctatgtgcat acatgaagca aaacggaggc    900
   tacgtcaatg gagttggcta tgcccttaga aagttgggtg agattcctaa cgtctacaac    960
   tacatcgatg ccgctcacca tggctggata ggctgggatt ctaactttgg accctccgtg   1020
   gacatcttct acgaagctgc aaatgcctct ggtagcaccg ttgactatgt gcatggattc   1080
   atctctaaca cggcaaacta tagcgctact gttgaaccgt atctagacgt aaacggcacc   1140
   gtgaatgggc aactgataag gcaatctaag tgggtcgact ggaaccaata cgttgatgag   1200
   ttgagtttcg tccaagactt gaggcaagct ctcatagcca aagggtttcg ttctgatatc   1260
   ggcatgctca tagacacttc tagaaacgga tggggaggcc ctaatcgtcc aactggacct   1320
   agctcatcta cggaccttaa cacatacgtt gacgagtcac gaattgatag acgcatacac   1380
   cctggaaact ggtgcaacca agctggtgct ggcttgggtg aaagacctac cgtgaaccca   1440
   gctcctggtg ttgacgcata cgtttgggtt aagcctccgg gagaatctga tggagcttcc   1500
   gaggaaatcc ctaatgatga ggggaagggc ttcgatagaa tgtgcgatcc tacttaccaa   1560
   ggcaacgcta gaaacggcaa caatccatct ggtgctctac ctaatgctcc catatctggg   1620
   cactggtttt ctgcccaatt tcgggagctt ctagctaatg cttaccctcc cctctaa      1677